# EUROPEAN PATENT APPLICATION

(11) **EP 3 053 917 A1**
(43) Date of publication of application: **10.08.2016**
(21) Application number: 14851204.9
(22) Date of filing: 02.10.2014
(51) Int. Cl.: C07D 401/14, A61K 31/53, A61K 31/55, A61P 25/04, A61P 43/00, C07D 401/04, C07D 409/14, C07D 417/14, C07D 471/04, C07D 487/04, C07D 491/056, C07D 495/04, C07D 513/04

(54) **T-TYPE CALCIUM CHANNEL BLOCKER**

(30) Priority: 02.10.2013 JP 2013207405; 22.05.2014 JP 2014106313
(71) Applicant: Nissan Chemical Industries, Ltd., Chiyoda-ku Tokyo 101-0054 (JP)
(72) Inventor: NIWA, Masatoshi, Funabashi-shi Chiba 274-8507 (JP); INABA, Yusuke, Funabashi-shi Chiba 274-8507 (JP); IWAMOTO, Toshimasa, Funabashi-shi Chiba 274-8507 (JP); SHINTANI, Yusuke, Funabashi-shi Chiba 274-8507 (JP); NAGAI, Hiroshi, Funabashi-shi Chiba 274-8507 (JP); EGI, Jun, Funabashi-shi Chiba 274-8507 (JP); ADACHI, Michiaki, Funabashi-shi Chiba 274-8507 (JP); HIRAI, Yuichi, Shiraoka-shi Saitama 349-0294 (JP)
(74) Representative: Hoffmann Eitle
(86) International application number: PCT/JP2014/076423
(87) International publication number: WO 2015/050212

(57) **Abstract**

It is an object to provid a novel compound that has an excellent T-type calcium channel inhibitory activity and is specifically useful for prevention or treatment of pain, chronic kidney disease and atrial fibrillation. The present invention provides a novel triazinone compound of Formula (I): 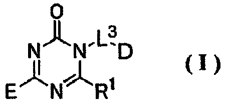 where each substituent in the formula is defined in detail in the description, and R¹ means a hydrogen atom, or a C₁₋₆ alkyl group, etc., E means a 7 to 14-membered non-aromatic fused heterocyclic group, L³ means a C₁₋₆ alkylene group, etc., D means a C₆₋₁₄ aryyl group or a 5 to 10-membered heteroaryl group each of which is optionally substituted, etc., a tautomer of the compound, or a pharmaceutically acceptable salt thereof, or a solvate thereof.

## Description

### TECHNICAL FIELD

The present invention relates to a novel compound having an inhibitory activity on a T-type voltage-dependent calcium channel.

### BACKGROUND ART

Voltage-dependent calcium channels are a transmembrane multisubunit proteins that control inflow of extracellular calcium ions into cells. The voltage-dependent calcium channels are further classified into various types in mammalian cells. Major types of the voltage-dependent calcium channels include L-type, T-type, N-type, P/Q type, and R-type calcium channels, which play individual roles in various tissues including skeletal muscles, cardiac muscles, lungs, smooth muscles, and brain. Among these types, the "T-type" (or "low-voltage activated-type") calcium channel is named after its characteristic that has a shorter (T = transient) opening time than the L-type calcium channel that has a longer (L = long-lasting) opening time [Non-Patent Document 1].

The T-type calcium channels have channel characteristics, which are known to be a factor to open the L-type calcium channels and a factor to fluctuate the action potential of sodium channels. Here, hyperexcitability of nerves due to an abnormality (abnormal firing) in fluctuations of the action potential of the sodium channels is believed to be a pathogenesis of neuropathic pains. The T-type calcium channels are supposed to relate to the abnormal firing, and blocking of the T-type calcium channels are believed to suppress the abnormal firing itself and to suppress pains [Non-Patent Document 2].

More specifically, the T-type calcium channels identified in various mammals including humans include three subtypes: α1G (Cav3.1), α1H (Cav3.2), and α1I (Cav3.3). Among the tree subtypes of the T-type calcium channels, α1H is expressed in the dorsal root ganglion (DRG) and the dorsal horn of the spinal cord, which relate to pain transmission [Non-Patent Document 2, Non-Patent Document 12]. In studies using α1H knockout mice, analgesic action has been disclosed in acute pain models (tail clip, tail flip, and hot plate tests), inflammatory pain models (capsaicin and formalin-induced tests), and visceral pain models (acetic acid and magnesium sulfate inductions). During the tests, no abnormality was observed in general behavior [Non-Patent Document 3].

Analgesic action has also been identified in neuropathic pain model rats (CCD) to which an antisense gene of α1H is administered to suppress the expression of α1H in the spinal cord [Non-Patent Document 4]. In addition, in the case of suppressing the expression in the DRG, analgesic action has been identified in neuropathic pain model rats (CCI) [Non-Patent Document 5].

As for the action on pains associated with diabetic neuropathy, in the DRG of pain model rats having diabetic neuropathy prepared by administration of streptozotocin, an increase in gene expression of α1H [Non-Patent Document 6] and an increase in T-type calcium channel current [Non-Patent Document 7] have been disclosed, and the pain suppressive action has also been identified by the intrathecal administration of an antisense gene of α1H to the pain model rats [Non-Patent Document 8]. It has been disclosed that the onset of pain has been completely suppressed in α1H knockout mice to which streptozotocin has been administered, and the expression of α1H in the DRG has increased and the administration of a T-type calcium channel inhibitor has provided an analgesic action in ob/ob mice as diabetic model mice [Non-Patent Document 9]. From these findings, compounds having the inhibitory activity on the T-type calcium channel should be used as a therapeutic agent for pain.

The T-type calcium channels are considered to relate to pains such as neuropathic pain, inflammatory pain, and cancer pain, as well as pathology of various diseases and disorders including epilepsy, essential tremor, schizophrenia, Parkinson's disease, depression, anxiety, sleep disorder, sleep disturbance, mental illness, schizophrenia, cardiac arrhythmia, hypertension, cancer, diabetes, overactive bladder, chronic kidney disease, sterility, and sexual dysfunction [Non-Patent Document 2, Non-Patent Document 3, Non-Patent Document 10, Non-Patent Document 11, Non-Patent Document 13, Non-Patent Document 14, Non-Patent Document 15, and Non-Patent Document 16].

Treatment methods for such diseases involve many problems, and thus there is a demand for development of novel pharmaceutical products. Although some compounds having the T-type calcium channel inhibitory activity have been disclosed (for example, see Patent Documents 1 to 3), there is a demand for development of new medicinal agents.

### Prior Art Documents

### Patent Documents

Patent Document 1: International Publication No. 2009/146540 pamphlet (WO 2009/146540)
Patent Document 2: International Publication No. 2011/115813 pamphlet (WO 2011/115813)
Patent Document 3: International Publication No. 2011/035159 pamphlet (WO 2011/035159)

### Non-Patent Documents

Non-Patent Document 1: Physiology of Neuron, Kyoto University Press (2009)
Non-Patent Document 2: British Journal of Pharmacology, Vol. 163, pp. 484-495 (2011)
Non-Patent Document 3: Genes, Brain and Behavior, Vol. 6, pp. 425-431 (2007)
Non-Patent Document 4: Acta Pharmacological Sinica, Vol. 27 (No. 12), pp. 1547-1552 (2006)
Non-Patent Document 5: The EMBO Journal, Vol. 24, pp. 315-324 (2005)
Non-Patent Document 6: Journal of Neurochemistry, Vol. 119 (No. 3), pp. 594-603 (2011)
Non-Patent Document 7: Journal of Neuroscience, Vol. 28 (No. 12), pp. 3305-3316 (2007)
Non-Patent Document 8: Pain, Vol. 145 (Nos. 1-2), pp. 184-195 (2009)
Non-Patent Document 9: Diabetes, Vol. 58, pp. 2656-2665 (2009)
Non-Patent Document 10: The Journal of Pharmacology and Experimental Therapeutics, Vol. 335, No. 2, pp. 409-417 (2010)
Non-Patent Document 11: Journal of Assisted Reproduction and Genetics, Vol. 28, No. 1, pp. 23-30 (2011)
Non-Patent Document 12: Pharmacological Reviews, Vol. 83, pp. 117-161 (2003)
Non-Patent Document 13: Neurourology and Urodynamics, Vol. 26, pp. 870-878 (2007)
Non-Patent Document 14: BJU International, Vol. 99 (No. 2), pp. 436-441 (2007)
Non-Patent Document 15: American Journal of Hypertension, Vol. 30, No. 8, pp. 1620-1631 (2012)
Non-Patent Document 16: Bioorganic and Medicinal Chemistry Letters, Vol. 23 No. 1, pp. 119-124 (2013)

### SUMMARY OF THE INVENTION

### Problem to be Solved by the Invention

The present invention provides a novel triazinone compounds which are T-type voltage-dependent calcium channel inhibitors. The present invention also provides a pharmaceutical compositions containing the compounds of the present invention.

### Means for Solving the Problem

As a result of intensive studies for developing inhibitors of a T-type voltage-dependent calcium channel, the inventors of the present invention have found that the compounds of the present invention has a high inhibitory activity on the T-type voltage-dependent calcium channel and have accomplished the present invention.

Specifically, the present invention has the following aspects.

(1) A compound of Formula (I): 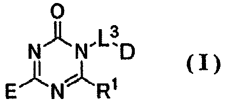 [wherein
   R¹ means
      a hydrogen atom
      a halogen atom,
      a C₁₋₆ alkyl group,
      a C₁₋₆ alkoxy group,
      a C₁₋₆ alkylthio group,
      a mono-C₁₋₆ alkylamino group,
      a di-C₁₋₆ alkylamino group,
      (the C₁₋₆ alkyl group, the C₁₋₆ alkoxy group, the C₁₋₆ alkylthio group, the mono-C₁₋₆ alkylamino group, and the di-C₁₋₆ alkylamino group are unsubstituted or substituted with one or more substituents identically or differently selected from a substituent group V⁸) or,
      a C₃₋₁₁ cycloalkyl group
      (the C₃₋₁₁ cycloalkyl group is unsubstituted or substituted with one or more substituents identically or differently selected from a substituent group V⁶));
   E means
      a 7 to 14-membered non-aromatic fused heterocyclic group
      (the 7 to 14-membered non-aromatic fused heterocyclic group is bonded to a carbon atom of a triazine skeleton in Formula (I) at a non-aromatic ring side; the non-aromatic ring side is unsubstituted or substituted with one or more substituents identically or differently selected from a substituent group V⁹; an oxo group, a thioxo group, or a hydroxyimino group is optionally substituted for hydrogen atoms of a methylene group in the non-aromatic ring; and an aromatic ring side is unsubstituted or substituted with one or more substituents identically or differently selected from the substituent group V⁶ (when the aromatic ring side has two or more substituents, the two substituents optionally form a ring together);
   L³ means
      a C₁₋₆ alkylene group
      (the C₁₋₆ alkylene group is unsubstituted or substituted with one or more substituents identically or differently selected from the substituent group V⁸ and one methylene group in the C₁₋₆ alkylene group is optionally replaced by a carbonyl group (>C=O) or a thiocarbonyl group (>C=S));
   D means
      a C₆₋₁₄ aryl group,
      a 5 to 10-membered heteroaryl group,
      or a 7 to 14-membered non-aromatic fused heterocyclic group
      (the C₆₋₁₄ aryl group, the 5 to 10-membered heteroaryl group, and the 7 to 14-membered non-aromatic fused heterocyclic group are unsubstituted or substituted with one or more substituents identically or differently selected from the substituent group V⁶);
   the substituent group V⁶ means a substituent group consisting of substituents constituting the substituent group V⁸, C₁₋₆ alkyl groups, C₂₋₆ alkenyl groups, and C₂₋₆ alkynyl groups (the C₁₋₆ alkyl groups, the C₂₋₆ alkenyl groups, and the C₂₋₆ alkynyl groups are unsubstituted or substituted with one or more substituents identically or differently selected from a substituent group V¹);
   the substituent group V⁸ means a substituent group consisting of substituents constituting a substituent group V^{a}, C₁₋₁₀ alkoxy groups, C₆₋₁₄ aryloxy groups, C₁₋₆ alkylthio groups, C₁₋₆ alkylcarbonyl groups, C₁₋₆ alkylsulfonyl groups, C₁₋₆ alkoxycarbonyl groups, mono-C₁₋₆ alkylamino groups, di-C₁₋₆ alkylamino groups, mono-C₁₋₆ alkylaminocarbonyl groups, di-C₁₋₆ alkylaminocarbonyl groups, mono-C₁₋₆ alkylaminosulfonyl groups, di-C₁₋₆ alkylaminosulfonyl groups, C₁₋₆ alkylcarbonylamino groups, C₁₋₆ alkylcarbonyloxy groups, C₁₋₆ alkylsulfonylamino groups, C₁₋₆ alkylsulfonyloxy groups (the C₁₋₁₀ alkoxy groups, the C₆₋₁₄ aryloxy groups, the C₁₋₆ alkylthio groups, the C₁₋₆ alkylcarbonyl groups, the C₁₋₆ alkylsulfonyl groups, the C₁₋₆ alkoxycarbonyl groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the mono-C₁₋₆ alkylaminosulfonyl groups, the di-C₁₋₆ alkylaminosulfonyl groups, the C₁₋₆ alkylcarbonylamino groups, the C₁₋₆ alkylcarbonyloxy groups, the C₁₋₆ alkylsulfonylamino groups, and the C₁₋₆ alkylsulfonyloxy groups are unsubstituted or substituted with one or more substituents identically or differently selected from the substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylsulfonylamino groups, C₃₋₆ cycloalkylsulfonyloxy groups, C₃₋₆ cycloalkylthio groups, C₃₋₁₁ cycloalkyl groups, 3 to 11-membered heterocyclyl groups, C₆₋₁₄ aryl groups, 5 to 10-membered heteroaryl groups, or 7 to 14-membered non-aromatic fused heterocyclic groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylsulfonylamino groups, the C₁₋₆ cycloalkylsulfonyloxy groups, the C₃₋₆ cycloalkylthio groups, the C₃₋₁₁ cycloalkyl groups, the 3 to 11-membered heterocyclyl groups, the C₆₋₁₄ aryl groups, the 5 to 10-membered heteroaryl groups, and the 7 to 14-membered non-aromatic fused heterocyclic groups are unsubstituted, or substituted with one or more substituents identically or differently selected from a substituent group V²);
   the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group;
   the substituent group V¹ means a substituent group consisting of substituents constituting the substituent group V^{a}, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more hydroxy groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di C₁₋₆ alkylaminocarbonyl groups, one or more C₁₋₆ alkylcarbonylamino groups, one or more C₁₋₆ alkylthio groups, or one or more C₁₋₆ alkylsulfonyl groups), C₃₋₁₁ cycloalkyl groups, C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 11-membered heterocyclyl groups, C₆₋₁₄ aryl groups, 5 to 10-membered heteroaryl groups, or 7 to 14-membered non-aromatic fused heterocyclic groups (the C₃₋₁₁ cycloalkyl groups, the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 11-membered heterocyclyl groups, the C₆₋₁₄ aryl groups, 5 to 10-membered heteroaryl groups, and 7 to 14-membered non-aromatic fused heterocyclic groups are unsubstituted or substituted with one or more hydroxyl groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkyl groups, one or more C₁₋₃ halo alkyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di C₁₋₆ alkylaminocarbonyl groups, one or more C₁₋₆ alkylcarbonylamino groups, one or more C₁₋₆ alkylthio groups, or one or more C₁₋₆ alkylsulfonyl groups);
   the substituent group V² means a substituent group consisting of substituents constituting the substituent group V¹, C₁₋₆ alkyl groups, and C₁₋₃ haloalkyl groups; and
   the substituent group V⁹ means substituents constituting the substituent group V^{a}, C₁₋₆ alkoxycarbonyl groups, C₁₋₆ alkylsulfonyloxy groups, C₁₋₆ alkylsulfonylamino groups, mono-C₁₋₆ alkylaminocarbonyl groups, di C₁₋₆ alkylaminocarbonyl groups, C₁₋₆ alkylcarbonylamino groups, C₁₋₆ alkylcarbonyloxy groups, mono-C₁₋₆ alkylaminosulfonyl groups, or di-C₁₋₆ alkylaminosulfonyl groups], a tautomer of the compound, or a pharmaceutically acceptable salt thereof, or a solvate thereof.
(2) A compound of Formula (I): 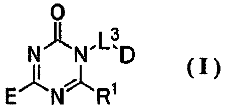 [wherein
   R¹ means
      a hydrogen atom,
      a halogen atom,
      a C₁₋₆ alkyl group,
      a C₁₋₆ alkoxy group,
      a C₁₋₆ alkylthio group,
      a mono-C₁₋₆ alkylamino group,
      a di-C₁₋₆ alkylamino group,
      (the C₁₋₆ alkyl group, the C₁₋₆ alkoxy group, the C₁₋₆ alkylthio group, the mono-C₁₋₆ alkylamino group, and the di-C₁₋₆ alkylamino group are unsubstituted or substituted with one or more substituents identically or differently selected from the substituent group V⁸), or
      a C₃₋₁₁ cycloalkyl group
      (the C₃₋₁₁ cycloalkyl group is unsubstituted or substituted with one or more substituents identically or differently selected from the substituent group V⁶);
   E means
      a 7 to 14-membered non-aromatic fused heterocyclic group,
      (the 7 to 14-membered non-aromatic fused heterocyclic group is bonded to a carbon atom of a triazine skeleton in Formula (I) at a non-aromatic ring side; the non-aromatic ring side is unsubstituted or substituted with one or more substituents identically or differently selected from the substituent group V⁹; and an aromatic ring side is unsubstituted or substituted with one or more substituents identically or differently selected from the substituent group V⁶ (when the aromatic ring side has two or more substituents, the two substituents optionally form a ring together));
   L³ means
      a C₁₋₆ alkylene group,
      (the C₁₋₆ alkylene group is unsubstituted or substituted with one or more substituents identically or differently selected from the substituent group V⁸ and one methylene group in the C₁₋₆ alkylene group is optionally replaced by a carbonyl group (>C=O) or a thiocarbonyl group (>C=S));
   D means
      a C₆₋₁₄ aryl group,
      a 5 to 10-membered heteroaryl group, or
      a 7 to 14-membered non-aromatic fused heterocyclic group
      (the C₆₋₁₄ aryl group, the 5 to 10-membered heteroaryl group, and the 7 to 14-membered non-aromatic fused heterocyclic group are unsubstituted or substituted with one or more substituents identically or differently selected from the substituent group V⁶);
   the substituent group V⁶ means a substituent group consisting of substituents constituting the substituent group V⁸, C₁₋₆ alkyl groups, C₂₋₆ alkenyl groups, and C₂₋₆ alkynyl groups (the C₁₋₆ alkyl groups, the C₂₋₆ alkenyl groups, and the C₂₋₆ alkynyl groups are unsubstituted or substituted with one or more substituents identically or differently selected from the substituent group V¹);
   the substituent group V⁸ is a substituent group consisting of substituents constituting the substituent group V^{a}, C₁₋₁₀ alkoxy groups, C₆₋₁₄ aryloxy groups, C₁₋₆ alkylthio groups, C₁₋₆ alkylcarbonyl groups, C₁₋₆ alkylsulfonyl groups, C₁₋₆ alkoxycarbonyl groups, mono-C₁₋₆ alkylamino groups, di-C₁₋₆ alkylamino groups, mono-C₁₋₆ alkylaminocarbonyl groups, di-C₁₋₆ alkylaminocarbonyl groups, mono-C₁₋₆ alkylaminosulfonyl groups, di-C₁₋₆ alkylaminosulfonyl groups, C₁₋₆ alkylcarbonylamino groups, C₁₋₆ alkylcarbonyloxy groups, C₁₋₆ alkylsulfonylamino groups, C₁₋₆ alkylsulfonyloxy groups (the C₁₋₁₀ alkoxy groups, the C₆₋₁₄ aryloxy groups, the C₁₋₆ alkylthio groups, the C₁₋₆ alkylcarbonyl groups, the C₁₋₆ alkylsulfonyl groups, the C₁₋₆ alkoxycarbonyl groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the mono-C₁₋₆ alkylaminosulfonyl groups, the di-C₁₋₆ alkylaminosulfonyl groups, the C₁₋₆ alkylcarbonylamino groups, the C₁₋₆ alkylcarbonyloxy groups, the C₁₋₆ alkylsulfonylamino groups, and the C₁₋₆ alkylsulfonyloxy groups are unsubstituted or substituted with one or more substituents identically or differently selected from the substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylsulfonylamino groups, C₃₋₆ cycloalkylsulfonyloxy groups, C₃₋₆ cycloalkylthio groups, C₃₋₁₁ cycloalkyl groups, 3 to 11-membered heterocyclyl groups, C₆₋₁₄ aryl groups, 5 to 10-membered heteroaryl groups, or 7 to 14-membered non-aromatic fused heterocyclic groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylsulfonylamino groups, the C₃₋₆ cycloalkylsulfonyloxy groups, the C₃₋₆ cycloalkylthio groups, the C₃₋₁₁ cycloalkyl groups, the 3 to 11-membered heterocyclyl groups, the C₆₋₁₄ aryl groups, the 5 to 10-membered heteroaryl groups, and the 7 to 14-membered non-aromatic fused heterocyclic groups are unsubstituted or substituted with one or more substituents identically or differently selected from the substituent group V²);
   the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group;
   the substituent group V¹ means a substituent group consisting of substituents constituting the substituent group V^{a}, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more hydroxyl groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di-C₁₋₆ alkylaminocarbonyl groups, one or more C₁₋₆ alkylcarbonylamino groups, one or more C₁₋₆ alkylthio groups, or one or more alkylsulfonyl groups), C₃₋₁₁ cycloalkyl groups, C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 11-membered heterocyclyl groups, C₆₋₁₄ aryl groups, 5 to 10-membered heteroaryl groups, or 7 to 14-membered non-aromatic fused heterocyclic groups (the C₃₋₁₁ cycloalkyl groups, the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 11-membered heterocyclyl groups, the C₆₋₁₄ aryl groups, 5 to 10-membered heteroaryl groups, and 7 to 14-membered non-aromatic fused heterocyclic groups are unsubstituted or substituted with one or more hydroxy groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkyl groups, one or more C₁₋₃ haloalkyl groups, one or more C₁₋₆ alkoxy groups, one or more C₁₋₃ haloalkoxy groups, one or more mono-C₁₋₆alkylamino groups, one or more di-C₁₋₆ alkylamino groups, one or more mono-C₁₋₆ alkylaminocarbonyl groups, one or more di C₁₋₆ alkylaminocarbonyl groups, one or more C₁₋₆ alkylcarbonylamino groups, one or more C₁₋₆ alkylthio groups, or one or more C₁₋₆ alkylsulfonyl groups);
   the substituent group V² means a substituent group consisting of substituents constituting the substituent group V¹, C₁₋₆alkyl groups, and C₁₋₃ haloalkyl groups; and
   the substituent group V⁹ means substituents constituting the substituent group V^{a}, C₁₋₆ alkoxycarbonyl groups, C₁₋₆ alkylsulfonyloxy groups, C₁₋₆ alkylsulfonylamino groups, mono-C₁₋₆ alkylaminocarbonyl groups, di C₁₋₆ alkylaminocarbonyl groups, C₁₋₆ alkylcarbonylamino groups, C₁₋₆ alkylcarbonyloxy groups, mono-C₁₋₆ alkylaminosulfonyl groups, or di-C₁₋₆ alkylaminosulfonyl groups],
      a tautomer of the compound, or a pharmaceutically acceptable salt thereof, or a solvate thereof.
(3) The compound, tautomer of the compound, or pharmaceutically acceptable salt thereof, or solvate thereof according to (1) or (2), in which L³ is a C₁₋₃ alkylene group.
(4) The compound, tautomer of the compound, or pharmaceutically acceptable salt thereof, or solvate thereof according to (3), in which L³ is a methylene group.
(5) The compound, tautomer of the compound, or pharmaceutically acceptable salt thereof, or solvate thereof according to any one of (1) to (4), in which R¹ is a hydrogen atom or a C₁₋₆ alkyl group.
(6) The compound, tautomer of the compound, or pharmaceutically acceptable salt thereof, or solvate thereof according to (5), in which R¹ is a hydrogen atom or a methyl group.
(7) The compound, tautomer of the compound, or pharmaceutically acceptable salt thereof, or solvate thereof according to any one of (1) to (6), in which E is any one of Formula (II)-1 to Formula (II)-11 shown in (II): 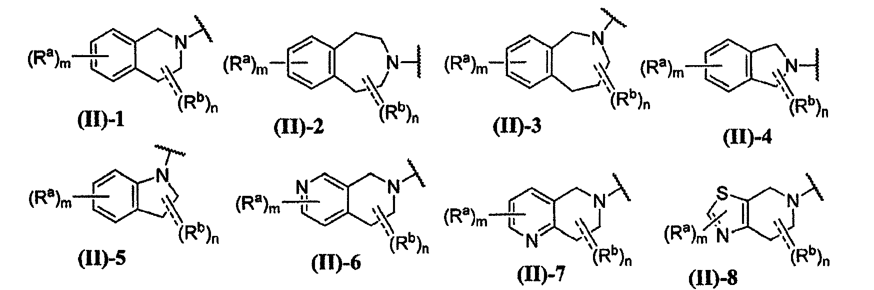 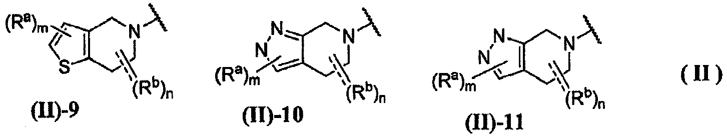 (where
   R^{b} means
      a hydrogen atom,
      a hydroxy group,
      an amino group,
      a halogen atom,
      a C₁₋₆ alkoxycarbonyl group
      an oxo group, or
      a hydroxyimino group;
   n is 1;
   R^{a} means a hydrogen atom,
      a cyano group,
      a halogen atom,
      a hydroxy group,
      an amino group,
      a C₁₋₆ alkyl group,
      a C₃₋₆ cycloalkyl group,
      a C₁₋₆ alkoxy group,
      a C₁₋₆ haloalkylsulfonyloxy group,
      a C₁₋₆ haloalkyl group,
      a C₁₋₆ haloalkoxy group,
      a C₁₋₆ alkyl group substituted with one hydroxy group,
      a C₁₋₆ alkoxy group substituted with one acetamido group, or
      a C₁₋₆ alkoxy group substituted with one C₃₋₆ cycloalkyl group; and
   m is 1 or 2; when m is 2, R^{a}s are the same as or different from each other and when m is 2 and two R⁸s are adjacent, the two R^{a}s optionally form a methylenedioxy group).
(8) The compound, tautomer of the compound, or pharmaceutically acceptable salt thereof, or solvate thereof according to (7), in which E is Formula (III): 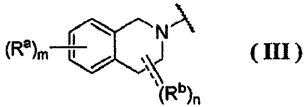 (where
   R^{b} means
      a hydrogen atom,
      a hydroxy group,
      an amino group,
      a halogen atom,
      a C₁₋₆ alkoxycarbonyl group,
      an oxo group, or
      a hydroxyimino group;
   n is 1;
   R^{a} means a hydrogen atom,
      a cyano group,
      a halogen atom,
      a hydroxy group,
      an amino group,
      a C₁₋₆ alkyl group,
      a C₃₋₆ cycloalkyl group,
      a C₁₋₆ alkoxy group,
      a C₁₋₆ haloalkylsulfonyloxy group,
      a C₁₋₆ haloalkyl group,
      a C₁₋₆ haloalkoxy group,
      a C₁₋₆ alkyl group substituted with one hydroxy group,
      a C₁₋₆ alkoxy group substituted with one acetamido group, or
      a C₁₋₆ alkoxy group substituted with one C₃₋₆ cycloalkyl group; and
   m is 1 or 2; when m is 2, R^{a}s are the same as or different from each other and when m is 2 and two R^{a}s are adjacent, the two R^{a}s optionally form a methylenedioxy group).
(9) The compound, tautomer of the compound, or pharmaceutically acceptable salt thereof, or solvate thereof according to any one of (1) to (8), in which D is a phenyl group or a 5 to 6-membered heteroaryl group (the phenyl group or the 5 to 6-membered heteroaryl group is unsubstituted or substituted with one or more halogen atoms, one or more nitro groups, one or more C₁₋₆ alkyl groups, one or more C₁₋₆ haloalkyl groups, one or more alkoxy groups, one or more C₁₋₆ haloalkoxy groups (the C₁₋₆ alkyl groups, the C₁₋₆ haloalkyl groups, the C₁₋₆ alkoxy groups, and the C₁₋₆ haloalkoxy groups are unsubstituted or substituted with one or more nitro groups, one or more C₁₋₆ alkoxy groups, or one or more C₁₋₃ haloalkoxy groups), one or more C₁₋₆ alkylsulfonylamino groups, or one or more C₁₋₆ alkylsulfonyloxy groups (the C₁₋₆ alkylsulfonylamino groups and the C₁₋₆ alkylsulfonyloxy groups are unsubstituted or substituted with one or more halogen atoms, one or more nitro groups, one or more alkoxy groups, or one or more C₁₋₃ haloalkoxy groups)).
(10) The compound, tautomer of the compound, or pharmaceutically acceptable salt thereof, or solvate thereof according to (9), in which D is a 5 to 6-membered heteroaryl group (the 5 to 6-membered heteroaryl group is unsubstituted or substituted with one or more halogen atoms, one or more nitro groups, one or more C₁₋₆ alkyl groups, one or more C₁₋₆ haloalkyl groups, one or more C₁₋₆ alkoxy groups, one or more C₁₋₆ haloalkoxy groups (the C₁₋₆ alkyl groups, the C₁₋₆ haloalkyl groups, the C₁₋₆ alkoxy groups, and the C₁₋₆ haloalkoxy groups are unsubstituted or substituted with one or more nitro groups, one or more C₁₋₆ alkoxy groups, or one or more C₁₋₃ haloalkoxy groups), one or more C₁₋₆ alkylsulfonylamino groups, or one or more C₁₋₆ alkylsulfonyloxy groups (the C₁₋₆ alkylsulfonylamino groups and the C₁₋₆ alkylsulfonyloxy groups are unsubstituted or substituted with one or more halogen atoms, one or more nitro groups, one or more C₁₋₆ alkoxy groups, or one or more C₁₋₃ haloalkoxy groups)).
(11) The compound, tautomer of the compound, or pharmaceutically acceptable salt thereof, or solvate thereof according to (10), in which D is a 5-membered heteroaryl group (the 5-membered heteroaryl group is unsubstituted or substituted with one or more C₁₋₆ alkyl groups or one or more C₁₋₆ haloalkyl groups).
(12) The compound, tautomer of the compound, or pharmaceutically acceptable salt thereof, or solvate thereof according to (11), in which D is a thienyl group substituted with a trifluoromethyl group or a thiazolyl group substituted with a trifluoromethyl group.
(13) The compound, tautomer of the compound, or pharmaceutically acceptable salt thereof, or solvate thereof according to (12), in which D is a
   5-trifluoromethylthiophen-2-yl group.
(14) The compound, tautomer of the compound, or pharmaceutically acceptable salt thereof, or solvate thereof according to any one of (1) to (6), in which E is either Formula (IV)-1 or Formula (IV)-2 shown in (IV): 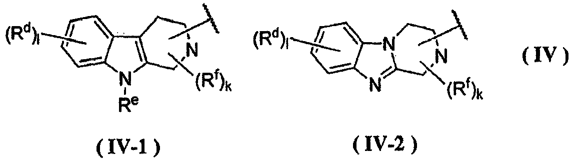 (wherein
   R^{f} means
      a hydrogen atom,
      a hydroxy group,
      an amino group, or
      a halogen atom;
   k is 1;
   R^{e} means
      a hydrogen atom,
      a C₁₋₆ alkyl group, or
      a C₁₋₆ haloalkyl group;
   R^{d} means
      a hydrogen atom,
      a hydroxy group,
      a halogen atom,
      an amino group,
      a C₁₋₆ alkyl group,
      a C₁₋₆ haloalkyl group,
      a C₁₋₆ alkoxy group,
      a C₁₋₆ haloalkoxy group,
      a mono-C₁₋₆ alkylamino group,
      a di-C₁₋₆ alkylamino group, or
      a C₁₋₆ alkylsulfonylamino group; and
   1 is 1 or 2, and when 1 is 2, R^{d}s are the same as or different from each other).
(15) The compound, tautomer of the compound, or pharmaceutically acceptable salt thereof, or solvate thereof according to (14), in which R^{d} is a halogen atom and 1 is 1.
(16) The compound, tautomer of the compound, or pharmaceutically acceptable salt thereof, or solvate thereof according to (14) or (15), in which D is a 5-membered heteroaryl group (the 5-membered heteroaryl group is unsubstituted or substituted with a C₁₋₆ alkyl group or a C₁₋₆ haloalkyl group).
(17) The compound, tautomer of the compound, or pharmaceutically acceptable salt thereof, or solvate thereof according to (16), in which D is a thienyl group substituted with a trifluoromethyl group or a thiazolyl group substituted with a trifluoromethyl group.
(18) The compound, tautomer of the compound, or pharmaceutically acceptable salt thereof, or solvate thereof according to (17), in which D is a
   5-trifluoromethylthiophen-2-yl group.
(19) A T-type calcium channel inhibitor comprising the compound, tautomer of the compound, or pharmaceutically acceptable salt thereof, or solvate thereof as described in any one of (1) to (18), as an active component.
(20) A preventive agent, a therapeutic agent, and/or an improving agent for a disease treatable by a T-type calcium channel inhibitory activity, comprising the T-type calcium channel inhibitor as described in (19) as an active component.
(21) A therapeutic agent for neuropathic pain comprising the T-type calcium channel inhibitor as described in (19) as an active component.
(22) A medicine comprising the compound, tautomer of the compound, or pharmaceutically acceptable salt thereof, or solvate thereof as described in any one of (1) to (18), as an active component.

### Effects of the Invention

The present invention can provide novel triazinone compounds that have an excellent T-type calcium channel inhibitory activity and are specifically useful for prevention or treatment of neuropathic pain.

### MODES FOR CARRYING OUT THE INVENTION

The present invention will now be described in further detail.

In the present invention, "n-" is normal, "i-" is iso, "s-" and "sec-" are secondary, "t-" and "tert-" are tertiary, "o-" is ortho, "m-" is meta, "p-" is para, "(E)-" is an E form, "(Z)-" is a Z form, "(R)-" and "(S)" are an R form and an S form that are optical isomers, "Ph" is phenyl, "Me" is methyl, "Bu" is butyl, "Boc" is tert-butoxycarbonyl, "Cbz" is benzyloxycarbonyl, "Ts" is p-toluenesulfonyl, and "Bn" is benzyl.

First, terms used for the explanation of chemical structures in the present specification will be described.

The "halogen atom" means a fluorine atom, a chlorine atom, a bromine atom, and an iodine atom.

The "C₁₋₃ alkyl group" (an alkyl group having a carbon atom number of 1 to 3) means a methyl group, an ethyl group, a propyl group, and an isopropyl group.

The "C₁₋₆ alkyl group" (an alkyl group having a carbon atom number of 1 to 6) means linear or branched alkyl groups having a carbon atom number of 1 to 6, and specific examples include methyl group, ethyl group, n-propyl group, isopropyl group, n-butyl group, isobutyl group, t-butyl group, n-pentyl group, and n-hexyl group.

The "C₁₋₃ alkylene group" (an alkylene group having a carbon atom number of 1 to 3) means a methylene group, an ethylene group, an ethane-1,1-diyl group, a propane-1,3-diyl group, a propane-1,2-diyl group, a propane-1,1-diyl group, and a propane-2,2-diyl group.

The "C₁₋₆ alkylene group" (an alkylene group having a carbon atom number of 1 to 6) means divalent substituents formed by removing a single hydrogen atom at any position of the "C₁₋₆ alkyl group" defined above, and specific examples include methylene group, ethylene group, an ethane-1,1-diyl group, propane-1,3-diyl group, propane-1,2-diyl group, propane-1,1-diyl group, propane-2,2-diyl group, 2,2-dimethyl-propane-1,3-diyl group, hexane-1,6-diyl group, and 3-methylbutane-1,2-diyl group.

The "C₁₋₃ haloalkyl group" (a haloalkyl group having a carbon atom number of 1 to 3) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₃ alkyl group" defined above, and specific examples include trifluoromethyl group, 2,2,2-trifluoroethyl group, pentafluoroethyl group, and 3-chloro-n-propyl group.

The "C₁₋₆ haloalkyl group" (a haloalkyl group having a carbon atom number of 1 to 6) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₆ alkyl group" defined above, and specific examples include trifluoromethyl group, pentafluoroethyl group, 2,2,2-trifluoro-1,1-dimethyl-ethyl group, 3-chloro-n-propyl group, and 4-chloro-n-butyl group.

The "C₃₋₁₁ cycloalkane" (a cycloalkane group having a carbon atom number of 3 to 11) means monocyclic-, condensed-, bridged-, or spiro-system aliphatic hydrocarbon rings having a ring-constituting carbon atom number of 3 to 11, and specific examples include cyclopropane, cyclobutane, cyclopentane, cyclohexane, cycloheptane, cyclooctane, adamantane, bicyclo[3.1.0]octane, bicyclo[2.2.1]heptane, and spiro[5.5]undecane.

The "C₃₋₁₁ cycloalkyl group" (a cycloalkyl group having a carbon atom number of 3 to 11) means monovalent substituents formed by removing a single hydrogen atom at any position of the "C₃₋₁₁ cycloalkane" defined above.

The "C₃₋₁₁ cycloalkylene group" (a cycloalkylene group having a carbon atom number of 3 to 11) means divalent substituents formed by removing two hydrogen atoms at any positions on different carbons of the "C₃₋₁₁ cycloalkane" defined above.

The "C₃₋₆ cycloalkane" (a cycloalkane group having a carbon atom number of 3 to 6) means cycloalkanes having a ring-constituting carbon atom number of 3 to 6 among the "C₃₋₁₁ cycloalkanes" defined above, and specific examples include cyclopropane, cyclobutane, cyclopentane, and cyclohexane.

The "C₃₋₆ cycloalkyl group" (a cycloalkyl group having a carbon atom number of 3 to 6) means monovalent substituents formed by removing a single hydrogen atom at any position of the "C₃₋₆ cycloalkane" defined above.

The "C₃₋₆ cycloalkylene group" (a cycloalkylene group having a carbon atom number of 3 to 6) means divalent substituents formed by removing two hydrogen atoms at any positions on different carbons of the "C₃₋₆ cycloalkane" defined above.

The "C₃₋₇ cycloalkane" (a cycloalkane group having a carbon atom number of 3 to 7) means cycloalkanes having a ring-constituting carbon atom number of 3 to 7 among the "C₃₋₁₁ cycloalkanes" defined above, and specific examples include cyclopropane, cyclobutane, cyclopentane, cyclohexane, and cycloheptane.

The "1,1-C₃₋₇ cycloalkylene group" (a cycloalkylene group having a 1,1-carbon atom number of 3 to 7) means divalent substituents formed by removing two hydrogen atoms on the same carbon of the "C₃₋₇ cycloalkane" defined above. The group is specifically exemplified by the structures shown in figures below.

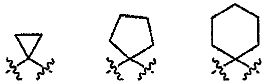

The "C₄₋₇ cycloalkane" (a cycloalkane group having a carbon atom number of 4 to 7) means cycloalkanes having a ring-constituting carbon atom number of 4 to 7 among the "C₃₋₁₁ cycloalkanes" defined above, and specific examples include cyclobutane, cyclopentane, cyclohexane, and cycloheptane.

The "C₄₋₇ cycloalkylene group" (a cycloalkylene group having a carbon atom number of 4 to 7) means divalent substituents formed by removing two hydrogen atoms at any positions on different carbons of the "C₄₋₇ cycloalkane" defined above.

The "C₃₋₁₁ cycloalkene" (a cycloalkene group having a carbon atom number of 3 to 11) means non-aromatic rings formed by replacing one or more of any bonds of the "C₃₋₁₁ cycloalkane" defined above by double bonds, and specific examples include cyclopropene, cyclobutene, cyclopentene, cyclohexene, cyclohexa-1,3-diene, cyclohexa-1,4-diene, bicyclo[2.2.1]hepta-2,5-diene, spiro[2.5]oct-4-ene, and 1,2,5,6-tetrahydronaphthalene.

The "C₃₋₁₁ cycloalkenyl group" (a cycloalkenyl group having a carbon atom number of 3 to 11) means monovalent substituents formed by removing a single hydrogen atom at any position of the "C₃₋₁₁ cycloalkene" defined above.

The "C₃₋₁₁ cycloalkenylene group" (a cycloalkenylene group having a carbon atom number of 3 to 11) means divalent substituents formed by removing two hydrogen atoms at any positions on different carbons of the "C₃₋₁₁ cycloalkene" defined above.

The "C₄₋₇ cycloalkene" (a cycloalkene group having a carbon atom number of 4 to 7) means cycloalkenes having a ring-constituting carbon atom number of 4 to 7 among the "C₃₋₁₁ cycloalkenes" defined above.

The "C₄₋₇ cycloalkenylene group" (a cycloalkenylene group having a carbon atom number of 4 to 7) means divalent substituents formed by removing two hydrogen atoms at any positions on different carbons of the "C₄₋₇ cycloalkene" defined above.

The "C₃₋₆ cycloalkene" (a cycloalkene group having a carbon atom number of 3 to 6) means cycloalkenes having a ring-constituting carbon atom number of 3 to 6 among the "C₃₋₁₁ cycloalkenes" defined above, and specific examples include cyclopropene, cyclobutene, cyclopentene, and cyclohexene.

The "C₃₋₆ cycloalkenylene group" (a cycloalkenylene group having a carbon atom number of 3 to 6) means divalent substituents formed by removing two hydrogen atoms at any positions on different carbons of the "C₃₋₆ cycloalkene" defined above.

The "C₂₋₆ alkenyl group" (an alkenyl group having a carbon atom number of 2 to 6) means linear or branched alkenyl groups having at least one double bond and a carbon atom number of 2 to 6, and specific examples include ethenyl (vinyl) group, 1-propenyl group, 2-propenyl (allyl) group, isopropenyl group, 1-butenyl group, 2-butenyl group, 3-butenyl (homoallyl) group, 4-pentenyl group, and 5-hexenyl group.

The "C₂₋₆ alkenylene group" (an alkenylene group having a carbon atom number of 2 to 6) means divalent substituents formed by removing a single hydrogen atom at any position of the "C₂₋₆ alkenyl group" defined above, and specific examples include ethene-1,1-diyl group, ethene-1,2-diyl group, propene-1,1-diyl group, propene-1,2-diyl group, propene-1,3-diyl group, but-1-ene-1,4-diyl group, but-1-ene-1,3-diyl group, but-2-ene-1,4-diyl group, buta-1,3-diene-1,4-diyl group, pent-2-ene-1,5-diyl group, hex-3-ene-1,6-diyl group, and hexa-2,4-diene-1,6-diyl group.

The "C₂₋₆ haloalkenyl group" (a haloalkenyl group having a carbon atom number of 2 to 6) means substituents formed by substituting one or more halogen atoms identically or differently selected from a substituent group consisting of a fluorine atom, a chlorine atom, a bromine atom, or an iodine atom for one or more hydrogen atoms at any positions of the "C₂₋₆ alkenyl group" defined above.

The "C₂₋₆ alkynyl group" (an alkynyl group having a carbon atom number of 2 to 6) means linear or branched alkynyl groups having at least one triple bond and a carbon atom number of 2 to 6, and specific examples include ethynyl group, 1-propynyl group, 2-propynyl group, 1-butyny group, 2-butyny group, 3-butyny group, 4-pentynyl group, and 5-hexynyl group.

The "C₂₋₆ alkynylene group" (an alkynylene group having a carbon atom number of 2 to 6) means divalent substituents formed by removing a single hydrogen atom at any position of the "C₂₋₆ alkynyl group" defined above. Specific examples of the group include ethyne-1,2-diyl group, propyne-1,3-diyl group, but-1-yne-1,4-diyl group, but-1-yne-1,3-diyl group, but-2-yne-1,4-diyl group, pent-2-yne-1,5-diyl group, pent-2-yne-1,4-diyl group, and hex-3-yne-1,6-diyl group.

The "C₁₋₆ alkoxy group" (an alkoxy group having a carbon atom number of 1 to 6) means linear or branched alkoxy groups having a carbon atom number of 1 to 6, and specific examples include methoxy group, ethoxy group, n-propoxy group, isopropoxy group, n-butoxy group, isobutoxy group, t-butoxy group, n-pentyloxy group, and n-hexyloxy group.

The "C₁₋₁₀ alkoxy group" (an alkoxy group having a carbon atom number of 1 to 10) means linear or branched alkoxy groups having a carbon atom number of 1 to 10, and specific examples include methoxy group, ethoxy group, n-propoxy group, isopropoxy group, n-butoxy group, isobutoxy group, t-butoxy group, n-pentyloxy group, n-hexyloxy group, n-heptyloxy group, n-octyloxy group, n-nonyloxy group, and n-decyloxy group.

The "C₃₋₆ cycloalkoxy group" (a cycloalkoxy group having a carbon atom number of 3 to 6) means groups formed by bonding a single "C₃₋₆ cycloalkyl group" to -O-, and specific examples include cyclopropoxy group, cyclobutoxy group, cyclopentyloxy group, and cyclohexyloxy group.

The "C₁₋₃ alkoxy group" (an alkoxy group having a carbon atom number of 1 to 3) means a methoxy group, an ethoxy group, a n-propoxy group, and an isopropoxy group.

The "C₁₋₆ haloalkoxy group" (a haloalkoxy group having a carbon atom number of 1 to 6) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₆ alkoxy group" defined above, and specific examples include trifluoromethoxy group, pentafluoroethoxy group, 2,2,2-trifluoro-1,1-dimethyl-ethoxy group, 3-chloro-n-propyloxy group, and 4-chloro-n-butoxy group.

The "C₁₋₃ haloalkoxy group" (a haloalkoxy group having a carbon atom number of 1 to 3) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₃ alkoxy group" defined above, and specific examples include trifluoromethoxy group, 2,2,2-trifluoroethoxy group, pentafluoroethoxy group, and 3-chloro-n-propyloxy group.

The "C₆₋₁₄ aromatic hydrocarbon ring" (an aromatic hydrocarbon ring having a carbon atom number of 6 to 14) means monocyclic or polycyclic aromatic hydrocarbon rings in which all the atoms constituting the ring are carbon atoms and the number of carbon atoms is 6 to 14, and includes bicyclic condensed rings composed of a monocyclic aromatic hydrocarbon ring and a monocyclic cycloalkanes or cycloalkenes. Specific examples of the ring include benzene, pentalene, naphthalene, azulene, anthracene, phenanthrene, indene, indane, dihydronaphthalene, and tetrahydronaphthalene.

The "C₆₋₁₄ aryl group" (an aryl group having a carbon atom number of 6 to 14) means monovalent substituents formed by removing a single hydrogen atom at any position on the aromatic ring of the "C₆₋₁₄ aromatic hydrocarbon ring" defined above.

The "C₆₋₁₄ arylene group" (an arylene group having a carbon atom number of 6 to 14) means divalent substituents formed by removing two hydrogen atoms at any positions on the aromatic ring of the "C₆₋₁₄ aromatic hydrocarbon ring" defined above.

The "5 to 10-membered aromatic heterocycle" means monocyclic or condensed aromatic heterocycles having a ring-constituting atom number of 5 to 10 and containing 1 to 5 hetero atoms as the atoms constituting the ring (the hetero atom is a nitrogen atom, an oxygen atom, or a sulfur atom), and specific examples include furan, thiophene, pyrrole, imidazole, triazole, tetrazole, thiazole, pyrazole, oxazole, isoxazole, isothiazole, thiadiazole, oxadiazole, pyridine, pyrazine, pyridazine, pyrimidine, triazine, purine, pteridine, quinoline, isoquinoline, naphthyridine, quinoxaline, cinnoline, quinazoline, phthalazine, imidazopyridine, imidazothiazole, imidazooxazole, benzothiazole, benzoxazole, benzimidazole, indole, isoindole, indazole, pyrrolopyridine, thienopyridine, furopyridine, benzothiadiazole, benzooxadiazole, pyridopyrimidine, benzofuran, benzothiophene, and thienofuran.

When having a C=N double bond, the "5 to 10-membered aromatic heterocycle" includes N-oxides of the aromatic heterocycles.

The "5 to 10-membered aromatic heterocycle containing NH" means aromatic heterocycles having -NH- among the "5 to 10-membered aromatic heterocycles" defined above, and specific examples include pyrrole, imidazole, triazole, tetrazole, pyrazole, purine, benzimidazole, and pyrrolopyridine.

The "5 to 10-membered heteroaryl group" means monovalent substituents formed by removing a single hydrogen atom at any position of the "5 to 10-membered aromatic heterocycle" defined above.

The "5 to 10-membered heteroaryl group containing NH" means heteroaryl groups having -NH- among the "5 to 10-membered heteroaryl groups" defined above.

The "5 to 10-membered heteroarylene group" means divalent substituents formed by removing two hydrogen atoms at any positions of the "5 to 10-membered aromatic heterocycle" defined above.

The "5 to 10-membered heteroarylene group containing NH" means heteroarylene groups having -NH- among the "5 to 10-membered heteroarylene groups" defined above.

The "5 to 6-membered aromatic heterocycle" means monocyclic aromatic heterocycles having a ring-constituting atom number of 5 to 6 among the "5 to 10-membered aromatic heterocycles" defined above, and specific examples include pyrrole, pyrazole, imidazole, triazole, tetrazole, pyridine, pyridazine, pyrimidine, pyrazine, triazine, furan, thiophene, thiazole, isothiazole, oxazole, isoxazole, oxadiazole, and thiadiazole.

The "5 to 6-membered heteroaryl group" means monovalent substituents formed by removing a single hydrogen atom at any position of the "5 to 6-membered aromatic heterocycle" defined above.

The "5-membered aromatic heterocycle" means monocyclic aromatic heterocycles having a ring-constituting atom number of 5 among the "5 to 6-membered aromatic heterocycles" defined above, and specific examples include pyrrole, pyrazole, imidazole, triazole, tetrazole, furan, thiophene, thiazole, isothiazole, oxazole, isoxazole, oxadiazole, and thiadiazole.

The "5-membered heteroaryl group" means monovalent substituents formed by removing a single hydrogen atom at any position of the "5-membered aromatic heterocycle" defined above.

The "3 to 11-membered non-aromatic heterocycle" means a non-aromatic heterocycle that
1) has a ring-constituting atom number of 3 to 11,
2) contains 1 to 5 hetero atoms as the atoms constituting the ring (the hetero atom is a nitrogen atom, an oxygen atom, or a sulfur atom),
3) may contain a methylene group in the ring in which an oxo group, a thioxo group, or a hydroxyimino group may be substituted for the hydrogen atoms of the methylene group,
4) may contain a double bond or a triple bond in the ring,
5) may contain a sulfur atom as the atom constituting the ring, the sulfur atom being optionally replaced by a sulfinyl group or a sulfonyl group, and
6) is monocyclic-, condensed-, (in a condensed non-aromatic heterocycle, a non-aromatic ring is condensed with a non-aromatic ring to form a bicyclic condensed ring), bridged-, or spiro-system non-aromatic heterocycles, and
   specific examples include aziridine, azetidine, pyrrolidine, piperidine, azepane, azocane, tetrahydrofuran, tetrahydropyran, morpholine, thiomorpholine, piperazine, thiazolidine, 1,4-dioxane, imidazoline, and a thiazoline.

The "3 to 11-membered non-aromatic heterocycle containing NH" means non-aromatic heterocycles having NH among the "3 to 11-membered non-aromatic heterocycles" defined above, and specific examples include aziridine, azetidine, pyrrolidine, piperidine, piperazine, morpholine, thiomorpholine, thiazolidine, imidazoline, and thiazoline.

The "3 to 11-membered heterocyclyl group" means monovalent substituents formed by removing a hydrogen atom at any position of the "3 to 11-membered non-aromatic heterocycle" defined above.

The "3 to 11-membered heterocyclyl group containing NH" means heterocyclyl groups having -NH- among the "3 to 11-membered heterocyclyl groups" defined above.

The "3 to 11-membered heterocyclylene group" means divalent substituents formed by removing two hydrogen atoms at any positions on different atoms of the "3 to 11-membered non-aromatic heterocycle" defined above.

The "3 to 11-membered heterocyclylene group containing NH" means heterocyclylene groups having -NH- among the "3 to 11-membered heterocyclylene groups" defined above.

The "4 to 7-membered non-aromatic heterocycle" means a monocyclic non-aromatic heterocycle that
1) has a ring-constituting atom number of 4 to 7,
2) contains 1 to 3 hetero atoms as the atoms constituting the ring (the hetero atom is a nitrogen atom, an oxygen atom, or a sulfur atom),
3) may contain a methylene group in the ring in which an oxo group may be substituted for the hydrogen atoms of the methylene group,
4) may contain a double bond or a triple bond in the ring, and
5) may contain a sulfur atom as the atom constituting the ring, the sulfur atom being optionally replaced by a sulfinyl group or a sulfonyl group, and
   specific examples include azetidine, pyrrolidine, pyrrolidinone, oxazolidine, isoxazolidine, thiazolidine, isothiazolidine, piperazine, piperazinone, piperidine, piperidinone, morpholine, thiomorpholine, oxetane, tetrahydrofuran, 1,3-dioxolane, tetrahydropyran, 1,4-dioxane, oxepane, and homomorpholine.

The "4 to 7-membered heterocyclyl group" means monovalent substituents formed by removing a hydrogen atom at any position of the "4 to 7-membered non-aromatic heterocycle" defined above.

The "4 to 7-membered heterocyclylene group" means divalent substituents formed by removing two hydrogen atoms at any positions on different atoms of the "4 to 7-membered non-aromatic heterocycle" defined above.

The "7 to 14-membered non-aromatic fused heterocycle" means a heterocycle that
1) has a ring-constituting atom number of 7 to 14,
2) is a condensed ring constituted by a non-aromatic ring and an aromatic ring,
3) contains one or more hetero atoms as the atoms constituting the ring (the hetero atom is a nitrogen atom, an oxygen atom, or a sulfur atom),
4) may contain a methylene group in the ring in which an oxo group, a thioxo group, or a hydroxyimino group may be substituted for the hydrogen atoms of the methylene group,
5) may contain a double bond or a triple bond in the ring, and
6) may contain a sulfur atom as the atom constituting the ring, the sulfur atom being optionally replaced by a sulfinyl group or a sulfonyl group, and
   specific examples include 1,2-benzopyran, isochroman, chroman, indoline, isoindoline, indazoline, azaindane, azatetrahydronaphthalene, azachroman, tetrahydrobenzofuran, tetrahydrobenzothiophene, 1,2,3,4-tetrahydroisoquinoline, 1, 2, 3, 4-tetrahydroquinoline, 3,4-dihydro-2H-benzo[b][1,4]dioxepine, indan-1-one, 6,7-dihydro-5H-cyclopentapyrazine, 5,6-dihydro-4H-cyclopenta[b]thiophene, 4,5,6,7-tetrahydro-benzo[b]thiophene, 2,3-dihydro-isoindol-1-one, 3,4-dihydro-2H-isoquinolin-1-one, 3,4-dihydro-2H-benzo[b]oxepin-5-one, 1-H-benzo[d]imidazol-2(3H)-thione, 1,2,3,4-tetrahydroquinoxaline, 5,6,7,8-tetrahydro-1,6-naphthyridine, and 1,2,3,4,5,6-hexahydrobenzo[d]azocine.

The "7 to 14-membered non-aromatic fused heterocycle containing NH" means non-aromatic heterocycles having -NH- at a non-aromatic ring side among the "7 to 14-membered non-aromatic fused heterocycles" defined above, and specific examples include indoline, isoindoline, indazoline, 1,2,3,4-tetrahydroisoquinoline, 1,2,3,4-tetrahydroquinoline, 2,3-dihydro-isoindol-1-one, 3,4-dihydro-2H-isoquinolin-1-one, and 1-H-benzo[d]imidazol-2(3H)-thione.

The "7 to 14-membered non-aromatic fused heterocyclic group" means monovalent substituents formed by removing a single hydrogen atom at any position of the "7 to 14-membered non-aromatic fused heterocycle" defined above.

The "7 to 14-membered non-aromatic fused heterocyclic group containing NH" means monovalent substituents formed by removing a single hydrogen atom at any position of the "7 to 14-membered non-aromatic fused heterocycle containing NH" defined above.

The "9 to 11-membered non-aromatic fused heterocycle" means a fused heterocycle that
1) has a ring-constituting atom number of 9 to 11,
2) is a condensed ring constituted by a non-aromatic ring and an aromatic ring,
3) contains one or more hetero atoms as the atoms constituting the ring (the hetero atom is a nitrogen atom, an oxygen atom, or a sulfur atom),
4) may contain a methylene group in the ring in which an oxo group, a thioxo group, or a hydroxyimino group may be substituted for the hydrogen atoms of the methylene group,
5) may contain a double bond or a triple bond in the ring, and
6) may contain a sulfur atom as the atom constituting the ring, the sulfur atom being optionally replaced by a sulfinyl group or a sulfonyl group, and
   specific examples include 1,2-benzopyran, isochroman, chroman, indoline, isoindoline, indazoline, azaindane, azatetrahydronaphthalene, azachroman, tetrahydrobenzofuran, tetrahydrobenzothiophene, 1,2,3,4-tetrahydroisoquinoline, 1,2,3,4-tetrahydroquinoline, 3,4-dihydro-2H-benzo[b][1,4]dioxepine, indan-1-one, 6,7-dihydro-5H-cyclopentapyrazine, 5,6-dihydro-4H-cyclopenta[b]thiophene, 4,5,6,7-tetrahydro-benzo[b]thiophene, 2,3-dihydro-isoindol-1-one, 3,4-dihydro-2H-isoquinolin-1-one, 3,4-dihydro-2H-benzo[b]oxepin-5-one, 1-H-benzo[d]imidazol-2(3H)-thione, 1,2,3,4-tetrahydroquinoxaline, and 5,6,7,8-tetrahydro-1,6-naphthyridine.

The "9 to 11-membered non-aromatic fused heterocycle containing NH" means non-aromatic heterocycles having -NH- at a non-aromatic ring side among the "9 to 11-membered non-aromatic fused heterocycles" defined above, and specific examples include indoline, isoindoline, indazoline, 1,2,3,4-tetrahydroisoquinoline, 1,2,3,4-tetrahydroquinoline, 2,3-dihydro-isoindol-1-one, 3,4-dihydro-2H-isoquinolin-1-one, and 1-H-benzo[d]imidazol-2(3H)-thione.

The "9 to 11-membered non-aromatic fused heterocyclic group" means monovalent substituents formed by removing a single hydrogen atom at any position of the "9 to 11-membered non-aromatic fused heterocycle" defined above.

The "9 to 11-membered non-aromatic fused heterocyclic group containing NH" means monovalent substituents formed by removing a single hydrogen atom at any position of the "9 to 11-membered non-aromatic fused heterocycle containing NH" defined above.

The phrase "two substituents form a ring together" means that adjacent substituents are bonded each other to form a 5 or 6-membered ring. Specific examples of the formed ring include 1,3-dioxolane and 1,4-dioxane.

The "C₁₋₆ alkylthio group" (an alkylthio group having a carbon atom number of 1 to 6) means groups formed by bonding the single "C₁₋₆ alkyl group" to -S-, and specific examples include methylthio group, ethylthio group, n-propylthio group, isopropylthio group, n-butylthio group, isobutylthio group, t-butylthio group, n-pentylthio group, and n-hexylthio group.

The "C₃₋₆ cycloalkylthio group" (a cycloalkylthio group having a carbon atom number of 3 to 6) means groups formed by bonding the single "C₃₋₆ cycloalkyl group" to -S-, and specific examples include cyclopropylthio group, cyclobutylthio group, cyclopentylthio group, and cyclohexylthio group.

The "C₁₋₆ haloalkylthio group" (a haloalkylthio group having a carbon atom number of 1 to 6) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₆ alkylthio group" defined above.

The "C₁₋₆ alkylsulfonyl group" (an alkylsulfonyl group having a carbon atom number of 1 to 6) means groups formed by bonding the single "C₁₋₆ alkyl group" to a sulfonyl group, and specific examples include methylsulfonyl group, ethylsulfonyl group, n-propylsulfonyl group, isopropylsulfonyl group, n-butylsulfonyl group, isobutylsulfonyl group, t-butylsulfonyl group, n-pentylsulfonyl group, and n-hexylsulfonyl group.

The "C₁₋₆ alkylsulfonylamino group" (an alkylsulfonylamino group having a carbon atom number of 1 to 6) means groups formed by bonding the single "C₁₋₆ alkylsulfonyl group" to -NH-, and specific examples include methylsulfonylamino group, ethylsulfonylamino group, n-propylsulfonylamino group, isopropylsulfonylamino group, n-butylsulfonylamino group, isobutylsulfonylamino group, t-butylsulfonylamino group, n-pentylsulfonylamino group, and n-hexylsulfonylamino group.

The "C₁₋₆ alkylsulfonyloxy group" (an alkylsulfonyloxy group having a carbon atom number of 1 to 6) means groups formed by bonding the single "C₁₋₆ alkylsulfonyl group" to -O-, and specific examples include methylsulfonyloxy group, ethylsulfonyloxy group, n-propylsulfonyloxy group, isopropylsulfonyloxy group, n-butylsulfonyloxy group, isobutylsulfonyloxy group, t-butylsulfonyloxy group, n-pentylsulfonyloxy group, and n-hexylsulfonyloxy group.

The "C₁₋₆ haloalkylsulfonyloxy group" (a haloalkylsulfonyloxy group having a carbon atom number of 1 to 6) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₆ alkylsulfonyloxy group" defined above, and specific examples include trifluoromethylsulfonyloxy group and trichloromethylsulfonyloxy group.

The "C₃₋₆ cycloalkylsulfonyl group" (a cycloalkylsulfonyl group having a carbon atom number of 3 to 6) means groups formed by bonding the single "C₃₋₆ cycloalkyl group" to a sulfonyl group, and specific examples include cyclopropylsulfonyl group, cyclobutylsulfonyl group, cyclopentylsulfonyl group, and cyclohexylsulfonyl group.

The "C₃₋₆ cycloalkylsulfonylamino group" (a cycloalkylsulfonylamino group having a carbon atom number of 3 to 6) means groups formed by bonding the single "C₃₋₆ cycloalkylsulfonyl group" to -NH-, and specific examples include cyclopropylsulfonylamino group, cyclobutylsulfonylamino group, cyclopentylsulfonylamino group, and cyclohexylsulfonylamino group.

The "C₃₋₆ cycloalkylsulfonyloxy group" (a cycloalkylsulfonyloxy group having a carbon atom number of 3 to 6) means groups formed by bonding the single "C₃₋₆ cycloalkylsulfonyl group" to -O-, and specific examples include cyclopropylsulfonyloxy group, cyclobutylsulfonyloxy group, cyclopentylsulfonyloxy group, and cyclohexylsulfonyloxy group.

The "C₁₋₆ haloalkylsulfonyl group" (a haloalkylsulfonyl group having a carbon atom number of 1 to 6) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₆ alkylsulfonyl group" defined above.

The "C₁₋₆ alkoxycarbonyl group" (an alkoxycarbonyl group having a carbon atom number of 1 to 6) means groups formed by bonding the single "C₁₋₆ alkoxy group" to a carbonyl group, and specific examples include methoxycarbonyl group, ethoxycarbonyl group, n-propoxycarbonyl group, isopropoxycarbonyl group, n-butoxycarbonyl group, isobutoxycarbonyl group, t-butoxycarbonyl group, n-pentyloxycarbonyl group, and n-hexyloxycarbonyl group.

The "mono-C₁₋₆ alkylamino group" (a mono-alkyl-amino group having a carbon atom number of 1 to 6) means groups formed by bonding the single "C₁₋₆ alkyl group" to -NH-, and specific examples include methylamino group, ethylamino group, n-propylamino group, isopropylamino group, n-butylamino group, isobutylamino group, t-butylamino group, n-pentylamino group, and n-hexylamino group.

The "di-C₁₋₆ alkylamino group" (a di-alkyl-amino group having a carbon atom number of 1 to 6) means groups formed by bonding the two "C₁₋₆ alkyl groups", which may be the same as or different from each other, to -N-, and specific examples include dimethylamino group, diethylamino group, di-n-propylamino group, diisopropylamino group, di-n-butylamino group, diisobutylamino group, di-t-butylamino group, di-n-pentylamino group, di-n-hexylamino group, N-ethyl-N-methylamino group, N-methyl-N-n-propylamino group, N-isopropyl-N-methylamino group, N-n-butyl-N-methylamino group, N-isobutyl-N-methylamino group, N-t-butyl-N-methylamino group, N-methyl-N-n-pentylamino group, N-n-hexyl-N-methylamino group, N-ethyl-N-n-propylamino group, N-ethyl-N-isopropylamino group, N-n-butyl-N-ethylamino group, N-ethyl-N-isobutylamino group, N-t-butyl-N-ethylamino group, N-ethyl-N-n-pentylamino group, and N-ethyl-N-n-hexylamino group.

The "mono-C₃₋₆ cycloalkylamino group" (a mono-cycloalkyl-amino group having a carbon atom number of 3 to 6) means groups formed by bonding the single "C₃₋₆ cycloalkyl group" to -NH-, and specific examples include cyclopropylamino group, cyclobutylamino group, cyclopentylamino group, and cyclohexylamino group.

The "di-C₃₋₆ cycloalkylamino group" (a di-cycloalkyl-amino group having a carbon atom number of 3 to 6) means groups formed by bonding the two "C₃₋₆ cycloalkyl groups", which may be the same as or different from each other, to -N-, and specific examples include dicyclopropylamino group, dicyclobutylamino group, dicyclopentylamino group, and dicyclohexylamino group.

The "C₁₋₆ alkylcarbonyl group" (an alkylcarbonyl group having a carbon atom number of 1 to 6) means groups formed by bonding the single "C₁₋₆ alkyl group" to a carbonyl group, and specific examples include acetyl group, propionyl group, butyryl group, isobutyryl group, pentanoyl group, 3-methylbutanoyl group, pivaloyl group, hexanoyl group, and heptanoyl group.

The "C₃₋₆ cycloalkylcarbonyl group" (a cycloalkylcarbonyl group having a carbon atom number of 3 to 6) means groups formed by bonding the single "C₃₋₆ cycloalkyl group" to a carbonyl group, and specific examples include cyclopropylcarbonyl group, cyclobutylcarbonyl group, cyclopentylcarbonyl group, cyclohexylcarbonyl group, and cycloheptylcarbonyl group.

The "C₁₋₆ haloalkylcarbonyl group" (a haloalkylcarbonyl group having a carbon atom number of 1 to 6) means substituents formed by substituting one or more halogen atoms identically or differently selected from a substituent group consisting of a fluorine atom, a chlorine atom, a bromine atom, and an iodine atom for one or more hydrogen atoms at any positions of the "C₁₋₆ alkylcarbonyl group" defined above.

The "mono-C₁₋₆ alkylaminocarbonyl group" (a mono-alkyl-amino-carbonyl group having a carbon atom number of 1 to 6) means groups formed by bonding the single "mono-C₁₋₆ alkylamino group" to a carbonyl group, and specific examples include methylaminocarbonyl group, ethylaminocarbonyl group, n-propylaminocarbonyl group, isopropylaminocarbonyl group, n-butylaminocarbonyl group, isobutylaminocarbonyl group, t-butylaminocarbonyl group, n-pentylaminocarbonyl group, and n-hexylaminocarbonyl group.

The "di-C₁₋₆ alkylaminocarbonyl group" (a di-alkyl-amino-carbonyl group having a carbon atom number of 1 to 6) means groups formed by bonding the single "di-C₁₋₆ alkylamino group" to a carbonyl group, and specific examples include dimethylaminocarbonyl group, diethylaminocarbonyl group, di-n-propylaminocarbonyl group, diisopropylaminocarbonyl group, di-n-butylaminocarbonyl group, diisobutylaminocarbonyl group, di-t-butylaminocarbonyl group, di-n-pentylaminocarbonyl group, di-n-hexylaminocarbonyl group, N-ethyl-N-methylaminocarbonyl group, N-methyl-N-n-propylaminocarbonyl group, N-isopropyl-N-methylaminocarbonyl group, N-n-butyl-N-methylaminocarbonyl group, N-isobutyl-N-methylaminocarbonyl group, N-t-butyl-N-methylaminocarbonyl group, N-methyl-N-n-pentylaminocarbonyl group, N-n-hexyl-N-methylaminocarbonyl group, N-ethyl-N-n-propylaminocarbonyl group, N-ethyl-N-isopropylaminocarbonyl group, N-n-butyl-N-ethylaminocarbonyl group, N-ethyl-N-isobutylaminocarbonyl group, N-t-butyl-N-ethylaminocarbonyl group, N-ethyl-N-n-pentylaminocarbonyl group, and N-ethyl-N-n-hexylaminocarbonyl group.

The "C₁₋₆ alkylcarbonylamino group" (an alkylcarbonylamino group having a carbon atom number of 1 to 6) means groups formed by bonding the single "C₁₋₆ alkylcarbonyl group" to -NH-, and specific examples include methylcarbonylamino group, ethylcarbonylamino group, n-propylcarbonylamino group, isopropylcarbonylamino group, n-butylcarbonylamino group, isobutylcarbonylamino group, t-butylcarbonylamino group, n-pentylcarbonylamino group, and n-hexylcarbonylamino group.

The "C₁₋₆ alkylcarbonyloxy group" (an alkylcarbonyloxy group having a carbon atom number of 1 to 6) means groups formed by bonding the single "C₁₋₆ alkylcarbonyl group" to -O-, and specific examples include methylcarbonyloxy group, ethylcarbonyloxy group, n-propylcarbonyloxy group, isopropylcarbonyloxy group, n-butylcarbonyloxy group, isobutylcarbonyloxy group, t-butylcarbonyloxy group, n-pentylcarbonyloxy group, and n-hexylcarbonyloxy group.

The "mono-C₁₋₆ alkylaminosulfonyl group" (a mono-alkyl-amino-sulfonyl group having a carbon atom number of 1 to 6) means groups formed by bonding the single "mono-C₁₋₆ alkylamino group" to a sulfonyl group, and specific examples include methylaminosulfonyl group, ethylaminosulfonyl group, n-propylaminosulfonyl group, isopropylaminosulfonyl group, n-butylaminosulfonyl group, isobutylaminosulfonyl group, t-butylaminosulfonyl group, n-pentylaminosulfonyl group, and n-hexylaminosulfonyl group.

The "di-C₁₋₆ alkylaminosulfonyl group" (a di-alkyl-amino-sulfonyl group having a carbon atom number of 1 to 6) means groups formed by bonding the single "di-C₁₋₆ alkylamino group" to a sulfonyl group, and specific examples include dimethylaminosulfonyl group, diethylaminosulfonyl group, di-n-propylaminosulfonyl group, diisopropylaminosulfonyl group, di-n-butylaminosulfonyl group, diisobutylaminosulfonyl group, di-t-butylaminosulfonyl group, di-n-pentylaminosulfonyl group, di-n-hexylaminosulfonyl group, N-ethyl-N-methylaminosulfonyl group, N-methyl-N-n-propylaminosulfonyl group, N-isopropyl-N-methylaminosulfonyl group, N-n-butyl-N-methylaminosulfonyl group, N-isobutyl-N-methylaminosulfonyl group, N-t-butyl-N-methylaminosulfonyl group, N-methyl-N-n-pentylaminosulfonyl group, N-n-hexyl-N-methylaminosulfonyl group, N-ethyl-N-n-propylaminosulfonyl group, N-ethyl-N-isopropylaminosulfonyl group, N-n-butyl-N-ethylaminosulfonyl group, N-ethyl-N-isobutylaminosulfonyl group, N-t-butyl-N-ethylaminosulfonyl group, N-ethyl-N-n-pentylaminosulfonyl group, and N-ethyl-N-n-hexylaminosulfonyl group.

The "C₆₋₁₄ arylcarbonyl group" (an arylcarbonyl group having a carbon atom number of 6 to 14) means groups formed by bonding the single "C₆₋₁₄ aryl group" to a carbonyl group, and specific examples include phenylcarbonyl group, 1-naphthylcarbonyl group, and 2-naphthylcarbonyl group.

The "C₆₋₁₄ arylcarbonyloxy group" (an arylcarbonyloxy group having a carbon atom number of 6 to 14) means groups formed by bonding the single "C₆₋₁₄ arylcarbonyl group" to an oxy group, and specific examples include phenylcarbonyloxy group, a 1-naphthylcarbonyloxy group, and 2-naphthylcarbonyloxy group.

The "C₆₋₁₄ aryloxy group" (an aryloxy group having a carbon atom number of 6 to 14) means groups formed by bonding the single "C₆₋₁₄ aryl group" to -O-, and specific examples include phenoxy group, 1-naphthyloxy group, and 2-naphthyloxy group.

Preferred structures for each substituent in the present invention will be described next.

The substituent R¹ is preferably a hydrogen atom, a halogen atom, a C₁₋₆ alkoxy group, a mono-C₁₋₆ alkylamino group, a di-C₁₋₆ alkylamino group, or a C₁₋₆ alkyl group (the C₁₋₆ alkoxy group, the mono-C₁₋₆ alkylamino group, the di-C₁₋₆ alkylamino group, and the C₁₋₆ alkyl group are unsubstituted or substituted with one or more substituents identically or differently selected from a substituent group V⁷).

The substituent group V⁷ means a substituent group consisting of a hydroxy group, halogen atoms, a cyano group, a nitro group, C₁₋₆ alkoxy groups, C₁₋₆ alkylthio groups, C₁₋₆ alkoxycarbonyl groups (the C₁₋₆ alkoxy groups, the C₁₋₆ alkylthio groups, and the C₁₋₆ alkoxycarbonyl groups are unsubstituted or substituted with one or more substituents identically or differently selected from the substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, C₃₋₆ cycloalkyl groups, 4 to 7-membered heterocyclyl groups, a phenyl group, and 5 to 6-membered heteroaryl groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the C₃₋₆ cycloalkyl groups, the 4 to 7-membered heterocyclyl groups, the phenyl group, and the 5 to 6-membered heteroaryl groups are unsubstituted or substituted with one or more substituents identically or differently selected from the substituent group V²);

The substituent R¹ is more preferably a hydrogen atom, a C₁₋₆ alkyl group, or a C₁₋₆ alkoxy group (the C₁₋₆ alkyl group and the C₁₋₆ alkoxy group are unsubstituted or substituted with one or more halogen atoms).

The substituent R¹ is further more preferably a hydrogen atom or a methyl group.

E is a 7 to 14-membered non-aromatic fused heterocyclic group (the 7 to 14-membered non-aromatic fused heterocyclic group is bonded to a carbon atom of a triazine skeleton in Formula (I) at a non-aromatic ring side; the non-aromatic ring side is unsubstituted or substituted with one or more substituents identically or differently selected from a substituent group V⁹; an oxo group, a thioxo group or hydroxyimino group is optionally substituted for hydrogen atoms of a methylene group in the non-aromatic ring; and an aromatic ring side is unsubstituted or substituted with one or more substituents identically or differently selected from the substituent group V⁶ (when the aromatic ring side has two or more substituents, the two substituents optionally form a ring together)).

E is more preferably a 9 to 11-membered non-aromatic fused heterocyclic group (the 9 to 11-membered non-aromatic fused heterocyclic group is bonded to a carbon atom of a triazine skeleton in Formula (I) at a non-aromatic ring side; the non-aromatic ring side is unsubstituted or substituted with one or more substituents identically or differently selected from the substituent group V⁹; an oxo group, a thioxo group, or a hydroxyimino group is optionally substituted for the hydrogen atoms of a methylene group in the non-aromatic ring; and an aromatic ring side is unsubstituted or substituted with one or more substituents identically or differently selected from the substituent group V⁶ (when the aromatic ring side has two or more substituents, the two substituents optionally form a ring together)).

E is further more preferably any one of Formula (II)-1 to Formula (II)-11 shown in (II): 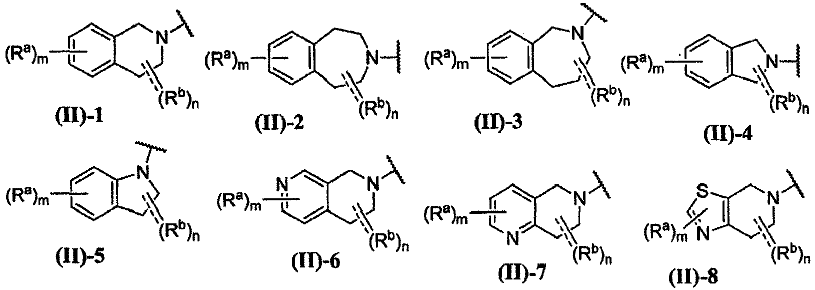 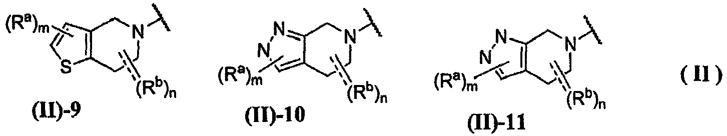 (where
R^{b} means
   a hydrogen atom,
   a hydroxy group,
   an amino group,
   a halogen atom,
   a C₁₋₆ alkoxycarbonyl group,
   an oxo group, or
   a hydroxyimino group;
n is 1;
R^{a} means a hydrogen atom,
   a cyano group,
   a halogen atom,
   a hydroxy group,
   an amino group,
   a C₁₋₆ alkyl group,
   a C₃₋₆ cycloalkyl group,
   a C₁₋₆ alkoxy group,
   a C₁₋₆ haloalkylsulfonyloxy group,

   a C₁₋₆ haloalkyl group,
   a C₁₋₆ haloalkoxy group,
   a C₁₋₆ alkyl group substituted with one hydroxy group,
   a C₁₋₆ alkoxy group substituted with one acetamido group,
   a C₁₋₆ alkoxy group substituted with one C₃₋₆ cycloalkyl group; and
m is 1 or 2; when m is 2, R^{a}s are the same as or different from each other and when m is 2 and two R^{a}s are adjacent, the two R^{a}s optionally form a methylenedioxy group).
E is particularly preferably Formula (III) 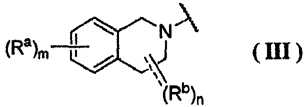 (wherein
   R^{b} means
      a hydrogen atom,
      a hydroxy group,
      an amino group,
      a halogen atom,
      a C₁₋₆ alkoxycarbonyl group,
      an oxo group, or
      a hydroxyimino group;
   n is 1;
   R^{a} means a hydrogen atom,
      a cyano group,
      a halogen atom,
      a hydroxy group,
      an amino group,
      a C₁₋₆ alkyl group,
      a C₃₋₆ cycloalkyl group,
      a C₁₋₆ alkoxy group,
      a C₁₋₆ haloalkylsulfonyloxy group,
      a C₁₋₆ haloalkyl group,
      a C₁₋₆ haloalkoxy group,
      a C₁₋₆ alkyl group substituted with one hydroxy group,
      a C₁₋₆ alkoxy group substituted with one acetamido group,
      a C₁₋₆ alkoxy group substituted with one C₃₋₆ cycloalkyl group; and
   m is 1 or 2; when m is 2, R^{a}s are the same as or different from each other and when m is 2 and two R^{a}s are adjacent, the two R^{a}s optionally form a methylenedioxy group).

As other preferable structures, E is more preferably either (IV)-1 or (IV)-2 shown in (IV): 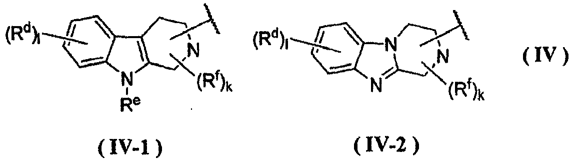 (wherein
R^{f} means
   a hydrogen atom,
   a hydroxy group,
   an amino group, or
   a halogen atom;
k is 1;
R^{e} means
   a hydrogen atom,
   a C₁₋₆ alkyl group, or
   a C₁₋₆ haloalkyl group;
R^{d} means
   a hydrogen atom,
   a hydroxy group,
   a halogen atom,
   an amino group,
   a C₁₋₆ alkyl group,
   a C₁₋₆ haloalkyl group,
   a C₁₋₆ alkoxy group,
   a C₁₋₆ haloalkoxy group,
   a mono-C₁₋₆ alkylamino group,
   a di-C₁₋₆ alkylamino group, or
   a C₁₋₆ alkylsulfonylamino group; and
1 is 1 or 2, and when 1 is 2, R^{d}s are the same as or different from each other).
E is particularly preferably either (V)-1 or (V)-2 shown in (V).

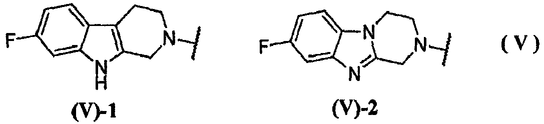

L³ is preferably a C₁₋₃ alkylene group (the C₁₋₃ alkylene group is unsubstituted or substituted with one or more substituents identically or differently selected from a substituent group V³).

The substituent group V³ is a substituent group consisting of a hydroxy group, halogen atoms, a cyano group, a nitro group, C₃₋₆ cycloalkyl groups, C₁₋₆ alkoxy groups, and C₁₋₆ haloalkoxy groups.

L³ is more preferably a methylene group.

D is preferably a C₆₋₁₄ aryl group, a 5 to 10-membered heteroaryl group, or a 7 to 14-membered non-aromatic fused heterocyclic group (the C₆₋₁₄ aryl group, the 5 to 10-membered heteroaryl group, and the 7 to 14-membered non-aromatic fused heterocyclic group are unsubstituted or substituted with one or more substituents identically or differently selected from a substituent group V⁴).

D is more preferably a phenyl group, a 5 to 10-membered heteroaryl group, or a 9 to 11-membered non-aromatic fused heterocyclic group (the phenyl group, the 5 to 10-membered heteroaryl group, and the 9 to 11-membered non-aromatic fused heterocyclic group are unsubstituted or substituted with one or more substituents identically or differently selected from the substituent group V⁴).

The substituent group V⁴ is a substituent group consisting of a hydroxy group, halogen atoms, a cyano group, a nitro group, C₁₋₆ alkyl groups, C₁₋₆ haloalkyl groups, C₁₋₆ alkoxy groups, C₁₋₆ haloalkoxy groups, C₃₋₆ cycloalkyl groups, C₃₋₆ cycloalkoxy groups, mono-C₃₋₆ cycloalkylamino groups, di-C₃₋₆ cycloalkylamino groups, and C₃₋₆ cycloalkylthio groups (the C₁₋₆ alkyl groups, the C₁₋₆ alkoxy groups, the C₃₋₆ cycloalkyl groups, the C₃₋₆ cycloalkoxy groups, the mono-C₃₋₆ cycloalkylamino groups, the di-C₃₋₆ cycloalkylamino groups, and the C₃₋₆ cycloalkylthio groups are unsubstituted or substituted with one or more hydroxy groups, one or more amino groups, one or more nitro groups, one or more cyano groups, one or more 3 to 11-membered heterocyclyl groups, one or more C₆₋₁₄ aryl groups, or one or more 5 to 10-membered heteroaryl groups (the 3 to 11-membered heterocyclyl groups, the C₆₋₁₄ aryl groups, and the 5 to 10-membered heteroaryl groups are unsubstituted or substituted with one or more carboxy groups, one or more carbamoyl groups, one or more sulfamoyl groups, one or more phosphono groups, one or more sulfo groups, one or more tetrazolyl groups, one or more formyl groups, one or more nitro groups, one or more cyano groups, one or more halogen atoms, one or more hydroxy groups, one or more amino groups, one or more C₁₋₆ alkyl groups, one or more C₁₋₃ haloalkyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di-C₁₋₆ alkylaminocarbonyl groups, one or more C₁₋₆ alkylcarbonylamino groups, one or more C₁₋₆ alkylthio groups, or one or more C₁₋₆ alkylsulfonyl groups)).

D is further preferably a phenyl group or a 5 to 6-membered heteroaryl group (the phenyl group and the 5 to 6-membered heteroaryl group are unsubstituted or substituted with one or more substituents identically or differently selected from the substituent group V⁴).

D is particularly preferably any one of (VI)-1 to (VI)-4 shown in (VI): 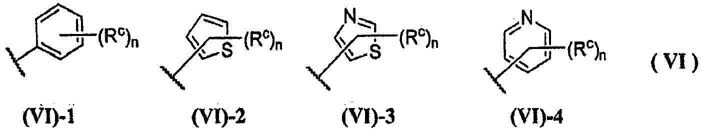 (where n is 0 to 3; R^{c} means a substituent selected from the substituent group V⁴; and when n is 2 or 3, R^{c}s are the same as or different from each other).

D is most preferably either (VII)-1 or (VII)-2 shown in (VII): 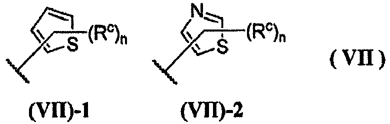 (where n is 1; and R^{c} is a C₁₋₆ alkyl group or a C₁₋₆ haloalkyl group).

Compounds preferably used for the T-type calcium channel inhibitor and for the preventive agent, the therapeutic agent, and/or the improving agent for a disease treatable by a T-type calcium channel inhibitory activity of the present invention are shown below.
1) A compound of Formula (I) 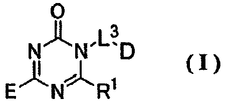 [wherein
R¹ means
   a hydrogen atom,
   a halogen atom,
   a C₁₋₆ alkyl group,
   a C₁₋₆ alkoxy group,
   a C₁₋₆ alkylthio group,
   a mono-C₁₋₆ alkylamino group,
   a di-C₁₋₆ alkylamino group,
   (the C₁₋₆ alkyl group, the C₁₋₆ alkoxy group, the C₁₋₆ alkylthio group, the mono-C₁₋₆ alkylamino group, and the di-C₁₋₆ alkylamino group are unsubstituted or substituted with one or more substituents identically or differently selected from the substituent group V⁸), or
   a C₃₋₁₁ cycloalkyl group
   (the C₃₋₁₁ cycloalkyl group is unsubstituted or substituted with one or more substituents identically or differently selected from the substituent group V⁶);
E means
   a 7 to 14-membered non-aromatic fused heterocyclic group
   (the 7 to 14-membered non-aromatic fused heterocyclic group is bonded at a non-aromatic ring side and a carbon atom of a triazine skeleton in Formula (I) and the non-aromatic ring side are unsubstituted or substituted with one or more substituents identically or differently selected from the substituent group V⁹; an oxo group, a thioxo group, or a hydroxyimino group is optionally substituted for hydrogen atoms of a methylene group in the non-aromatic ring; and an aromatic ring side is unsubstituted or substituted with one or more substituents identically or differently selected from the substituent group V⁶ (when the aromatic ring side has two or more substituents, the two substituents optionally form a ring together);
L³ means
   a C₁₋₆ alkylene group,
   (the C₁₋₆ alkylene group is unsubstituted or substituted with one or more substituents identically or differently selected from the substituent group V⁸ and one methylene group in the C₁₋₆ alkylene group is optionally replaced by a carbonyl group (>C=O) or a thiocarbonyl group (>C=S));
D is
   a C₆₋₁₄ aryl group,
   a 5 to 10-membered heteroaryl group, or
   a 7 to 14-membered non-aromatic fused heterocyclic group
   (the C₆₋₁₄ aryl group, the 5 to 10-membered heteroaryl group, and the 7 to 14-membered non-aromatic fused heterocyclic group are unsubstituted or substituted with one or more substituents identically or differently selected from the substituent group V⁶)], a tautomer of the compound, or a pharmaceutically acceptable salt thereof, or a solvate thereof.

2) The compound, tautomer of the compound, or pharmaceutically acceptable salt thereof, or solvate thereof according to 1), in which L³ is a C₁₋₃ alkylene group (the C₁₋₃ alkylene group is unsubstituted or substituted with one or more substituents identically or differently selected from the substituent group V⁷).
3) The compound, tautomer of the compound, or pharmaceutically acceptable salt thereof, or solvate thereof according to 2), in which L³ is a methylene group.
4) The compound, tautomer of the compound, or pharmaceutically acceptable salt thereof, or solvate thereof according to any one of 1) to 3), in which E is a 9 to 11-membered non-aromatic fused heterocyclic group (the 9 to 11-membered non-aromatic fused heterocyclic group is bonded to a carbon atom of a triazine skeleton in Formula (I) at a non-aromatic ring side; the non-aromatic ring side is unsubstituted or substituted with one or more substituents identically or differently selected from the substituent group V⁹; an oxo group, a thioxo group, or a hydroxyimino group is optionally substituted for hydrogen atoms of a methylene group in the non-aromatic ring; and an aromatic ring side is unsubstituted or substituted with one or more substituents identically or differently selected from the substituent group V⁶ (when the aromatic ring side has two or more substituents, the two substituents optionally form a ring together).
5) The compound, tautomer of the compound, or pharmaceutically acceptable salt thereof, or solvate thereof according to any one of (1) to (4), in which E is any one of Formula (II)-1 to Formula (II)-11 shown in (II): 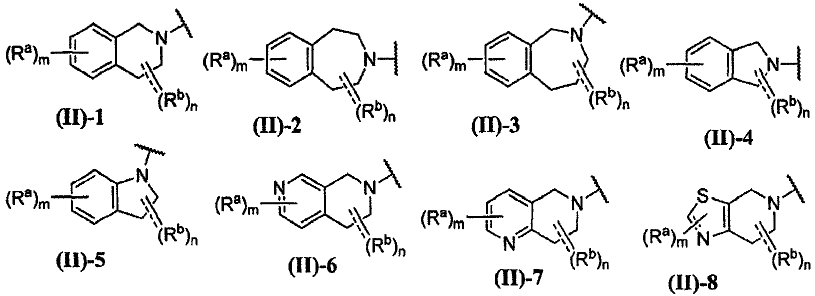 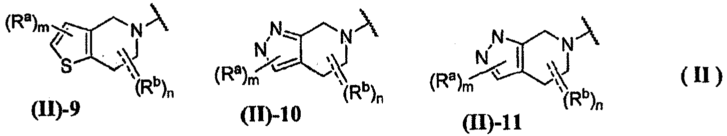 (where
R^{b} means
   a hydrogen atom,
   a hydroxy group,
   an amino group,
   a halogen atom,
   a C₁₋₆ alkoxycarbonyl group,
   an oxo group, or
   a hydroxyimino group;
n is 1;
R^{a} means a hydrogen atom,
   a cyano group,
   a halogen atom,
   a hydroxy group,
   an amino group,
   a C₁₋₆ alkyl group,
   a C₃₋₆ cycloalkyl group,
   a C₁₋₆ alkoxy group,
   a C₁₋₆ haloalkylsulfonyloxy group,
   a C₁₋₆ haloalkyl group,
   a C₁₋₆ haloalkoxy group,
   a C₁₋₆ alkyl group substituted with one hydroxy group,
   a C₁₋₆ alkoxy group substituted with one acetamido group, or
   a C₁₋₆ alkoxy group substituted with one C₃₋₆ cycloalkyl group; and
m is 1 or 2; when m is 2, R^{a}s are the same as or different from each other and when m is 2 and two R^{a}s are adjacent, the two R^{a}s optionally form a methylenedioxy group).

6) The compound, tautomer of the compound, or pharmaceutically acceptable salt thereof, or solvate thereof according to 5), in which E is Formula (III) 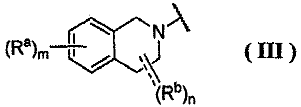 (wherein
R^{b} means
   a hydrogen atom,
   a hydroxy group,
   an amino group,
   a halogen atom,
   a C₁₋₆ alkoxycarbonyl group,
   an oxo group, or
   a hydroxyimino group;
n is 1;
R^{a} means a hydrogen atom,
   a cyano group,
   a halogen atom,
   a hydroxy group,
   an amino group,
   a C₁₋₆ alkyl group,
   a C₃₋₆ cycloalkyl group,
   a C₁₋₆ alkoxy group,
   a C₁₋₆ haloalkylsulfonyloxy group,
   a C₁₋₆ haloalkyl group,
   a C₁₋₆ haloalkoxy group,
   a C₁₋₆ alkyl group substituted with one hydroxy group,
   a C₁₋₆ alkoxy group substituted with one acetamido group, or
   a C₁₋₆ alkoxy group substituted with one C₃₋₆ cycloalkyl group; and
m is 1 or 2; when m is 2, R^{a}s are the same as or different from each other and when m is 2 and two R^{a}s are adjacent, the two R^{a}s optionally form a methylenedioxy group).

7) The compound, tautomer of the compound, or pharmaceutically acceptable salt thereof, or solvate thereof according to any one of 1) to 6), in which D is a C₆₋₁₄ aryl group, a 5 to 10-membered heteroaryl group, or a 7 to 14-membered non-aromatic fused heterocyclic group (the C₆₋₁₄ aryl group, the 5 to 10-membered heteroaryl group, and the 7 to 14-membered non-aromatic fused heterocyclic group are unsubstituted or substituted with one or more substituents identically or differently selected from the substituent group V⁴).
8) The compound, tautomer of the compound, or pharmaceutically acceptable salt thereof, or solvate thereof according to 7), in which D is a phenyl group, a 5 to 10-membered heteroaryl group, or a 9 to 11-membered non-aromatic fused heterocyclic group (the phenyl group, the 5 to 10-membered heteroaryl group, and the 9 to 11-membered non-aromatic fused heterocyclic group are unsubstituted or substituted with one or more substituents identically or differently selected from the substituent group V⁴).
9) The compound, tautomer of the compound, or pharmaceutically acceptable salt thereof, or solvate thereof according to 8), in which D is a phenyl group or a 5 to 6-membered heteroaryl group (the phenyl group and the 5 to 6-membered heteroaryl group have one or more substituents identically or differently selected from the substituent group V⁴).
10) The compound, tautomer of the compound, or pharmaceutically acceptable salt thereof, or solvate thereof according to 9), in which D is a phenyl group (the phenyl group has one or more substituents identically or differently selected from the substituent group V⁴).
11) The compound, tautomer of the compound, or pharmaceutically acceptable salt thereof, or solvate thereof according to 9), in which D is a 5 to 6-membered heteroaryl group (the 5 to 6-membered heteroaryl group is unsubstituted or substituted with one or more halogen atoms, one or more nitro groups, one or more C₁₋₆ alkyl groups, one or more C₁₋₆ haloalkyl groups, one or more C₁₋₆ alkoxy groups, one or more C₁₋₆ haloalkoxy groups (the C₁₋₆ alkyl groups, the C₁₋₆ haloalkyl groups, the C₁₋₆ alkoxy groups, and C₁₋₆ haloalkoxy groups are unsubstituted or substituted with one or more nitro groups, one or more C₁₋₆ alkoxy groups, or one or more C₁₋₃ haloalkoxy groups), one or more C₁₋₆ alkylsulfonylamino groups, or one or more C₁₋₆ alkylsulfonyloxy groups (the C₁₋₆ alkylsulfonylamino groups and the C₁₋₆ alkyl sulfonyloxy groups are unsubstituted or substituted with one or more halogen atoms, one or more nitro groups, one or more C₁₋₆ alkoxy groups, or one or more C₁₋₃ haloalkoxy groups)).
12) The compound, tautomer of the compound, or pharmaceutically acceptable salt thereof, or solvate thereof according to 11), in which D is a 5-membered heteroaryl group (the 5-membered heteroaryl group is unsubstituted or substituted with one or more C₁₋₆ alkyl groups or one or more C₁₋₆ haloalkyl groups).
13) The compound, tautomer of the compound, or pharmaceutically acceptable salt thereof, or solvate thereof according to 12), in which D is a thienyl group substituted with a trifluoromethyl group or a thiazolyl group substituted with a trifluoromethyl group.
14) The compound, tautomer of the compound, or pharmaceutically acceptable salt thereof, or solvate thereof according to 13), in which D is a 5-trifluoromethylthiophen-2-yl group.
15) The compound, tautomer of the compound, or pharmaceutically acceptable salt thereof, or solvate thereof according to any one of 1) to 3), in which E is either Formula (IV)-1 to Formula (IV)-2 shown in (IV): 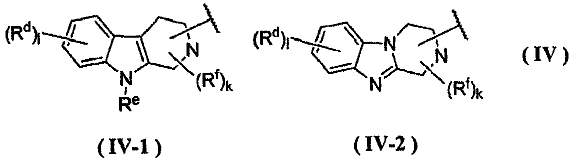 (wherein
R^{f} means
   a hydrogen atom,
   a hydroxy group,
   an amino group, or
   a halogen atom;
k is 1;
R^{e} means
   a hydrogen atom,
   a C₁₋₆ alkyl group, or
   a C₁₋₆ haloalkyl group;
R^{d} means
   a hydrogen atom,
   a hydroxy group,
   a halogen atom,
   an amino group,
   a C₁₋₆ alkyl group,
   a C₁₋₆ haloalkyl group,
   a C₁₋₆ alkoxy group,
   a C₁₋₆ haloalkoxy group,
   a mono-C₁₋₆ alkylamino group,
   a di-C₁₋₆ alkylamino group, or
   a C₁₋₆ alkylsulfonylamino group; and
1 is 1 or 2, and when 1 is 2, R^{d}s are the same as or different from each other).

16) The compound, tautomer of the compound, or pharmaceutically acceptable salt thereof, or solvate thereof according to 15), in which R^{d} is a halogen atom and 1 is 1.
17) The compound, tautomer of the compound, or pharmaceutically acceptable salt thereof, or solvate thereof according to 16), in which E is either Formula (V)-1 or Formula (V)-2 shown in (V). 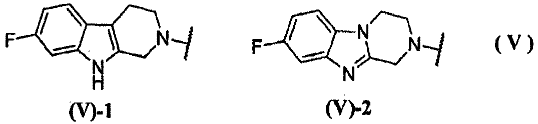
18) The compound, tautomer of the compound, or pharmaceutically acceptable salt thereof, or solvate thereof according to any one of 15) to 17), in which D is a 5-membered heteroaryl group (the 5-membered heteroaryl group is unsubstituted or substituted with one or more C₁₋₆ alkyl groups or one or more C₁₋₆ haloalkyl groups).
19) The compound, tautomer of the compound, or pharmaceutically acceptable salt thereof, or solvate thereof according to 18), in which D is a thienyl group substituted with a trifluoromethyl group or a thiazolyl group substituted with a trifluoromethyl group.
20) The compound, tautomer of the compound, or pharmaceutically acceptable salt thereof, or solvate thereof according to 19), in which D is a 5-trifluoromethylthiophen-2-yl group.
21) A compound of Formula (I): 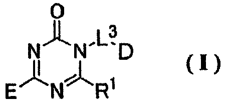 where R¹ is a hydrogen atom, L³ is a methylene group, and D and E are combinations listed in Table 1 below, a tautomer of the compound, or a pharmaceutically acceptable salt thereof, or a solvate thereof.
The symbols in Table 1 are the substituents shown in [D-E-1].

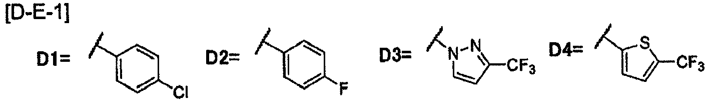

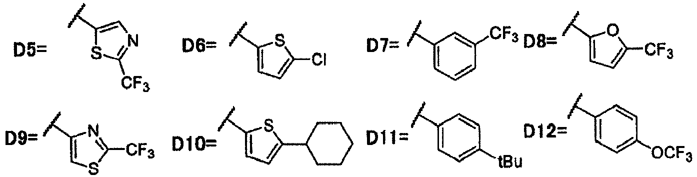

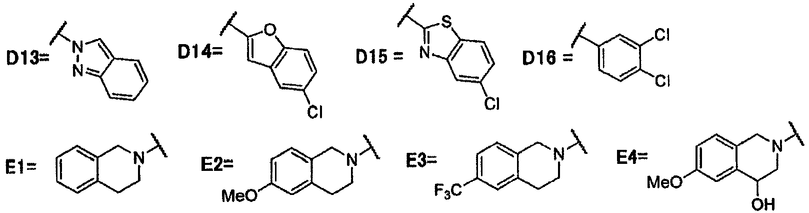

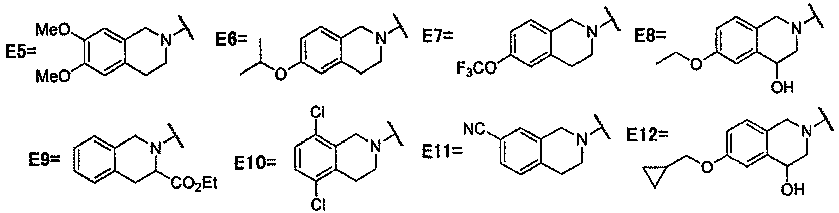

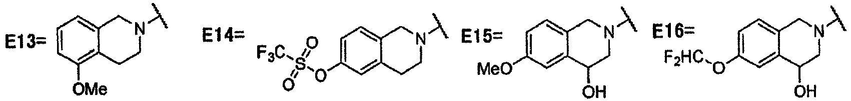

**(Table 1)**

| D | E | D | E | D | E | D | E |
|---|---|---|---|---|---|---|---|
| D 1 | E 1 | D 2 | E 1 | D 3 | E 1 | D 4 | E 1 |
| D 5 | E 1 | D 6 | E 1 | D 7 | E 1 | D 8 | E 1 |
| D 9 | E 1 | D 1 0 | E 1 | D 1 1 | E 1 | D 1 2 | E 1 |
| D 1 3 | E 1 | D 1 4 | E 1 | D 1 5 | E 1 | D 1 6 | E 1 |
| D 1 | E 2 | D 2 | E 2 | D 3 | E 2 | D 4 | E 2 |
| D 5 | E 2 | D 6 | E 2 | D 7 | E 2 | D 8 | E 2 |
| D 9 | E 2 | D 1 0 | E 2 | D 1 1 | E 2 | D 1 2 | E 2 |
| D 1 3 | E 2 | D 1 4 | E 2 | D 1 5 | E 2 | D 1 6 | E 2 |
| D 1 | E 3 | D 2 | E 3 | D 3 | E 3 | D 4 | E 3 |
| D 5 | E 3 | D 6 | E 3 | D 7 | E 3 | D 8 | E 3 |
| D 9 | E 3 | D 1 0 | E 3 | D 1 1 | E 3 | D 1 2 | E 3 |
| D 1 3 | E 3 | D 1 4 | E 3 | D 1 5 | E 3 | D 1 6 | E 3 |
| D 1 | E 4 | D 2 | E 4 | D 3 | E 4 | D 4 | E 4 |
| D 5 | E 4 | D 6 | E 4 | D 7 | E 4 | D 8 | E 4 |
| D 9 | E 4 | D 1 0 | E 4 | D 1 1 | E 4 | D 1 2 | E 4 |
| D 1 3 | E 4 | D 1 4 | E 4 | D 1 5 | E 4 | D 1 6 | E 4 |
| D 1 | E 5 | D 2 | E 5 | D 3 | E 5 | D 4 | E 5 |
| D 5 | E 5 | D 6 | E 5 | D 7 | E 5 | D 8 | E 5 |
| D 9 | E 5 | D 1 0 | E 5 | D 1 1 | E 5 | D 1 2 | E 5 |
| D 1 3 | E 5 | D 1 4 | E 5 | D 1 5 | E 5 | D 1 6 | E 5 |
| D 1 | E 6 | D 2 | E 6 | D 3 | E 6 | D 4 | E 6 |
| D 5 | E 6 | D 6 | E 6 | D 7 | E 6 | D 8 | E 6 |
| D 9 | E 6 | D 1 0 | E 6 | D 1 1 | E 6 | D 1 2 | E 6 |
| D 1 3 | E 6 | D 1 4 | E 6 | D 1 5 | E 6 | D 1 6 | E 6 |
| D 1 | E 7 | D 2 | E 7 | D 3 | E 7 | D 4 | E 7 |
| D 5 | E 7 | D 6 | E 7 | D 7 | E 7 | D 8 | E 7 |
| D 9 | E 7 | D 1 0 | E 7 | D 1 1 | E 7 | D 1 2 | E 7 |
| D 1 3 | E 7 | D 1 4 | E 7 | D 1 5 | E 7 | D 1 6 | E 7 |
| D 1 | E 8 | D 2 | E 8 | D 3 | E 8 | D 4 | E 8 |
| D 5 | E 8 | D 6 | E 8 | D 7 | E 8 | D 8 | E 8 |
| D 9 | E 8 | D 1 0 | E 8 | D 1 1 | E 8 | D 1 2 | E 8 |
| D 1 3 | E 8 | D 1 4 | E 8 | D 1 5 | E 8 | D 1 6 | E 8 |
| D 1 | E 9 | D 2 | E 9 | D 3 | E 9 | D 4 | E 9 |
| D 5 | E 9 | D 6 | E 9 | D 7 | E 9 | D 8 | E 9 |
| D 9 | E 9 | D 1 0 | E 9 | D 1 1 | E 9 | D 1 2 | E 9 |
| D 1 3 | E 9 | D 1 4 | E 9 | D 1 5 | E 9 | D 1 6 | E 9 |
| D 1 | E 1 0 | D 2 | E 1 0 | D 3 | E 1 0 | D 4 | E 1 0 |
| D 5 | E 1 0 | D 6 | E 1 0 | D 7 | E 1 0 | D 8 | E 1 0 |
| D 9 | E 1 0 | D 1 0 | E 1 0 | D 1 1 | E 1 0 | D 1 2 | E 1 0 |
| D 1 3 | E 1 0 | D 1 4 | E 1 0 | D 1 5 | E 1 0 | D 1 6 | E 1 0 |
| D 1 | E 1 1 | D 2 | E 1 1 | D 3 | E 1 1 | D 4 | E 1 1 |
| D 5 | E 1 1 | D 6 | E 1 1 | D 7 | E 1 1 | D 8 | E 1 1 |
| D 9 | E 1 1 | D 1 0 | E 1 1 | D 1 1 | E 1 1 | D 1 2 | E 1 1 |
| D 1 3 | E 1 1 | D 1 4 | E 1 1 | D 1 5 | E 1 1 | D 1 6 | E 1 I |
| D 1 | E 1 2 | D 2 | E 1 2 | D 3 | E 1 2 | D 4 | E 1 2 |
| D 5 | E 1 2 | D 6 | E 1 2 | D 7 | E 1 2 | D 8 | E 1 2 |
| D 9 | E 1 2 | D 1 0 | E 1 2 | D 1 1 | E 1 2 | D 1 2 | E 1 2 |
| D 1 3 | E 1 2 | D 1 4 | E 1 2 | D 1 5 | E 1 2 | D 1 6 | E 1 2 |
| D 1 | E 1 3 | D 2 | E 1 3 | D 3 | E 1 3 | D 4 | E 1 3 |
| D 5 | E 1 3 | D 6 | E 1 3 | D 7 | E 1 3 | D 8 | E 1 3 |
| D 9 | E 1 3 | D 1 0 | E 1 3 | D 1 1 | E 1 3 | D 1 2 | E 1 3 |
| D 1 3 | E 1 3 | D 1 4 | E 1 3 | D 1 5 | E 1 3 | D 1 6 | E 1 3 |
| D 1 | E 1 4 | D 2 | E 1 4 | D 3 | E 1 4 | D 4 | E 1 4 |
| D 5 | E 1 4 | D 6 | E 1 4 | D 7 | E 1 4 | D 8 | E 1 4 |
| D 9 | E 1 4 | D 1 0 | E 1 4 | D 1 1 | E 1 4 | D 1 2 | E 1 4 |
| D 1 3 | E 1 4 | D 1 4 | E 1 4 | D 1 5 | E 1 4 | D 1 6 | E 1 4 |
| D 1 | E 1 5 | D 2 | E 1 5 | D 3 | E 1 5 | D 4 | E 1 5 |
| D 5 | E 1 5 | D 6 | E 1 5 | D 7 | E 1 5 | D 8 | E 1 5 |
| D 9 | E 1 5 | D 1 0 | E 1 5 | D 1 1 | E 1 5 | D 1 2 | E 1 5 |
| D 1 3 | E 1 5 | D 1 4 | E 1 5 | D 1 5 | E 1 5 | D 1 6 | E 1 5 |
| D 1 | E 1 6 | D 2 | E 1 6 | D 3 | E 1 6 | D 4 | E 1 6 |
| D 5 | E 1 6 | D 6 | E 1 6 | D 7 | E 1 6 | D 8 | E 1 6 |
| D 9 | E 1 6 | D 1 0 | E 1 6 | D 1 1 | E 1 6 | D 1 2 | E 1 6 |
| D 1 3 | E 1 6 | D 1 4 | E 1 6 | D 1 5 | E 1 6 | D 1 6 | E 1 6 |

22) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 1 (where D1 to D16 and E1 to E16 in Table are the substituents shown in [D-E-2], in 22)). 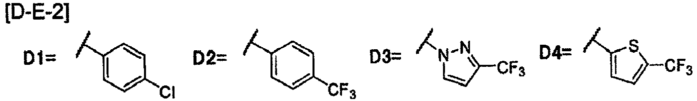 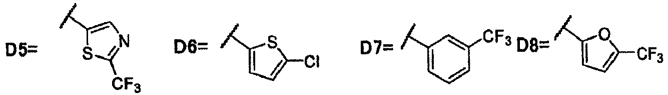 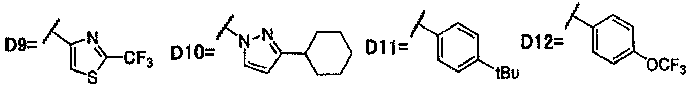 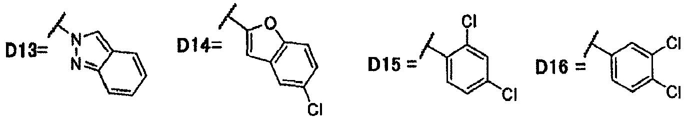 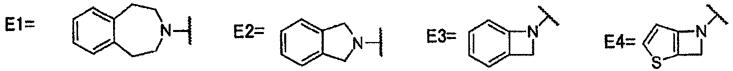 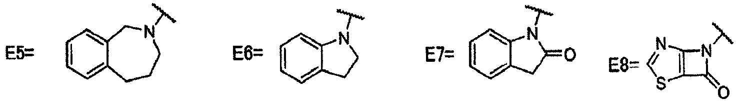 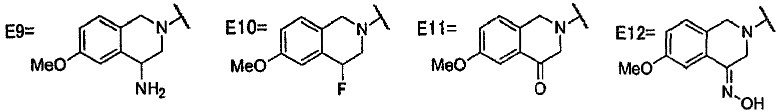 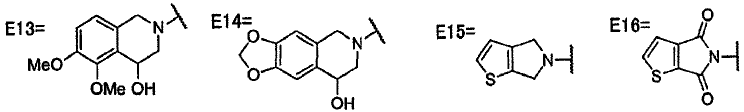
23) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 1 (where D1 to D16 and E1 to E16 in Table are the substituents shown in [D-E-3], in 23)). 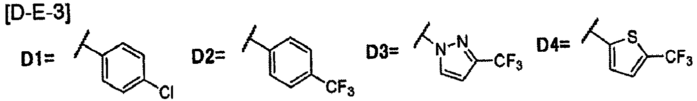 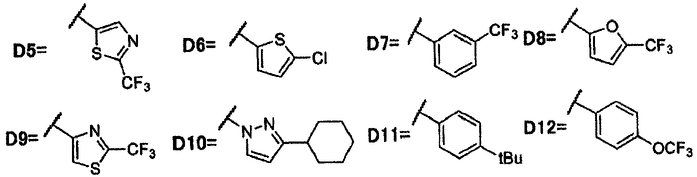 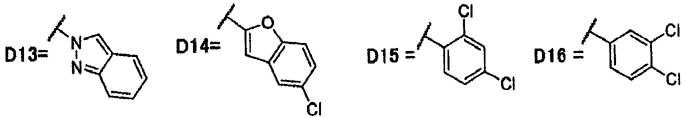 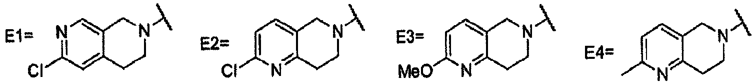 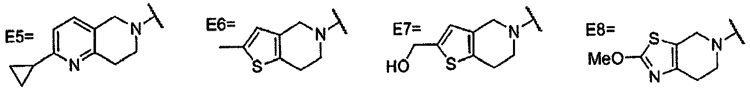 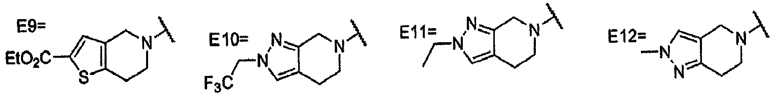 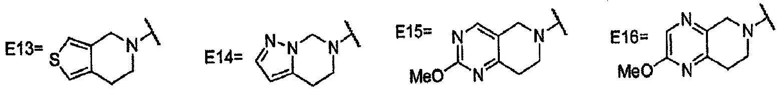
24) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 1 (where D1 to D16 and E1 to E16 in Table are the substituents shown in [D-E-4], in 24)). 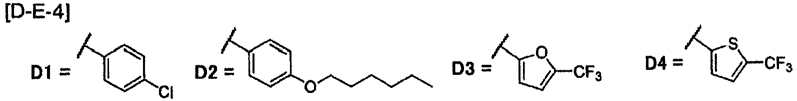 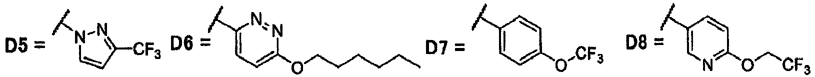 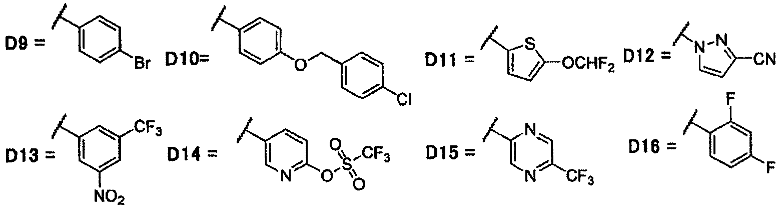 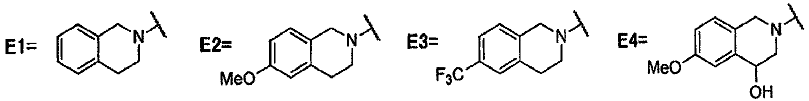 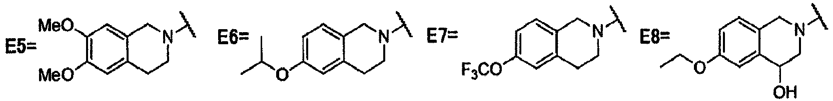 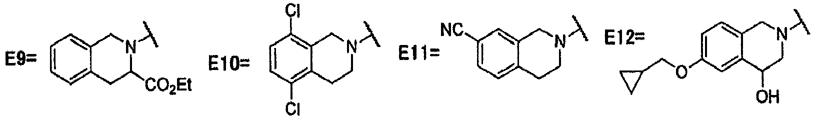 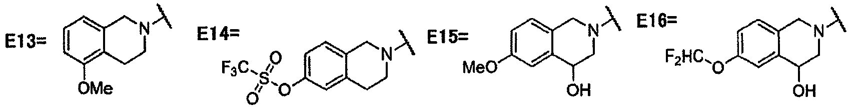
25) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 1 (where D1 to D16 and E1 to E16 in Table are the substituents shown in [D-E-5], in 25)). 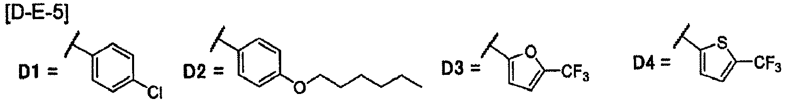 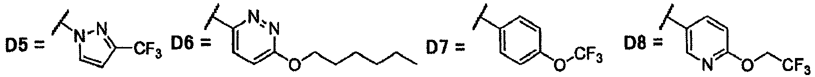 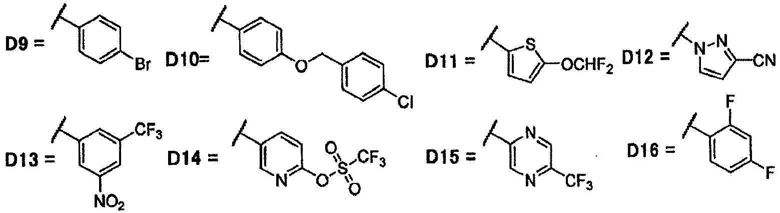 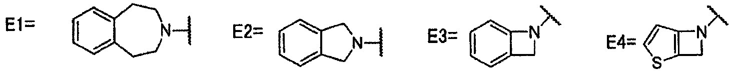 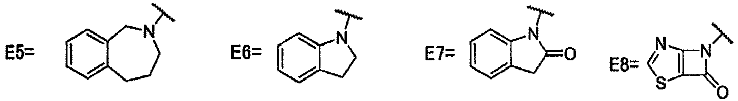 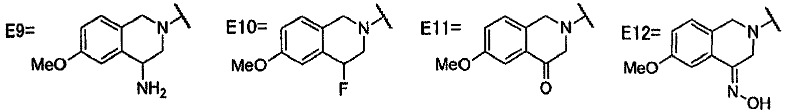 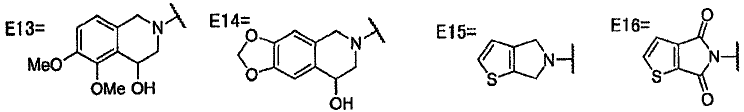
26) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 1 (where D1 to D16 and E1 to E16 in Table are the substituents shown in [D-E-6], in 26)). 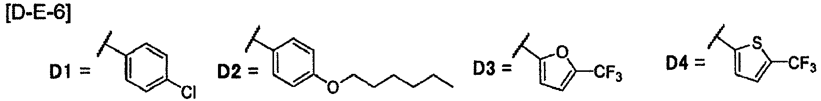 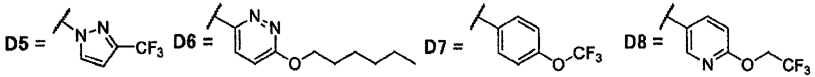 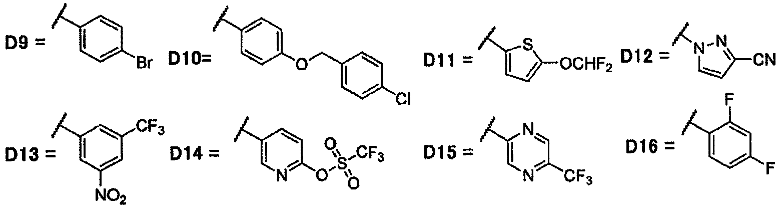 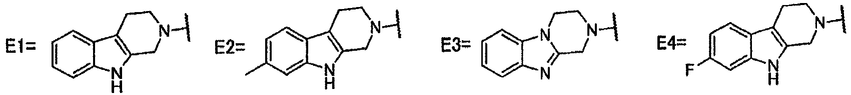 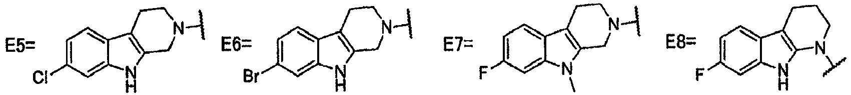 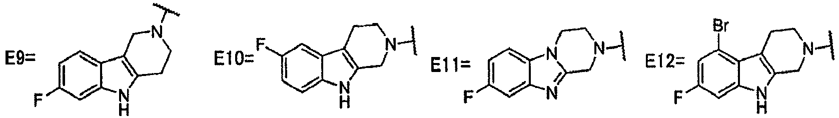 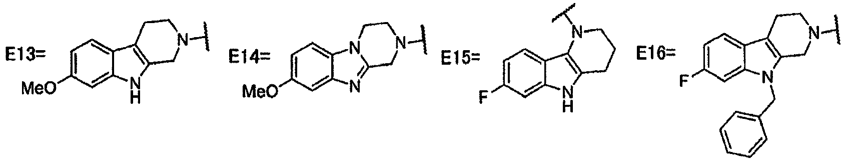
27) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 1 (where D1 to D16 and E1 to E16 in Table are the substituents shown in [D-E-7], in 27)). 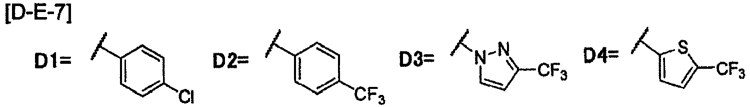 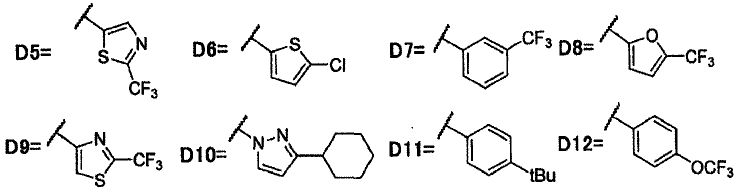 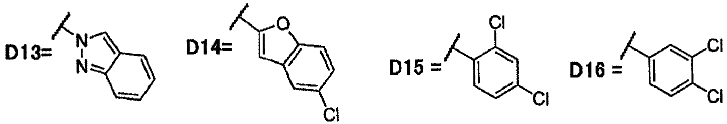 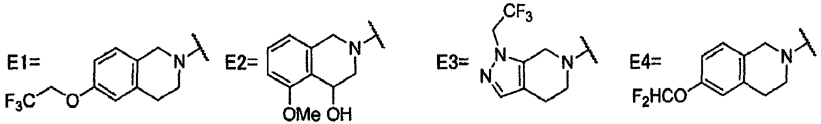 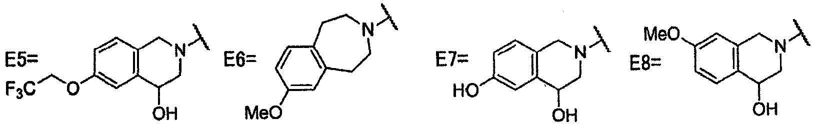 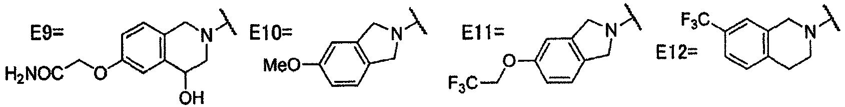 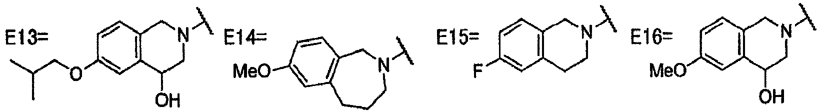
28) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 2 (where D1 to D8 and E1 to E8 in Table are the substituents shown in [D-E-8], in 28)).

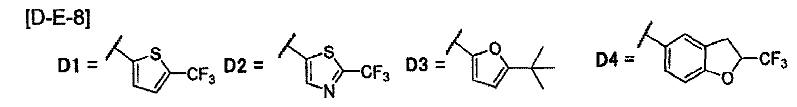

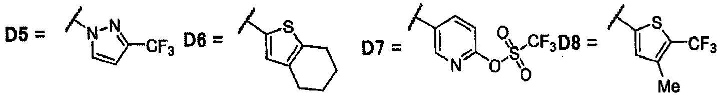

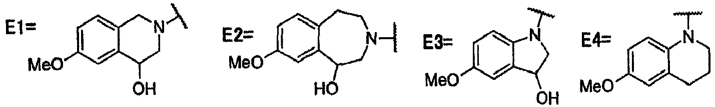

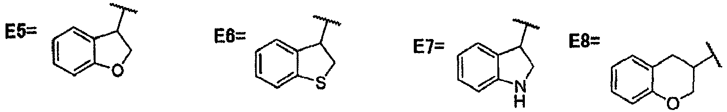

**(Table 2)**

| D | E | D | E | D | E | D | E |
|---|---|---|---|---|---|---|---|
| D 1 | E 1 | D 2 | E 1 | D 3 | E 1 | D 4 | E 1 |
| D 5 | E 1 | D 6 | E 1 | D 7 | E 1 | D 8 | E 1 |
| D 1 | E 2 | D 2 | E 2 | D 3 | E 2 | D 4 | E 2 |
| D 5 | E 2 | D 6 | E 2 | D 7 | E 2 | D 8 | E 2 |
| D 1 | E 3 | D 2 | E 3 | D 3 | E 3 | D 4 | E 3 |
| D 5 | E 3 | D 6 | E 3 | D 7 | E 3 | D 8 | E 3 |
| D 1 | E 4 | D 2 | E 4 | D 3 | E 4 | D 4 | E 4 |
| D 5 | E 4 | D 6 | E 4 | D 7 | E 4 | D 8 | E 4 |
| D 1 | E 5 | D 2 | E 5 | D 3 | E 5 | D 4 | E 5 |
| D 5 | E 5 | D 6 | E 5 | D 7 | E 5 | D 8 | E 5 |
| D 1 | E 6 | D 2 | E 6 | D 3 | E 6 | D 4 | E 6 |
| D 5 | E 6 | D 6 | E 6 | D 7 | E 6 | D 8 | E 6 |
| D 1 | E 7 | D 2 | E 7 | D 3 | E 7 | D 4 | E 7 |
| D 5 | E 7 | D 6 | E 7 | D 7 | E 7 | D 8 | E 7 |
| D 1 | E 8 | D 2 | E 8 | D 3 | E 8 | D 4 | E 8 |
| D 5 | E 8 | D 6 | E 8 | D 7 | E 8 | D 8 | E 8 |

29) The compound of Formula (I), a tautomer of the compound, or a pharmaceutically acceptable salt thereof, or a solvate thereof, in which R¹ is a hydrogen atom, L³ is a methylene group, and
E and D are combinations listed in Table 2 (where D1 to D8 and E1 to E8 in Table are the substituents shown in [D-E-9], in 29)). 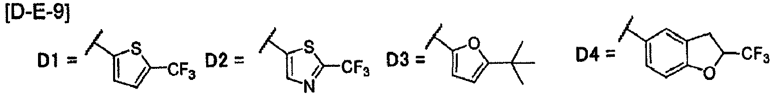 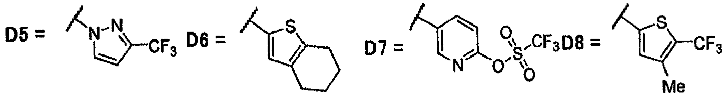 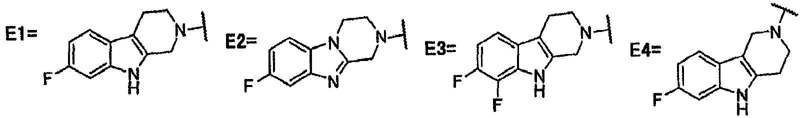 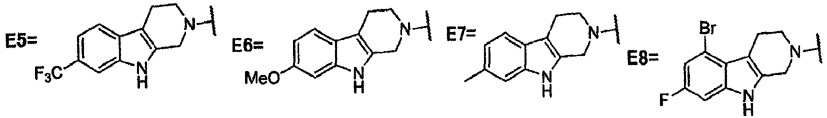
30) The compound including the combination according to any one of 21) to 29), a tautomer of the compound, or a pharmaceutically acceptable salt thereof, or a solvate thereof, in which R¹ is a methoxy group instead of the hydrogen atom.
31) The compound including the compound, tautomer of the compound, or pharmaceutically acceptable salt thereof, or solvate thereof comprising the combination according to any one of 21) to 29), in which R¹ is a n-butoxy group instead of the hydrogen atom.
32) The compound including the combination according to any one of 21) to 29), a tautomer of the compound, or a pharmaceutically acceptable salt thereof, or a solvate thereof, in which R¹ is a trifluoromethoxy group instead of the hydrogen atom.
33) The compound including the combination according to any one of 21) to 29), a tautomer of the compound, or a pharmaceutically acceptable salt thereof, or a solvate thereof, in which R¹ is a trifluoromethyl group instead of the hydrogen atom.
34) The compound including the combination according to any one of 21) to 29), a tautomer of the compound, or a pharmaceutically acceptable salt thereof, or a solvate thereof, in which R¹ is a methyl group instead of the hydrogen atom.
35) The compound including the combination according to any one of 21) to 34), a tautomer of the compound, or a pharmaceutically acceptable salt thereof, or a solvate thereof, in which L³ is an ethylene group instead of the methylene group.
36) The compound including the combination according to any one of 21) to 34), a tautomer of the compound, or a pharmaceutically acceptable salt thereof, or a solvate thereof, in which L³ is a propane-1,3-diyl group instead of the methylene group.
37) A T-type calcium channel inhibitor including the compound, tautomer of the compound, or pharmaceutically acceptable salt thereof, or solvate thereof as described in any one of 1) to 36), as an active component.
38) A preventive agent, a therapeutic agent, and/or an improving agent for a disease treatable by a T-type calcium channel inhibitory activity including the T-type calcium channel inhibitor as described in 37) as an active component.
39) A therapeutic agent for neuropathic pain including the T-type calcium channel inhibitor as described in 37) as an active component.
40) A medicine including the compound, tautomer of the compound, or pharmaceutically acceptable salt thereof, or solvate thereof as described in any one of 1) to 36), as an active component.

The present invention also includes compounds obtained through, for example, tautomerization or geometric isomerization of the compound of Formula (I) of the present invention regardless of endocyclic or exocyclic isomerization, mixtures of them, and mixtures of respective isomers. If the compound has an asymmetric center or if an asymmetric center is generated by isomerization, the present invention includes respective optical isomers and mixtures of optical isomers at any ratio. If having two or more asymmetric centers, the compound further includes diastereomers due to optical isomerism of the respective asymmetric centers. The compound of the present invention includes mixtures containing all isomers at any ratio. For example, diastereomers can be separated by a method well-known to a person skilled in the art, such as fractional crystallization and column chromatography, and an optically active compound can be produced by an organic chemical technique well-known for the purpose.

The compound of Formula (I) of the present invention or pharmaceutically acceptable salts thereof can be present in any crystal form depending on production conditions and can be present as any hydrate. These crystal forms, hydrates, and mixtures of them are also included in the scope of the present invention. The compound may be present as a solvate containing an organic solvent such as acetone, ethanol, 1-propanol, and 2-propanol, and such solvates are also included in the scope of the present invention.

The present invention includes pharmaceutically acceptable salts of Formula (I) of the present invention.

The compound of Formula (I) of the present invention can be converted into a pharmaceutically acceptable salt, as necessary, or a free form of the compound can be converted from such a salt. Examples of the pharmaceutically acceptable salt of the present invention include
alkali metal salts (such as lithium, sodium, and potassium salts),
alkaline earth metal salts (such as magnesium and calcium salts),
ammonium salts,
organic base salt,
amino acid salts,
inorganic acid salts (such as hydrochlorates, hydrobromides, phosphates, and sulfates), and
organic acid salts (such as acetates, citrates, maleates, fumarates, tartarates, benzenesulfonates, methanesulfonates, and p-toluenesulfonates).

The present invention also includes prodrugs of the compound of Formula (I) of the present invention.

Prodrugs are derivatives that are derived from a pharmaceutical compound and have a chemically or metabolically degradable group and are compounds that are degraded by solvolysis or under physiological conditions in vivo into a pharmacologically active, pharmaceutical compound. Methods for selecting and producing an appropriate prodrug derivative are described in Design of Prodrugs (Elsevier, Amsterdam 1985), for example.

For the present invention, if having a hydroxy group, the compound is reacted with an appropriate acyl halide, an appropriate acid anhydride, or an appropriate halogenated alkyloxycarbonyl compound to produce an acyloxy derivative as a prodrug, for example. Particularly preferred structures for the prodrug are exemplified by -O-COC₂H₅, -O-CO(t-Bu), -O-COC₁₅H₃₁, -O-CO(m-CO₂Na-Ph) -O-COCH₂CH₂CO₂Na, -OCOCH(NH₂)CH₃, -O-COCH₂N(CH₃)₂, or -O-CH₂O(=O)CH₃.

If the compound included in the present invention has an amino group, the compound having an amino group is reacted with an appropriate acid halide, an appropriate mixed acid anhydride, or an appropriate halogenated alkyloxycarbonyl compound to produce a prodrug, for example. Particularly preferred structures for the prodrug are exemplified by -N-CO(CH₂)₂₀OCH₃, -N-COCH(NH₂)CH₃, and -N-CH₂OC(=O)CH₃.

The preventive agent, the therapeutic agent, and/or the improving agent that are used for a disease treatable by a T-type calcium channel inhibitory activity and include the T-type calcium channel inhibitor of the present invention as an active component can be typically administered in oral administration forms such as tablets, capsules, powdered drugs, granules, pills, and syrups, rectal administration forms, transdermal absorption forms, or injection forms. The agent can be administered as a single therapeutic agent or as a mixture with other therapeutic agents. Such an agent can be singly administered but is typically administered in the form of a pharmaceutical composition. Such a formulation can be produced by adding pharmacologically, pharmaceutically acceptable additives in usual ways. In other words, the oral formulations may contain common additives such as diluents, lubricants, bonding agents, disintegrants, wetting agents, plasticizers, and coating agents. Liquid formulations for oral administration may be aqueous suspensions, oily suspensions, solutions, emulsions, syrups, elixirs, or other forms, or may be produced as dry syrups, to which water or another appropriate solvent is added before use. The liquid formulations may contain common additives such as suspending agents, flavors, diluents, and emulsifiers. For rectal administration, the agent can be administered as suppositories. The suppositories can contain appropriate substances such as cacao butter, laurin butter, macrogol, glycerogelatin, Witepsol, sodium stearate, and mixtures of them as a base and, as necessary, emulsifiers, suspending agents, preservatives, and other additives. For the injections, pharmaceutical components including solvents or solubilizing agents such as distilled water for injection, physiological saline, 5% glucose solution, and propylene glycol, pH regulators, tonicity agents, and stabilizing agents may be used to form aqueous dosage forms or dosage forms that need dissolution before use.

The pharmaceutical composition of the present invention includes the compound (or a pharmaceutically acceptable salt of the compound) of the present invention as an active component, pharmaceutically acceptable carriers, and, if needed, one or a plurality of additional therapeutic agents or adjuvants. Examples of such an additional therapeutic agent can include i) cannabinoid receptor agonists or antagonists, ii) narcotic analgesics (opioid analgesics), iii) serotonin (5-HT) receptor agonists or antagonists, iv) sodium channel blockers, v) NMDA receptor agonists or antagonists, vi) COX-2 selective inhibitors, vii) NK1 antagonists, viii) non-steroidal anti-inflammatory drugs ("NSAIDs"), ix) selective serotonin reuptake inhibitors ("SSRIs") and/or selective serotonin and norepinephrine reuptake inhibitors ("SSNRIs"), x) tricyclic antidepressants, xi) GABA receptor agonists, xii) lithium, xiii) valproates, xiv) Neurontin (gabapentin), xv) pregabalin, xvi) adrenergic receptor agonists or antagonists, xvii) neurotropin, xviii) capsaicin (TRPV1) receptor agonists or antagonists, xix) CGRP receptor antagonists, xx) steroids, xxi) bisphosphonates, and xxii) histamine receptor antagonists. The composition contains compositions suitable for oral, rectal, local, and parenteral (including subcutaneous, intramuscular, and intravenous) administrations. An optimum route for any administration is determined depending on a specific host and specific conditions and seriousness of the host to which the active component is to be administered. The pharmaceutical composition can be favorably administered in a single unit dosage form and can be prepared by any method well-known in the field of pharmaceutics.

To administer the medicinal agent of the present invention to a human, the dose is determined depending on the age and conditions of a patient. The dose for adults is typically about 0.1 mg/human/day to 1,000 mg/human/day through oral or rectal administration and about 0.05 mg/human/day to 500 mg/human/day through injection, for example. These numerical values are merely illustrative values, and the dose is determined depending on the conditions of a patient.

The compound or the pharmaceutical composition of the present invention are intended to be used for all diseases to which T-type calcium channels relate. The target disease includes pain.

With regard to pain, the target disease is specifically chronic pain and more specifically neuropathic pain.

In more detail, the pain is classified into chronic pains and acute pains including neuropathic pain, inflammatory pain, cancer pain, and visceral pain. Causative diseases of the pain are exemplified by diabetic neuropathy, traumatic neurological disorder, nerve compression, strangulation, spinal cord injury, cerebral apoplexy, fibromyalgia syndrome, carpal tunnel syndrome, osteoarthritis, rheumatoid arthritis and multiple sclerosis, herpes zoster, herpes simplex, syphilis, nerve disorders induced by cancer chemotherapy, HIV, and HIV treatment, chronic joint pain, postherpetic neuralgia, neuroma pain, trigeminal neuralgia, phantom limb pain, postoperative pain, stump pain, tooth pain, plexus neuropathy, glossopharyngeal neuralgia, laryngeal neuralgia, migraine, carcinomatous neuropathy, polyneuropathy, causalgia, low back pain, complex regional pain syndrome (CRPS), and thalamic pain.

Pains derived from other pains except the above, however, are also included in the target disease of the present invention.

Examples of other diseases except the pain include diseases associated with central nervous system (CNS) disorders, diseases associated with bladder function disorders, cerebral apoplexy, itching, atopic dermatitis, hypertension, hyperaldosteronemia, edema, ischemic heart diseases, age-related macular degeneration, cancer, diabetes mellitus, sterility, and sexual dysfunction. Examples of the diseases associated with central nervous system (CNS) disorders include epilepsy, essential tremor, schizophrenia, Parkinson's disease, manic-depressive illness, bipolar disorder, depression, anxiety, dementia, drug dependence, Huntington's disease, and sleep disturbance. Examples of the diseases associated with bladder function disorder include overactive bladder.

The compound is also clinically used for the treatment of epilepsy and partial and generalized tonic seizure. The compound is also useful for neuroprotection under ischemic conditions caused by cerebral apoplexy or neurotrauma and is useful for the treatment of multiple sclerosis. The compound is useful for the treatment of tachyarrhythmia. The compound is useful for the treatment of depression, more specifically, depressive disorders, for example, sudden or recurrent major depressive disorder, and mood disorders such as dysthymic disorder and bipolar disorders, for example, bipolar I disorder, bipolar II disorder, and cyclothymic disorder; and neuropsychiatric disorders including panic disorder with or without agoraphobia, agoraphobia with no panic disorder history, specific phobias, for example, phobia specific to animals and anthropophobia, obsessive-compulsive disorder, stress disorders such as posttraumatic stress disorder and acute stress disorder, and anxiety disorders such as generalized anxiety disorder.

In addition to primates including human beings, various other mammals can be treated by the method of the present invention. Examples of the treatable mammals include, but are not limited to, rodents (for example, mice), cattle, sheep, goats, horses, dogs, and cats. The method can also be performed on other species such as birds (for example, chickens).

When specifically used for the treatment of depression or anxiety, the compound of the present invention can be used in combination with other antidepressants or antianxiety drugs such as norepinephrine reuptake inhibitors, selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), α-adrenergic receptor antagonists, atypical antidepressants, benzodiazepines, 5-HT1A agonists or antagonists, specifically, 5-HT1A partial agonists, neurokinin-1 receptor antagonists, corticotropin-releasing factor (CRF) antagonists, and pharmaceutically acceptable salts of them.

In addition, the compound of the present invention can be administered in order to prevent the conditions and the disorders described above and to prevent other conditions and disorders to which calcium channel activity relates, in a dose level effective in the prevention.

Creams, ointments, jellies, solutions, or suspensions containing the compound can be locally used. Mouthwashes and gargles are included within the local applications for the purpose of the present invention.

The compound of the present invention can be synthesized by the methods shown below, but the production methods below are typical examples of the production method and are not intended to limit the production method.

In a typical method for producing the compound of the present invention, for smooth reactions, a reaction in the presence of an acid or a base may efficiently proceed, and a reaction under microwave irradiation may efficiently proceed.

Among the typical production methods shown below, in schemes (1) to (4), (7), (9), (10), and (12), a reaction particularly in the presence of a base such as potassium carbonate and triethylamine may be efficient for a smooth reaction.

Among the typical production methods shown below, in schemes (6) and (10), a reaction particularly in the presence of a base such as sodium hydroxide, potassium hydroxide, potassium tert-butoxide, sodium tert-butoxide, cesium carbonate, potassium carbonate, and triethylamine may be efficient for a smooth reaction.

Among the typical production methods shown below, in scheme (5), a reaction particularly with a Bronsted acid catalyst such as acetic acid, trifluoroacetic acid, hydrochloric acid, sulfuric acid, and p-toluenesulfonic acid or with a Lewis acid such as titanium tetrachloride, a trifluoroborane-diethyl ether complex, and scandium triflate may be efficient for a smooth reaction.

In the typical production methods shown below, each of reagents and raw materials can be appropriately used in an equimolar amount or an excess molar amount relative to one compound of raw materials.

In the typical production methods of the compound of the present invention shown below, general formulae of intermediates and a final product are shown in each step, but the general formulae of these intermediates and final products also generally include derivatives protected with protective groups. A derivative protected with a protective group means a compound that can yield a target compound by hydrolysis, reduction, oxidation, dehydration, halogenation, alkylation, or a similar reaction, as necessary, and includes a compound protected with a protective group that is acceptable for organic synthetic chemistry, for example.

Protection and deprotection can be carried out with well-known protective groups through protection and deprotection reactions (see Protective Groups in Organic Synthesis, Fourth edition, by T.W. Greene, John Wiley & Sons Inc, 2006, for example).

Hydrolysis, reduction, oxidation, dehydration, and halogenation can be carried out by well-known transformation methods of functional groups (see Comprehensive Organic Transformations, Second Edition, by R.C. Larock, Wiley-VCH, 1999, for example).

### (Symbol in typical production method)

In the typical production methods shown below, symbols in the drawings are as follows unless otherwise noted.

In formulae, R¹, L³, D, and E are the same as in General Formula (I) unless otherwise noted.

R^{L} is a leaving group such as a halogen atom, a methanesulfonyloxy group, a methanethiol group, and a p-toluenesulfonyloxy group.

X is a halogen atom.

R^{PR} is a hydrogen atom or a protective group such as a Boc group and a Cbz group.

Of the compounds of Formula (I), compound (1)-3 can be produced by the production method of scheme (1) below, for example.

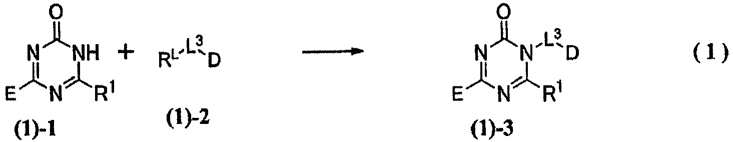

The compound (1)-3 can be synthesized using compound (1)-1 and compound (1)-2 in an appropriate solvent of Without solvent at from 0°C to a heat-reflux temperature.

Of the compounds of Formula (I), compound (2)-3 can be produced by the production method of scheme (2) below, for example. (In the scheme, D is a 3 to 11-membered non-aromatic heterocycle (compound) containing NH or a 5 to 10-membered aromatic heterocycle (compound) containing NH)

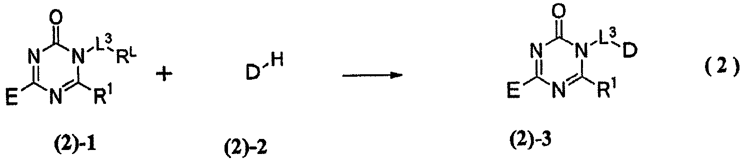

The compound (2)-3 can be synthesized using compound (2)-1 and compound (2)-2 in an appropriate solvent or without solvent at from 0°C to a heat-reflux temperature.

Of the compounds of Formula (I), compound (3)-3 can be produced by the production method of scheme (3) below, for example. (In the scheme, E is a 7 to 14-membered non-aromatic fused heterocycle (compound) containing NH)

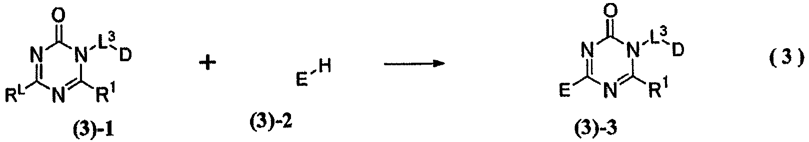

The compound (3)-3 can be synthesized using compound (3)-1 and compound (3)-2 in an appropriate solvent or without solvent at from 0°C to a heat-reflux temperature.

Of the compounds of Formula (I), compound (4)-3 can be produced by the production method of scheme (4) below, for example. (In the scheme, R¹ is a C₁₋₆ alkoxy group and other symbols are the same as the definitions of Formula (I))

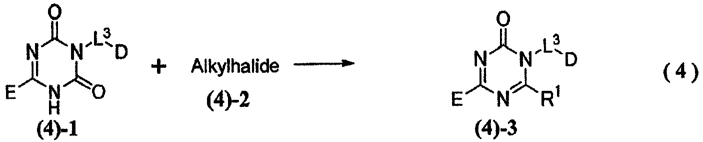

The compound (4)-3 can be synthesized from compound (4)-1 with alkyl halide (4)-2 in an appropriate solvent or without solvent at from 0°C to a heat-reflux temperature.

### Raw material synthesis 1

Compound (5)-2 can be produced by the production method of scheme (5) below, for example. (In the scheme, X is a halogen atom)

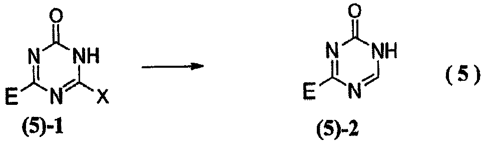

The compound (5)-2 can be synthesized by catalytic hydrogenation of compound (5)-1 with palladium or a similar catalyst in an appropriate solvent or without solvent at from 0°C to a heat-reflux temperature.

### Raw material synthesis 2

Compound (6)-2 can be produced by the production method of scheme (6) below, for example. (In the scheme, X is a halogen atom)

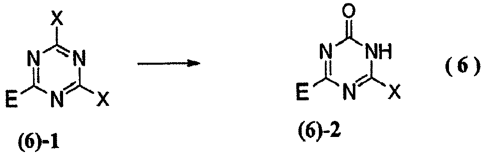

The compound (6)-2 can be synthesized by hydrolysis of compound (6)-1 with a base such as sodium hydroxide in an appropriate solvent or without solvent at from 0°C to a heat-reflux temperature.

### Raw material synthesis 3

Compound (7)-3 can be produced by the production method of scheme (7) below, for example. (In the scheme, E is a 7 to 14-membered non-aromatic fused heterocycle (compound) containing NH)

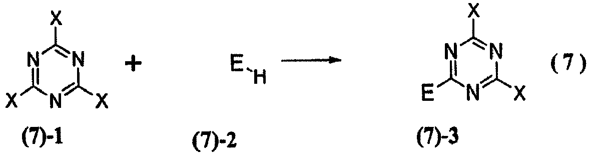

The compound (7)-3 can be synthesized using compound (7)-1 and compound (7)-2 in an appropriate solvent or without solvent at from 0°C to a heat-reflux temperature.

### Raw material synthesis 4

Compound (8)-3 can be produced by the production method of scheme (8) below, for example. (In the scheme, L³ is -CH₂-)

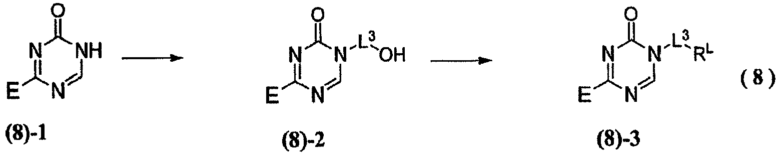

Compound (8)-2 can be obtained by reaction of compound (8)-1 with formaldehyde or paraformaldehyde in an appropriate solvent or without solvent at from 0°C to a heat-reflux temperature.

The compound (8)-3 can be synthesized by halogenation of the compound (8)-2 with thionyl chloride or a similar reagent or by sulfonyl esterification of the compound (8)-2 with p-toluenesulfonyl chloride, methanesulfonyl chloride, or a similar reagent.

### Raw material synthesis 5

Compound (9)-4 can be produced by the production method of scheme (9) below, for example.

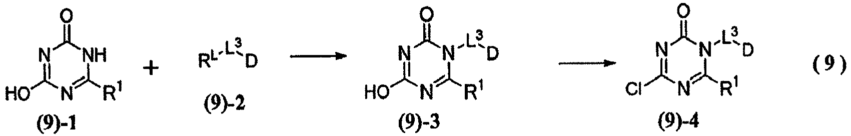

The compound (9)-3 can be synthesized using compound (9)-1 and compound (9)-2 in an appropriate solvent or without solvent at from 0°C to a heat-reflux temperature.

The compound (9)-4 can be synthesized by reaction of the compound (9)-3 with an equal amount or excess amount of phosphorus oxychloride in an appropriate solvent or without solvent at from 0°C to a heat-reflux temperature.

### Raw material synthesis 6

Compound (10)-4 can be produced by the production method of scheme (10) below, for example.

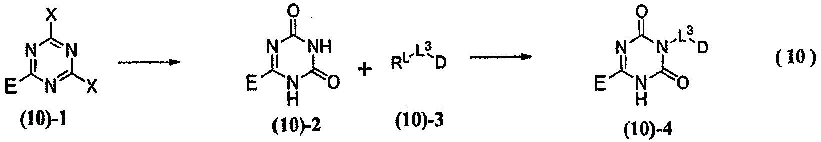

The compound (10)-4 can be obtained by hydrolysis of compound (10)-1 and subsequent reaction with compound (10)-3. Compound (10)-2 can be synthesized by hydrolysis of the compound (10)-1 with a base such as sodium hydroxide and potassium hydroxide in an appropriate solvent or without solvent at from 0°C to a heat-reflux temperature.

The compound (10)-4 can be synthesized using compound (10)-2 and compound (10)-3 in an appropriate solvent or without solvent at from 0°C to a heat-reflux temperature.

### Raw material synthesis 7

Compound (11)-6 can be produced by the production method of scheme (11) below, for example.

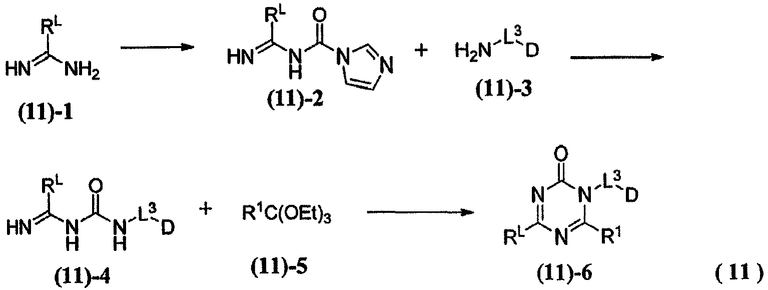

Compound (11)-2 can be synthesized by reaction of compound (11)-1 and an equal amount or excess amount of carbodiimidazole in an appropriate solvent or without solvent at from 0°C to a heat-reflux temperature.

Compound (11)-4 can be synthesized by reaction of the compound (11)-2 and an equal amount or excess amount of compound (11)-3 in an appropriate solvent or without solvent at from 0°C to a heat-reflux temperature.

Compound (11)-6 can be synthesized by reaction of the compound (11)-4 and an equal amount or excess amount of compound (11)-5 in an appropriate solvent or without solvent at from 0°C to a heat-reflux temperature.

### Raw material synthesis 8

Compound (12)-3 can be produced by the production method of scheme (12) below, for example (In the scheme, G is a C₁₋₆ alkoxycarbonyl group or a carboxy group).

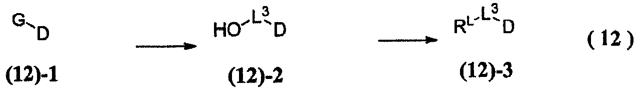

Compound (12)-2 can be synthesized from compound (12)-1 with an equal amount or excess amount of a reducing agent such as a borane-THF complex, sodium borohydride, and lithium aluminum hydride in an appropriate solvent or without solvent at from 0°C to a heat-reflux temperature.

The compound (12)-3 can be synthesized from the compound (12)-2 with an equal amount or excess amount of a chlorinating agent such as thionyl chloride in an appropriate solvent or without solvent at from 0°C to a heat-reflux temperature or synthesized from the compound (12)-2 with a sulfonyl chloride such as p-toluenesulfonyl chloride and methanesulfonyl chloride in an appropriate solvent or without solvent at from 0°C to a heat-reflux temperature.

The production method is not limited to the above, and the compound of Formula (1) can be synthesized by a common method for synthesizing a triazine compound. The common method for synthesizing a triazine compound is described in the following document: Heterocyclic Compounds, New Edition, Applications (Kodansha Ltd., 2004) pp. 167 to 195.

The partial structure of E can be synthesized by a common method for synthesizing a non-aromatic fused heterocycle. The common method for synthesizing a non-aromatic fused heterocycle is described in the following document: Heterocyclic Compounds, New Edition, Basics (Kodansha Ltd., 2004) and Applications (Kodansha Ltd., 2004).

Of the partial structure of E, a tetrahydrobenzo[4,5]imidazo[1,2-a]pyrazine ring can be synthesized by a common method described below. Bulletin de la Societe Chimique de France, 1991, pp. 255-259.

### Synthesis method:

The compound of the present invention can be prepared in accordance with the scheme described above and the procedures provided in Examples described below. The substituents are the same as the above unless otherwise defined or obvious to a person skilled in the art.

Novel compounds of the present invention can be easily synthesized through techniques known to a person skilled in the art, for example, described in Advanced Organic Chemistry, March, the fifth edition, John Wiley and Sons, New York, NY, 2001; Advanced Organic Chemistry, Carey and Sundberg, Vols. A and B, the third edition, Plenum Press, Inc., New York, NY, 1990; Protective groups in Organic Synthesis, Green and Wuts, the second edition, John Wiley and Sons, New York, NY, 1991; Comprehensive Organic Transformations, Larock, VCH Publishers, Inc., New York, NY, 1988; Handbook of Heterocyclic Chemistry, Katritzky and Pozharskii, the second edition, Pergamon, New York, NY, 2000; and reference documents cited therein.

Other reference documents referred for the synthesis of novel compounds in the present invention include Buckwald et al., Tetrahedron, 2004, Vol. 60, pp. 7397-7403; Li et al., Tetrahedron Lett., 2004, Vol. 45, pp. 4257-4260; and Jean et al., J. Org. Chem., 2004, Vol. 69, pp. 8893-8902. Starting materials for the compound can be prepared by standard synthetic conversions of chemical precursors that are easily available from commercial suppliers including Aldrich Chemical Co. (Milwaukee, WI); Sigma Chemical Co. (St. Louis, MO); Lancaster Synthesis (Windham, N.H.); Ryan Scientific (Columbia, S.C.); Maybridge (Cornwall, UK); Matrix Scientific (Columbia, S.C.); Arcos (Pittsburgh, PA); and Trans World Chemicals (Rockville, MD).

The procedures for synthesizing compounds described in the present specification can include one or a plurality of steps of protection and deprotection of functional groups and purification (for example, recrystallization, distillation, column chromatography, flash chromatography, medium-pressure column chromatography, thin-layer chromatography (TLC), radial chromatography, and high-pressure liquid chromatography (HPLC)). A product can be characterized by various techniques well-known in the chemical field, including proton and carbon-13 nuclear magnetic resonance (¹H and ¹³C NMR), infrared and ultraviolet spectroscopy (IR and UV), X-ray crystallography, elementary analysis, and HPLC-mass analysis (HPLC-MS). The procedures for the protection and deprotection of functional groups and the methods of purification, structure identification, and quantitative determination are well-known to a person skilled in the chemical synthesis.

Solvents preferably used for the synthesis of the compound by the reactions at least partially dissolve one or all of reactants and do not disadvantageously react with any of reactants or reaction products. Specific examples of the preferred solvent include aromatic hydrocarbons (for example, toluene and xylene), halogenated solvents (for example, methylene chloride, chloroform, carbon tetrachloride, and chlorobenzene), ethers (for example, diethyl ether, diisopropyl ether, tert-butyl methyl ether, diglyme, tetrahydrofuran, 1,4-dioxane, and anisole), nitriles (for example, acetonitrile and propionitrile), ketones (for example, 2-butanone, diethyl ketone, and tert-butyl methyl ketone), alcohols (for example, methanol, ethanol, n-propanol, isopropanol, n-butanol, and t-butanol), N,N-dimethylformamide (DMF), dimethyl sulfoxide (DMSO), and water. Mixtures of two or more of the solvents may also be used.

Examples of the base preferably used for the synthesis of the compound of the present invention typically include
alkali metal hydrides and alkaline earth metal hydrides (for example, lithium hydride, sodium hydride, potassium hydride, and calcium hydride),
alkali metal amides (for example, lithium diisopropylamide (LDA), lithium hexamethyldisilazide (LHMDS), potassium hexamethyldisilazide (KHMDS), lithium amide, sodium amide, and potassium amide), alkali metal carbonates and alkaline earth metal carbonates (for example, lithium carbonate, sodium carbonate, cesium carbonate, sodium hydrogen carbonate, and cesium hydrogen carbonate),
alkali metal alkoxides and alkaline earth metal alkoxides (for example, sodium methoxide, sodium ethoxide, potassium tert-butoxide, and magnesium ethoxide), alkali metal alkyls (for example, methyllithium, n-butyllithium, sec-butyllithium, t-butyllithium, and phenyllithium), alkyl magnesium halides, organic bases (for example, trimethylamine, triethylamine, triisopropylamine, N,N-diisopropylethylamine, piperidine, N-methylpiperidine, morpholine, N-methylmorpholine, pyridine, collidine, lutidine, and 4-dimethylaminopyridine), and
bicyclic amines (for example, 1,8-diazabicyclo[5.4.0]undec-7-ene (DBU) and 1,4-diazabicyclo[2.2.2]octane (DABCO)).

Functional groups of the compounds shown in the schemes below can be treated by a standard functional group conversion technique operable by a person skilled in the art, yielding an intended compound according to the present invention, if appropriate.

Other variations and modifications are obvious to a person skilled in the art and are included in the scope and teaching of the present invention. The present invention is not limited to descriptions other than the description in the claims below. Examples

The present invention will be described in detail with reference to Reference Synthesis Examples, Synthesis Examples, Test Examples, and Formulation Examples below. The present invention, however, is not limiting to these Examples.

In Examples, NMR means a nuclear magnetic resonance spectrum, LC/MS means liquid chromatography/mass spectrometry, (v/v) means (volume/volume), and expression of Rf and expression of Ex in the drawings mean Reference Synthesis Example and Synthesis Example, respectively.

Morphology means a shape.

When ¹H-NMR data is described, the data is measured at 300 MHz and represents chemical shifts δ (unit: ppm) (split patterns and integral values) of signals determined by using tetramethylsilane as an internal standard. "s" means a singlet, "d" means a doublet, "t" means a triplet, "q" means a quartet, "quint" means a quintet, "sextet" means a sextet, "septet" means a septet, "dd" means a double doublet, "ddd" means a double double doublet, "m" means a multiplet, "br" means broad, "brs" means a broad singlet, "J" means a coupling constant, "CDCl₃" means deuterated chloroform, and "DMSO-d₆" means deuterated dimethyl sulfoxide.

As a micro-wave reactor, Initiator sixty manufactured by Biotage Gb Ltd. was used.

In the purification by silica gel column chromatography, any one of Hi-Flash column manufactured by Yamazen Ltd., Silica gel 60 manufactured by Merck & Co., Inc., and PSQ60B manufactured by Fuji Silysia Chemical Ltd. was used, unless otherwise stated.

In the purification by silica gel (amino-based) column chromatography, Hi-Flash Amino Column manufactured by Yamazen Ltd. or DM1020 manufactured by Fuji Silysia Chemical Ltd. was used, unless otherwise stated.

In the purification by silica gel thin-layer chromatography, PLC Plate manufactured by Merck & Co., Inc. was used, unless otherwise stated.

In the purification by amino-based thin-layer chromatography, PLCP5 Plates NH manufactured by Fuji Silysia Chemical Ltd. was used, unless otherwise stated.

In the purification by preparative high-performance liquid chromatography, 6A Preparative High-performance Liquid Chromatography System manufactured by SHIMADZU CORPORATION was used, unless otherwise stated.

LC/MS was measured using an ESI (electrospray ionization) method under following conditions. "ESI+" means an ESI positive ion mode, "ESI-" means an ESI negative ion mode, "LC/MS: cond." means analysis conditions of LC/MS, and "RT" means a retention time.

### LC/MS Conditions 1:

Apparatus: Waters Micromass ZQ
Column: Waters SunFire C18 (3.5µm, 4.6x20 mm)
Column temperature: 40°C
Solvents used
   Solution A: 0.1 % formic acid aqueous solution
   Solution B: 0.1% formic acid - acetonitrile solution

### Elution conditions used:

The measurement was started at a flow rate of 0.4 mL/min and a mixing ratio of the solution A and the solution B of 90/10 (v/v), and then the mixing ratio of the solution A and the solution B was linearly changed to 15/85 (v/v) in 3 minutes.

Thereafter, the mixing ratio of the solution A and the solution B was fixed at 15/85 (v/v) for 2 minutes, and then the mixing ratio of the solution A and the solution B and the flow rate were linearly changed to 90/10 (v/v) and 0.5 mL/min, respectively, in 0.5 minute. Thereafter these conditions were fixed for 2.5 minutes.

### LC/MS Conditions 2:

Apparatus: Thermo LTQ XL
Column: Waters AQUITY UPLC BEH C18 (1.7 µm, 2.1x50 mm)
Column temperature: 40°C
Solvents used
   Solution A: 0.1% formic acid aqueous solution
   Solution B: 0.1 % formic acid - acetonitrile solution

### Elution conditions used:

Conditions were fixed at a flow rate of 0.6 mL/min and a mixing ratio of the solution A and the solution B of 90/10 (v/v) and the measurement was started, and then, after 0.5 minutes, the mixing ratio of the solution A and the solution B was linearly changed to 10/90 (v/v) in 2.5 minutes.

Thereafter, the mixing ratio of the solution A and the solution B was fixed at 10/90 (v/v) for 0.7 minutes, and then the mixing ratio of the solution A and the solution B and the flow rate were linearly changed to 90/10 (v/v) and 0.8 mL/min, respectively, in 0.1 minute, followed by fixing these conditions for 1 minute.

Thereafter, the mixing ratio of the solution A and the solution B was fixed at 90/10 (v/v) and the flow rate was linearly changed to 0.6 ml/min in 0.1 minute.

### LC/MS Conditions 3:

Apparatus: Waters Micromass ZQ2000
Column: Waters SunFire C18 (3.5 µm, 2.1x50 mm)
Column temperature: 40°C
Solvents used
   Solution A: Acetonitrile solution
   Solution B: 0.1% formic acid aqueous solution

### Elution conditions used:

Conditions were fixed at a flow rate of 0.4 mL/min and a mixing ratio of the solution A and the solution B of 15/85 (v/v) and the measurement was started, and then the mixing ratio of the solution A and the solution B was linearly changed to 85/15 (v/v) in 3 minutes.

Thereafter, the mixing ratio of the solution A and the solution B was fixed at 15/85 (v/v) for 2 minutes, and then the mixing ratio of the solution A and the solution B was linearly changed to 95/5 (v/v) in 0.5 minute. Thereafter these conditions were fixed for 1.5 minutes.

### Reference Synthesis Example 1

### Tert-butyl 4,6-dihydroxy-3,4-dihydroisoguinoline-2(1H)- carboxvlate

To an aqueous solution (5.0 mL) of 3-(2-amino-1-hydroxyethyl)phenol hydrochloride (2.00 g, 10.5 mmol), 30% formalin aqueous solution (10 mL) and 1M hydrochloric acid (5.0 mL) were added and the resultant reaction mixture was stirred at 100°C for 3.5 hours. After completion of the reaction, the reaction solution was concentrated under reduced pressure and tetrahydrofuran (3.0 mL), water (1.0 mL), di-tert-butyl dicarbonate (2.30 g, 10.5 mmol), and sodium hydroxide (1.20 g, 30.0 mmol) were added to the obtained residue, followed by stirring the resultant reaction solution overnight at room temperature. After completion of the reaction, water was added to the reaction solution and the resultant mixture was extracted with ethyl acetate. The organic layer was concentrated under reduced pressure and the obtained residue was purified by silica gel column chromatography (hexane/ethyl acetate = 1/1) to obtain the title compound (Rf1) (2.17g, yield 78%) as a yellow oily product.

### Reference Synthesis Example 2

### Tert-butyl 4-hydroxy-6-methoxy-3,4-dihydroisoquinoline-2(1H)-carboxylate

To a N,N-dimethylformamide solution (5.0 mL) of tert-butyl 4,6-dihydroxy-3,4-dihydroisoquinoline -2(1H)-carboxylate (2.17 g, 8.18 mmol) synthesized in Reference Synthesis Example 1, methyl iodide (5.80 g, 40.9 mmol) and potassium carbonate (5.70 g, 41.2 mmol) were added and the resultant reaction solution was stirred at room temperature for 1.5 hours. After completion of the reaction, water was added to the reaction solution and the resultant mixture was extracted with ethyl acetate. The organic layer was dried over anhydrous magnesium sulfate and concentrated under reduced pressure. The obtained residue was purified by silica gel column chromatography (hexane/ethyl acetate = 1/1) to obtain the title compound (Rf2) (876 mg, yield 38%) as a colorless oily product.

### Reference Synthesis Example 3

### Tert-butyl 2-methoxy-6,7-dihydrothiazolo[5,4-c]pyridine-5(4H)-carboxylate

To a methanol solution (2.0 mL) of tert-butyl 2-bromo-6,7-dihydrothiazolo[5,4-c]pyridine-5(4H)-carboxylate (114 mg, 0.357 mmol), a methanol solution (1.0 mL) of sodium methoxide (28.9 mg, 0.588 mmol) was added and the resultant reaction solution was stirred for 2 hours under reflux by heating. After completion of the reaction, the reaction solution was concentrated and water was added to the resultant residue, followed by extracting the resultant mixture with ethyl acetate. The organic layer was dried over anhydrous sodium sulfate and concentrated under reduced pressure. The obtained residue was purified by silica gel column chromatography (hexane/ethyl acetate = 4/1) to obtain the title compound (Rf3) (54.0 mg, yield 56%) as a colorless oily product.

### Reference Synthesis Example 4

### 2-Methoxy-4,5,6,7-tetrahydrothiazolo[5,4-c]pyridine trifluoroacetate

To tert-butyl 2-methoxy-6,7-dihydrothiazolo[5,4-c]pyridine-5(4H)-carboxylate (54.0 mg, 0.200 mmol) synthesized in Reference Synthesis Example 3, trifluoroacetic acid (1.0 mL) was added and the resultant reaction solution was stirred at room temperature for 5 minutes. After completion of the reaction, the reaction solution was concentrated under reduced pressure to obtain the crude product of the title compound (Rf4). The crude product was used as it was in the next process.

### Reference Synthesis Example 5

### Tert-butyl 4-hydroxy-6-isopropoxy-3,4-dihydroisoguinoline-2(1H)-carboxylate

To a N,N- dimethylformamide solution (2.0 mL) of tert-butyl 4,6-dihydroxy-3,4-dihydroisoquinoline-2(1H)-carboxylate (100 mg, 0.377 mmol) synthesized in Reference Synthesis Example 1, 2-bromopropane (38.9 µL, 0.414 mmol), potassium carbonate (62.5 mg, 0.452 mmol), and sodium iodide (catalytic amount) were added and the resultant reaction solution was stirred overnight at 70°C. After completion of the reaction, water was added to the reaction solution and the resultant mixture was extracted with toluene. The organic layer was concentrated under reduced pressure and the obtained residue was purified by silica gel column chromatography (hexane/ethyl acetate) to obtain the title compound (Rf5) (19.4 mg, yield 17%) as a colorless oily product.

### Reference Synthesis Example 6

### 6-Isopropoxy-1,2,3,4-tetrahydroisoquinolin-4-ol hydrochloride

To a 1,4- dioxane (120 µL) of tert-butyl 4-hydroxy-6-isopropoxy-3,4-dihydroisoquinoline-2(1H)-carboxylate (19.4 mg, 0.0631 mmol) synthesized in Reference Synthesis Example 5, 4M hydrogen chloride/1,4-dioxane (120 µL) was added and the resultant reaction solution was stirred overnight at room temperature. After completion of the reaction, the reaction solution was concentrated to obtain the crude product of the title compound (Rf6) as a light yellow solid, which was used as it was in the next step.

### Reference Synthesis Example 7

### Tert-butyl 4-hydroxy-6-isobutoxy-3,4-dihydroisoquinoline-2(1H)-carboxylate

1-Bromo-2-methyl propane (45.1 µL, 0.414 mmol) was used instead of 2-bromo propane to obtain the title compound (Rf7) (14.1 mg, yield 12%) as a colorless oily product by synthesis in a similar manner to Reference Synthesis Example 5.

### Reference Synthesis Example 8

### 6-Isobutoxy-1,2,3,4-tetrahydroisoquinolin-4-ol hydrochloride

Tert-butyl 4-hydroxy-6-isobutoxy-3,4-dihydroisoquinoline-2(1H)-carboxylate (14.9 mg, 46.4 µmol) synthesized in Reference Synthesis Example 7 was used instead of the Rf5 compound to obtain the crude product of the title compound (Rf8) as a light yellow solid by synthesis in a similar manner to Reference Synthesis Example 6. The crude compound was used as it was in the next step.

### Reference Synthesis Example 9

### Tert-butyl 6-(cyanomethoxy)-4-hydroxy-3,4-dihydroisoquinoline-2(1H)-carboxylate

2-Bromo-acetonitrile (28.9 µL, 0.377 mmol) was used instead of 2-bromopropane to obtain the title compound (Rf9) (19.3 mg, yield 16%) as a light yellow oily product by synthesis in a similar manner to Reference Synthesis Example 5.

### Reference Synthesis Example 10

### 2-[(4-Hydroxy-1,2,3,4-tetrahydroisoquinolin-6-yl)oxy]acetonitrile hydrochloride Tert-butyl

6-(cyanomethoxy)-4-hydroxy-3,4-dihydroisoquinoline-2(1H)-carboxylate (19.3 mg, 63.4 µmol) synthesized in Reference Synthesis Example 9 was used instead of the Rf5 compound to obtain the crude product of the title compound (Rf10) as a light yellow solid by synthesis in a similar manner to Reference Synthesis Example 6. The crude compound was used as it was in the next step.

### Reference Synthesis Example 11

### Tert-butyl 5-(2,2,2-trifluoroethoxy)isoindoline-2-carboxylate

Tert-butyl 5-hydroxy-isoindoline-2-carboxylate (20.0 mg, 0.0850 mmol) and 2,2,2-trifluoroethyl trifluoromethanesulfonate (14.7 µL, 0.102 mmol) were used to obtain the crude product of the title compound (Rf11) as a light brown oily product by synthesis in a similar manner to Reference Synthesis Example 2. The crude product was used as it was in the next step.

### Reference Synthesis Example 12

### 5-(2,2,2-Trifluoroethoxy)isoindoline hydrochloride

The crude product of tert-butyl 5- (2,2,2-trifluoroethoxy)isoindoline-2-carboxylate synthesized in Reference Synthesis Example 11 was used instead of the Rf5 compound to obtain the crude product of the title compound (Rf12) as a light brown oily product by synthesis in a similar manner to Reference Synthesis Example 6.

### Reference Synthesis Example 13

### Tert-butyl 6-(difluoromethoxy)-3,4-dihydroisoquinoline-2(1H)-carboxylate

To a N,N-dimethylformamide solution (2.0 mL) of tert-butyl 6-hydroxy-3,4-dihydroisoquinoline-2(1H)-carboxylate (249 mg, 1.00 mmol), sodium chlorodifluoroacetate (152 mg, 1.00 mmol) and cesium carbonate (652 mg, 2.00 mmol) were added and the resultant reaction solution was stirred at 80°C for 5 hours. After completion of the reaction, water was added to the reaction solution and the resultant mixture was extracted with ethyl acetate. The organic layer was dried over anhydrous sodium sulfate and concentrated under reduced pressure. The obtained residue was purified by silica gel column chromatography (hexane/ethyl acetate = 8/1) to obtain the title compound (Rf13) (92.5 mg, yield 31%) as a colorless oily product.

### Reference Synthesis Example 14

### 6-(Difluoromethoxy)-1,2,3,4-tetrahydroisoquinoline hydrochloride

To tert-butyl 6-(difluoromethoxy)-3,4-dihydroisoquinoline-2(1H)-carboxylate (92.5 mg, 0.309 mmol) synthesized in Reference Synthesis Example 13, 4M hydrogen chloride/1,4-dioxane (2.0 mL) was added and the resultant reaction solution was stirred at room temperature for 2 hours. After completion of the reaction, ethyl acetate was added to the reaction solution and the precipitated solid was collected by filtration to obtain the title compound (Rf14) (57.9 mg, yield 80%) as a colorless solid.

### Reference Synthesis Example 15

### Tert-butyl 6-{[(trifluoromethyl)sulfonyl]oxy}-3,4-dihydroisoquinoline-2(1H)-carboxylate

To a dichloromethane solution (5.0 mL) of tert-butyl 6-hydroxy-3,4-dihydroisoquinoline-2(1H)-carboxylate (249 mg, 1.00 mmol), trifluoromethanesulfonic anhydride (337 µL, 2.00 mmol) and triethylamine (558 µL, 4.00 mmol) were added at 0°C and the resultant reaction solution was stirred overnight at room temperature. After completion of the reaction, a brine was added to the reaction solution and the resultant mixture was extracted with ethyl acetate. The organic layer was dried over anhydrous sodium sulfate and concentrated under reduced pressure. The obtained residue was purified by silica gel column chromatography (hexane/ethyl acetate = 8/1) to obtain the title compound (Rf15) (214 mg, yield 56%) as a colorless oily product.

### Reference Synthesis Example 16

### 1.2,3,4-Tetrahydroisoquinolin-6-yl trifluoromethanesulfonate hydrochloride Tert-butyl

6-{[(trifluoromethyl)sulfonyl]oxy}-3,4-dihydroisoquinoline-2(1H)-carboxylate (214 mg, 0.561 mmol) synthesized in Reference Synthesis Example 15 was used instead of the Rf13 compound to obtain the title compound (Rf16) (148 mg, yield 83%) as a colorless solid by synthesis in a similar manner to Reference Synthesis Example 14.

### Reference Synthesis Example 17

### Tert-butyl 6-(2,2,2-trifluoroethoxy)-3,4-dihydroisoquinoline-2(1H)-carboxylate

To an acetonitrile solution (3.0 mL) of tert-butyl 6-hydroxy-3,4-dihydroisoquinoline-2(1H)-carboxylate (249 mg, 1.00 mmol), 2,2,2-trifluoroethyl trifluoromethanesulfonate (232 mg, 1.00 mmol) and potassium carbonate (276 mg, 2.00 mmol) were added and the resultant reaction solution was stirred at room temperature for 16 hours. After completion of the reaction, water was added to the reaction solution and the resultant mixture was extracted with ethyl acetate. The organic layer was dried over anhydrous sodium sulfate and concentrated under reduced pressure. The obtained residue was purified by silica gel column chromatography (hexane/ethyl acetate = 8/1) to obtain the title compound (Rf17) (256 mg, yield 77%) as a colorless solid.

### Reference Synthesis Example 18

### 6-(2,2,2-Trifluoroethoxy)-1,2,3,4-tetrahydroisoquinoline hydrochloride

Tert-butyl 6-(2,2,2-trifluoroethoxy)-3,4-dihydroisoquinoline-2(1H)-carboxylate (256 mg, 0.773 mmol) synthesized in Reference Synthesis Example 17 was used instead of the Rf13 compound to obtain the title compound (Rf18) (149 mg, yield 72%) as a colorless solid by synthesis in a similar manner to Reference Synthesis Example 14.

### Reference Synthesis Example 19

### Tert-butyl 4-hydroxy-6-(2,2,2-trifluoroethoxy)-3,4-dihydroisoquinoline-2(1H)-carboxylate

Tert-butyl 4,6-dihydroxy-3,4-dihydroisoquinoline-2(1H)-carboxylate (300 mg, 1.13 mmol) synthesized in Reference Synthesis Example 1 and 2,2,2-trifluoroethyl trifluoromethanesulfonate (163 µL, 1.13 mmol) were used to obtain the title compound (Rf19) (133 mg, yield 34%) as a colorless solid by synthesis in a similar manner to Reference Synthesis Example 2.

### Reference Synthesis Example 20

### 6-(2,2,2-Trifluoroethoxy)-1,2,3,4-tetrahydroisoquinolin-4-ol hydrochloride Tert-butyl

4-hydroxy-6-(2,2,2-trifluoroethoxy)-3,4-dihydroisoquinoline-2(1H)-carboxylate (10.0 mg, 0.0288 mmol) synthesized in Reference Synthesis Example 19 was used instead of the Rf5 compound to obtain the crude product of the title compound (Rf20) as a colorless solid in a similar manner to Reference Synthesis Example 6. The crude compound was used as it was in the next step.

### Reference Synthesis Example 21

### Tert-butyl 4-(1,3-dioxoisoindolin-2-yl)-6-methoxy-3,4-dihydroisoquinoline-2(1H)-carboxylate

To a dichloromethane (1.0 mL) and acetonitrile (1.0 mL) mixed solution of tert-butyl 4-hydroxy-6-methoxy-3,4-dihydroisoquinoline-2(1H)-carboxylate (87.7 mg, 0.314 mmol) synthesized in Reference Synthesis Example 2, triphenylphosphine (98.9 mg, 0.377 mmol), phthalimide (55.5 mg, 0.377 mmol), and diisopropyl azodicarboxylate (1.9M toluene solution) (198 µL, 0.377 mmol) were added dropwise and the resultant reaction solution was stirred at room temperature for 5 hours. After completion of the reaction, the reaction solution was purified by silica gel column chromatography (hexane/ethyl acetate = 4/1) to obtain the title compound (Rf21) (21.4 mg, yield 17%) as a colorless amorphous product.

### Reference Synthesis Example 22

### 2-(6-methoxy-1,2,3,4-tetrahydroisoquinolin-4-yl)isoindoline-1,3-dione hydrochloride

To tert-butyl 4-(1,3-dioxoisoindolin-2-yl)-6-methoxy-3,4-dihydroisoquinoline-2(1H)-carboxylate (21.4 mg, 0.0524 mmol) synthesized in Reference Synthesis Example 21, 4M hydrogen chloride/1,4-dioxane (1.0 mL) was added and the resultant reaction solution was stirred at room temperature for 1 hour. After completion of the reaction, diethyl ether was added to the reaction solution and the precipitated solid was collected by filtration to obtain the title compound (Rf22) (14.7 mg, yield 82%) as a colorless solid.

### Reference Synthesis Example 23

### 6-Methoxy-1,2,3,4-tetrahydroisoquinolin-4-amine

To an ethanol solution (2.0 mL) of 2-(6-methoxy-1,2,3,4-tetrahydroisoquinolin-4-yl)isoindoline-1,3-dione hydrochloride (14.7 mg, 0.0426 mmol) synthesized in Reference Synthesis Example 22, hydrazine monohydrate (12.0 µL, 0.245 mmol) was added and the resultant reaction solution was stirred at 90°C for 5.5 hours. After completion of the reaction, water was added to the reaction solution and the resultant mixture was concentrated under reduced pressure. The obtained solid was suspended in chloroform and filtered. The filtrate was concentrated under reduced pressure to obtain the title compound (Rf23) (8.60 mg, quantitative) as a colorless amorphous product.

### Reference Synthesis Example 24

### 6-Methyl-1-{[5-(trifluoromethyl)thiophen-2-yl] methyl}-1,3,5-triazine-2,4(1H,3H)-dione

To a dichloromethane solution (4.0 mL) of 6-methyl-4-(methylthio)-1-{[5-(trifluoromethyl)thiophen-2-yl]methyl}-1,3,5-triazin-2(1H )-one (161 mg, 0.500 mmol) synthesized in Reference Synthesis Example 34 described below, 3-chloroperbenzoic acid (65 wt.%) (266 mg, 1.00 mmol) was added at 0°C and the resultant reaction solution was stirred at room temperature for 2 hours. After completion of the reaction, a saturated aqueous sodium thiosulfate solution and a brine were added to the reaction solution and the resultant mixture was extracted with chloroform. The organic layer was dried over anhydrous sodium sulfate and concentrated under reduced pressure. The obtained residue was purifed by silica gel column chromatography (chloroform/ethyl acetate = 1/0 → 0/1) to obtain the title compound (Rf24) (134 mg, yield 92%) as a colorless solid.

### Reference Synthesis Example 25

### 4-Chloro-6-methy-1-{[5-(trifluoromethyl)thiophen-2-yl]methyl}-1,3,5-triazin-2(1H)-one

To a toluene solution (6.0 mL) of 6-methyl-1-{[5-(trifluoromethyl)thiophen-2-yl]methyl}-1,3,5-triazine-2,4(1H,3H)-dione (120 mg, 0.412 mmol) synthesized in Reference Synthesis Example 24, phosphorus oxychloride (70.6 µL, 0.824 mmol) and N,N-diisopropylethylamine (140 µL, 0.824 mmol) were added and the resultant reaction solution was stirred at 70°C for 2 hours. After completion of the reaction, toluene was added to the reaction solution and the resultant mixture was concentrated. To the obtained residue, chloroform (412 µL) was added to obtain a 0.1 M chloroform solution of the title compound (Rf25).

### Reference Synthesis Example 26

### Methyl N-({[(4-fluorobenzen-1-yl)methyl]amino}carbonyl)-carbamimidothioate

A tetrahydrofuran solution (15 mL) of methyl 1H-imidazole-1-carbonylcarbamimidothioate (1.00 g 5.43 mmol) synthesized in Reference Synthesis Example 31 described below was refluxed and a tetrahydrofuran solution (15 mL) of 4-fluorobenzylamine (412 µL, 3.62 mmol) was added dropwise thereto, followed by stirring the resultant reaction solution under reflux by heating for 30 minutes. After completion of the reaction, the reaction solution was concentrated under reduced pressure and the obtained residue was purified by silica gel column chromatography (chloroform/ethyl acetate = 4/1 → 2/3) to obtain the title compound (Rf26) (891 mg, quantitative) as a colorless oily product.

### Reference Synthesis Example 27

### 1-(4-Fluorobenzyl)-6-methyl-4-(methylthio)-1,3,5-triazin-2(1H)-one

To methyl N-({[(4-fluorobenzen-1-yl)methyl]amino}carbonyl)-carbamimidothioate (891 mg, 3.62 mmol) synthesized in Reference Synthesis Example 26, triethyl orthoacetate (4.0 mL) was added and the resultant reaction solution was stirred at 160°C for 1 hour. After completion of the reaction, the reaction solution was cooled to room temperature and purified by silica gel column chromatography (chloroform/ethyl acetate = 9/1 → 3/2) to obtain the title compound (Rf27) (832 mg, yield 87%) as a light yellow solid.

### Reference Synthesis Example 28

### Tert-butyl

### 6-(cyclopropylmethoxy)-4-hydroxy-3,4-dihydroisoquinoline-2(1H)-carboxyte

(Bromomethyl)cyclopropane (40.2 µL, 0.414 mmol) was used instead of methyl iodide to obtain the title compound (Rf28) (14.0 mg, yield 12%) as a colorless oily product by synthesis in a similar manner to Reference Synthesis Example 2.

### Reference Synthesis Example 29

### 6-(Cyclopropylmethoxy)-1,2,3,4-tetrahydroisoquinolin-4-ol hydrochloride

Tert-butyl 6-(cyclopropylmethoxy)-4-hydroxy-3,4-dihydroisoquinoline-2(1H)-carboxylate (14.0 mg, 48.3 µmol) synthesized in Reference Synthesis Example 28 was used instead of the Rf5 compound to obtain the crude product of the title compound (Rf29) as a light yellow solid by synthesis in a similar manner to Reference Synthesis Example 6. The crude compound was used as it was in the next step.

### Reference Synthesis Example 30

### 5,6-Dimethoxy-1,2,3,4-tetrahydroisoquinolin-4-ol hydrochloride

To N-(3,4-dimethoxybenzyl)-2,2-dimethoxyethanamine (158 mg, 0.619 mmol), 1 M hydrochloric acid was added and the resultant reaction solution was stirred for 1 day. After completion of the reaction, the reaction solution was cooled to 0°C and 1 M sodium hydroxide aqueous solution was added until the pH of the resultant liquid became 10, followed by extracting the resultant liquid with chloroform. The organic layer was dried over anhydrous sodium sulfate and concentrated under reduced pressure to obtain the crude product of the title compound (Rf30) as a yellow oily product.

### Reference Synthesis Example 31

### Methyl 1H-imidazole-1-carbonvlcarbamimidothioate

To S-methylisothiourea sulfate (8.35 g, 30.0 mmol), 1 M sodium hydroxide aqueous solution (60 mL) was added and then 1,1'-carbonyldiimidazole (9.73 g, 60.0 mmol) was slowly added with stirring at 0°C. The resultant reaction solution was stirred for 30 minutes. After completion of the reaction, the precipitated solid was collected by filtration and washed with ice-water to obtain the title compound (Rf32) (7.89 g, yield 72%) as a colorless solid.

### Reference Synthesis Example 32

### Methyl N-[({[5-(trifluoromethyl)thiophen-2-yl]methyl}amino)carbonyl]-carbamimidothioate

A tetrahydrofuran solution (30 mL) of methyl 1H-imidazole-1-carbonylcarbamimidothioate (5.70 g, 31.0 mmol) synthesized in Reference Synthesis Example 31 was stirred under reflux by heating and a tetrahydrofuran solution (26 mL) of [5-(trifluoromethyl)thiophen-2-yl]methanamine (2.81 g, 15.5 mmol) was added dropwise thereto, followed by stirring the resultant reaction solution for 30 minutes. After completion of the reaction, the reaction solution was concentrated under reduced pressure and the obtained residue was purified by silica gel column chromatography (hexane/ethyl acetate = 1/1) to obtain the title compound (Rf32) (3.28 g, yield 71%) as a colorless solid.

### Reference Synthesis Example 33

### 4-(Methylthio)-1-{[5-(trifluoromethyl)thiophen-2-yl]methyl}-1,3,5-triazin-2(1H)-one

To methyl N-[({[5-(trifluoromethyl)thiophen-2-yl]methyl}amino)carbonyl]-carbamimidothioate (2.68 g, 9.03 mmol) synthesized in Reference Synthesis Example 32, triethyl orthoformate (10 mL) was added and the resultant reaction solution was stirred at 150°C for 1 hour. After completion of the reaction, the reaction solution was cooled to room temperature. Thereafter, ethanol (20 mL) was added thereto and the resultant mixture was cooled to 0°C. The precipitated solid was collected by filtration and washed with ethanol to obtain the title compound (Rf33) (1.69 g, yield 61%) as a colorless solid.

### Reference Synthesis Example 34

### 6-Methyl-4-(methylthio)-1-{[5-(trifluoromethyl)thiophen-2-yl]methyl}-1,3,5-triazin-2(1 H)-one

To methyl N-[({[5-(trifluoromethyl)thiophen-2-yl]methyl}amino)carbonyl]-carbamimidothioate (59.5 mg, 0.200 mmol) synthesized in Reference Synthesis Example 32, triethyl orthoacetate (0.60 mL) was added and the resultant reaction solution was stirred at 150°C for 1 hour. After completion of the reaction, the reaction solution was purified by silica gel column chromatography (hexane/ethyl acetate = 3/2) to obtain the title compound (Rf34) (45.8 mg, yield 71%) as a yellow solid.

### Reference Synthesis Example 35

### Methyl N-[({[2-(trifluoromethyl)thiazol-5-yl]methyl}amino)carbonyl]-carbamimidothioate

[2- (Trifluoromethyl)thiazol-5-yl]methanamine (370 mg, 2.03 mmol) was used instead of [5-(trifluoromethyl)thiophen-2-yl]methanamine to obtain the title compound (Rf35) (607 mg, quantitative) as a yellow oily product by synthesis in a similar manner to Reference Synthesis Example 32.

### Reference Synthesis Example 36

### 6-Methyl-4-(methylthio)-1-{[2-(trifluoromethyl)thiazol-5-yl]methyl}-1,3,5-triazin-2(1H)-one

Methyl N-[({[2-(trifluoromethyl)thiazol-5-yl]methyl}amino)carbonyl]-carbamimidothioate (607 mg, 2.03 mmol) synthesized in Reference Synthesis Example 35 was used instead of the Rf32 compound to obtain the title compound (Rf36) (630 mg, yield 97%) as an orange color oily product by synthesis in a similar manner to Reference Synthesis Example 34.

### Reference Synthesis Example 37

### 5-(Hydroxymethyl)pyridin-2-yl trifluoromethanesulfonate

To a tetrahydrofuran solution (2.0 mL) of lithium aluminum hydride (19.0 mg, 0.450 mmol), ethyl 6-{[(trifluoromethyl)sulfonyl]oxy}nicotinate (112 mg, 0.375 mmol) was added and the resultant reaction solution was stirred at 0°C for 5 minutes. After completion of the reaction, 1 M sodium hydroxide aqueous solution, Celite, and ethyl acetate were added to the reaction solution and the resultant mixture was stirred and then filtered. The filtrate was extracted with ethyl acetate and the organic layer was washed with a brine, dried over anhydrous sodium sulfate, and concentrated under reduced pressure to obtain the crude product (62.9 mg) of the title compound (Rf37) as a yellow oily product.

### Reference Synthesis Example 38

### 5-(Chloromethyl)pyridin-2-yl trifluoromethanesulfonate

To a dichloromethane solution (1.2 mL) of 5-(hydroxymethyl)pyridin-2-yl trifluoromethanesulfonate (62.9 mg, 0.245 mmol) synthesized in Reference Synthesis Example 37, thionyl chloride (1.2 mL) was added at 0°C and the resultant reaction solution was stirred at room temperature for 2 hours. After completion of the reaction, the reaction solution was concentrated under reduced pressure to obtain the crude product (64.4 mg) of the title compound (Rf38) as a brown oily product.

### Reference Synthesis Example 39

### 5-[(1,3-Dioxoisoindolin-2-yl)methyl]pyridin-2-yl trifluoromethanesulfonate

To a tetrahydrofuran solution (16 mL) of 5-(hydroxymethyl)pyridin-2-yl trifluoromethanesulfonate (1.68 g, 6.55 mmol), triphenylphosphine (1.89 g, 7.20 mmol), and phthalimide (1.01 g, 6.88 mmol), diisopropyl azodicarboxylate (1.43 mL, 7.20 mmol) was added dropwise at 50°C and the resultant reaction solution was stirred at room temperature for 1 day. After completion of the reaction, the reaction solution was concentrated under reduced pressure and the obtained residue was purified by silica gel column chromatography (hexane/ethyl acetate) to obtain the crude product (3.39 g) of the title compound (Rf39) as a colorless solid.

### Reference Synthesis Example 40

### 5-(Aminomethyl)pyridin-2-yl trifluoromethanesulfonate

To an ethanol solution (50 mL) of 5-[(1,3-dioxoisoindolin-2-yl)methyl]pyridin-2-yl trifluoromethanesulfonate (3.39 g, 6.55 mmol) synthesized in Reference Synthesis Example 39, hydrazine monohydrate (635 µL, 13.1 mmol) was added and the resultant reaction solution was stirred for 3 hours under reflux by heating. After completion of the reaction, the reaction solution was filtered and the filtrate was concentrated under reduced pressure. Chloroform was added to the obtained residue and the resultant mixture was filtered again and the filtrate was concentrated under reduced pressure to obtain the crude product (2.84 g) of the title compound (Rf40) as a colorless amorphous product.

### Reference Synthesis Example 41

### 5-({3-[Imino(methylthio)methyl]ureido}methyl)pyridin-2-yl trifluoromethanesulfonate

5-(Aminomethyl)pyridin-2-yl trifluoromethanesulfonate (1.81 g, 9.83 mmol) was used instead of [5-(trifluoromethyl)thiophen-2-yl]methanamine to obtain the title compound (Rf41) (2.55 g, quantitative) as a yellow oily product by synthesis in a similar manner to Reference Synthesis Example 32.

### Reference Synthesis Example 42

### 5-{[4-(Methylthio)-2-oxo-1,3,5-triazin-1(2H)-yl]methyl}pyridin-2-yl trifluoromethanesulfonate

5-({3-[Amino(methylthio)methylene]ureido}methyl)pyridin-2-yl trifluoromethanesulfonate (1.00 g, 2.69 mmol) synthesized in Reference Synthesis Example 41 was used instead of the Rf32 compound to obtain the title compound (Rf42) (431 mg, yield 42%) as a colorless solid by synthesis in a similar manner to Reference Synthesis Example 33.

### Reference Synthesis Example 43

### 5-{[6-Methyl-4-(methylthio)-2-oxo-1,3,5-triazin-1(2H)-yl]methyl}pyridin-2-yl trifluoromethanesulfonate

5-({3-[Amino(methylthio)methylene]ureido}methyl)pyridin-2-yl trifluoromethanesulfonate (1.00 g, 2.69 mmol) synthesized in Reference Synthesis Example 41 was used instead of the Rf32 compound to obtain the title compound (Rf43) (568 mg, yield 53%) as a colorless oily product by synthesis in a similar manner to Reference Synthesis Example 34.

### Reference Synthesis Example 44

### 4-(4,5-Dihydro-1H-pyrazolo[3,4-c]pyridin-6(7H)-yl)-6-methyl-1-{[5-(trifluoromethyl)thi ophen-2-yl]methyl}-1,3,5-triazin-2(1H)-one

To a 1,4-dioxane solution (500 µL) of 4-(methylthio)-1-{[5-(trifluoromethyl)thiophen-2-yl]methyl}-1,3,5-triazin-2(1H)-one (161 mg, 0.500 mmol), 4,5,6,7-tetrahydro-1H-pyrazolo[3,4-c]pyridine dihydrochloride (118 mg, 0.600 mmol) and N,N-diisopropylethylamine (261 µL, 1.50 mmol) were added and the resultant reaction solution was stirred overnight under reflux by heating. After completion of the reaction, the reaction solution was purified by silica gel column chromatography (ethyl acetate/methanol = 1/0 → 8/1) to obtain the title compound (Rf44) (152 mg, yield 77%) as a colorless solid.

### Synthesis Example 1

### 4-[6-(Trifluoromethyl)-3,4-dihydroisoquinolin-2(1H)-yl]-1-{[5-(trifluoromethyl)thiophen -2-yl]methyl}-1,3,5-triazin-2(1H)-one

To a 1,4-dioxane solution (300 µL) of 4-(methylthio)-1-{[5-(trifluoromethyl)thiophen-2-yl]methyl}-1,3,5-triazin-2(1H)-one (30.7 mg, 0.100 mmol) synthesized in Reference Synthesis Example 33, 6-(trifluoromethyl)-1,2,3,4-tetrahydroisoquinoline hydrochloride (35.6 mg, 0.150 mmol) and triethylamine (27.9 µL, 0.200 mmol) were added and the resultant reaction solution was stirred for 4 hours under reflux by heating. After completion of the reaction, the reaction solution was concentrated and the obtained residue was purified by silica gel (amino-based) column chromatography (hexane/ethyl acetate = 1/1) to obtain the title compound (Ex1) (28.1 mg, yield 61 %) as a colorless solid.

### Synthesis Example 2

### 4-[6,7-Dimethoxy-3,4-dihydroisoquinolin-2(1H)-yl]-1-{[5-(trifluoromethyl)thiophen-2-yl ]methyl}-1,3,5-triazin-2(1H)-one

6,7-Dimethoxy-1,2,3,4-tetrahydroisoquinoline hydrochloride (34.5 mg, 0.150 mmol) was used instead of 6-(trifluoromethyl)-1,2,3,4-tetrahydroisoquinoline hydrochloride to obtain the title compound (Ex2) (yield 62%) as a colorless solid by synthesis in a similar manner to Synthesis Example 1.

### Synthesis Example 3

### 4-(4-Hydroxy-6-methoxy-3,4-dihydroisoquinolin-2(1H)-yl)-1-{[5-(trifluoromethyl)thiop hen-2-yl}methyl]-1,3,5-triazin-2(1H)-one

6-Methoxy-1,2,3,4-tetrahydroisoquinolin-4-ol hydrochloride (100 mg, 0.464 mmol) was used instead of 6-(trifluoromethyl)-1,2,3,4-tetrahydroisoquinoline hydrochloride to obtain the title compound (Ex3) (yield 21%) as a brown oily product by synthesis in a similar manner to Synthesis Example 1.

### Synthesis Example 4

### 4-(4-Hydroxy-6-methoxy-3,4-dihydroisoquinolin-2(1H)-yl)-6-methyl-1-{[5-(trifluoromet hyl)thiophen-2-yl]methyl}-1,3,5-triazin-2(1H)-one

6-Methyl-4-(methylthio)-1-{[5-(trifluoromethyl)thiophen-2-yl]methyl}-1,3,5-triazin -2(1H)-one (50.0 mg, 0.156 mmol) synthesized in Reference Synthesis Example 34 and 6-methoxy-1,2,3,4-tetrahydroisoquinolin-4-ol hydrochloride were used to obtain the title compound (Ex4) (35.0 mg, yield 50%) as a white solid by synthesis in a similar manner to Synthesis Example 1.

### Synthesis Example 5

### 6-Methyl-4-[6-(2,2,2-trifluoroethoxy)-3,4-dihydroisoquinolin-2(1H)-yl]-1-{[2-(trifluoro methyl)thiazol-5-yl]methyl}-1,3,5-triazin-2(1H)-one

6-Methyl-4-(methylthio)-1-{[2-(trifluoromethyl)thiazol-5-yl]methyl}-1,3,5-triazin-2 (1H)-one (25.0 mg, 0.0780 mmol) synthesized in Reference Synthesis Example 36 and 6-(2,2,2-trifluoroethoxy)-1,2,3,4-tetrahydroisoquinoline hydrochloride (23.0 mg, 0.0858 mmol) synthesized in Reference Synthesis Example 18 were used to obtain the title compound as a colorless solid (Ex5) (20.0 mg, yield 51%) by synthesis in a similar manner to Synthesis Example 1.

### Synthesis Example 6

### 2-(4-Oxo-5-{[5-(trifluoromethyl)thiophen-2-yl]methyl}-4,5-dihydro-1,3,5-triazin-2-yl)-1, 2,3,4-tetrahydroisoquinoline-7-carbonitrile

To a 1,4-dioxane solution (300 µL) of 4-(methylthio)-1-{[5-(trifluoromethyl)thiophen-2-yl]methyl}-1,3,5-triazin-2(1H)-one (30.7 mg, 0.100 mmol) synthesized in Reference Synthesis Example 33, 1,2,3,4-tetrahydroisoquinoline-7-carbonitrile (23.7 mg, 0.150 mmol) was added and the resultant reaction solution was stirred for 6 hours under reflux by heating. After completion of the reaction, the reaction solution was concentrated and the obtained residue was purified by silica gel (amino-based) column chromatography (hexane/ethyl acetate = 1/2) to obtain the title compound (Ex6) (13.4 mg, yield 39%) as a colorless solid.

### Synthesis Example 7

### 4-(3,4-Dihydroisoquinolin-2(1H)-yl)-1-{[5-(trifluoromethyl)thiophen-2-yl]methyl}-1,3,5 -triazin-2(1H)-one

1,2,3,4-Tetrahydroisoquinoline (18.8 µL, 0.150 mmol) was used instead of 1,2,3,4-tetrahydroisoquinoline-7-carbonitrile to obtain the title compound (Ex7) (27.4 mg, yield 70%) as a colorless solid by synthesis in a similar manner to Synthesis Example 6.

### Synthesis Example 8a

### 4-(4-Hydroxy-5-methoxy-3,4-dihydroisoquinolin-2(1H)-yl)-6-methyl-1-{[5-(trifluoromet hyl)thiophen-2-yl]methyl}-1,3,5-triazin-2(1H)-one and

### Synthesis Example 8b

### 4-(4-Hydroxy-7-methoxy-3,4-dihydroisoquinolin-2(1H)-yl)-6-methyl-1-{[5-(trifluoromet hyl) thiophen-2-yl]methyl}-1,3,5-triazin-2(1H)-one

To a 1,4-dioxane solution (1.0 mL) of 6-methyl-4-(methylthio)-1-{[5-(trifluoromethyl)thiophen-2-yl]methyl}-1,3,5-triazin-2(1H )-one (76.0 mg, 0.237 mmol) synthesized in Reference Synthesis Example 34, a mixture of 5-methoxy-1,2,3,4-tetrahydroisoquinolin-4-ol and 7-methoxy-1,2,3,4-tetrahydroisoquinolin-4-ol (85.0 mg, 0.474 mmol) was added and the resultant reaction solution was stirred for 8 hours under reflux by heating. After completion of the reaction, the reaction solution was concentrated under reduced pressure and the obtained residue was purified by silica gel thin-layer chromatography (ethyl acetate/chloroform) to obtain Synthesis Example 8a (Ex8a) (colorless amorphous product, 5.70 mg, yield 5%) and Synthesis Example 8b (Ex8b) (colorless amorphous product, 9.90 mg, yield 9%) being the title compounds.

### Synthesis Example 9a

### (R)-4-(4-Hydroxy-6-methoxy-3,4-dihydroisoquinolin-2(1H)-yl)-6-methyl-1-{[5-(trifluoromethyl)thiophen-2-yl]methyl}-1,3,5-triazin-2(1H)-one

### Synthesis Example 9b

### (S)-4-(4-Hydroxy-6-methoxy-3,4-dihydroisoquinolin-2(1H)-yl-6-methyl-1-{[5-(trifluoromethyl)thiophen-2-yl]methyl}-1,3,5-triazin-2(1H)-one

4-(4-Hydroxy-6-methoxy-3,4-dihydroisoquinolin-2(1H)-yl)-6-methyl-1-{[5-(trifluo romethyl)thiophen-2-yl]methyl}-1,3,5-triazin-2(1H)-one (15.0 mg, 0.0332 mmol) synthesized in Synthesis Example 4 was purified by preparative high performance liquid chromatography (gradient: hexane/ethanol 20/80 → 50/50, flow rate 12 mL/min, column: CHIRALPAK1 series, 5 µm, φ 20 mmx250 mm). A fraction containing a single optically-active substance that was eluted at a retention time of 32.55 minutes was concentrated to obtain Synthesis Example 9a (Ex9a) (2.54 mg, yield 17%) being the title compound as a colorless amorphous product and a fraction containing a single optically-active substance that was eluted at a retention time of 36.60 minutes was concentrated to obtain Synthesis Example 9b (Ex9b) (2.33 mg, yield 16%) being the title compound as a colorless amorphous product.

### Synthesis Example 10

### 5-{[4-(4-Hydroxy-6-methoxy-3,4-dihydroisoquinolin-2(1H)-yl)-6-methyl-2-oxo-1,3,5-tri azin-1(2H)-yl]methyl}pyridin-2-yl trifluoromethanesulfonate

To a 1,4-dioxane solution (2.0 mL) of 5-{[6-methyl-4-(methylthio)-2-oxo-1,3,5-triazin-1(2H)-yl]methyl}pyridin-2-yl trifluoromethanesulfonate (50.0 mg, 0.126 mmol) synthesized in Reference Synthesis Example 43, 6-methoxy-1,2,3,4-tetrahydroisoquinolin-4-ol (77.2 mg, 0.358 mmol) and triethylamine (0.250 mL, 1.79 mmol) were added and the resultant reaction solution was stirred for 20 hours under reflux by heating. After completion of the reaction, water was added to the reaction solution and the resultant mixture was extracted with ethyl acetate. The organic layer was concentrated under reduced pressure and the obtained residue was purified by silica gel column chromatography (hexane/ethyl acetate → ethyl acetate → acetone). To the obtained crude product, 2-propanol was added and the precipitated solid was collected by filtration and washed to obtain the title compound (Ex10) (2.50 mg, yield 3%) as a yellow solid.

### Synthesis Example 11

### 4-[6-(Difluoromethoxy)-3,4-dihydroisoquinolin-2(1H)-yl]-1-{[5-(trifluoromethyl)thiophe n-2-yl]methyl}-1,3,5-triazin-2(1H)-one

To a 1,4-dioxane solution (300 µL) of 4-(methylthio)-1-{{[5-(trifluoromethyl)thiophen-2-yl]methyl}-1,3,5-triazin-2(1H)-one (30.7 mg, 0.100 mmol) synthesized in Reference Synthesis Example 33, 6-(difluoromethoxy)-1,2,3,4-tetrahydroisoquinoline hydrochloride (28.3 mg, 0.120 mmol) synthesized in Reference Synthesis Example 14 and N,N- diisopropylethylamine (35.6 µL, 0.200 mmol) were added and the resultant reaction solution was stirred for 10 hours under reflux by heating. After completion of the reaction, the reaction solution was purified by silica gel column chromatography (hexane/ethyl acetate =1/1 → 1/2) to obtain the title compound (Ex11) (34.2 mg, yield 75%) as a colorless solid.

### Synthesis Example 12

### 2-(4-Oxo-5-{[5-(trifluoromethyl)thiophen-2-yl]methyl}-4,5-dihydro-1,3,5-triazin-2-yl)-1, 2,3,4-tetrahydroisoquinolin-6-yl trifluoromethanesulfonate

1,2,3,4-Tetrahydroisoquinolin-6-yl trifluoromethanesulfonate hydrochloride (38.1 mg, 0.120 mmol) synthesized in Reference Synthesis Example 16 was used instead of the Rf14 compound to obtain the title compound (Ex12) (yield 93%) as a colorless solid by synthesis in a similar manner to Synthesis Example 11.

### Synthesis Example 13

### 4-[6-(2,2,2-Trifluoroethoxy)-3,4-dihydroisoquinolin-2(1H)-yl]-1-{[5-(trifluoromethyl)thi ophen-2-yl]methyl}-1,3,5-triazin-2(1H)-one

6-(2,2,2-Trifluoroethoxy)-1,2,3,4-tetrahydroisoquinoline hydrochloride (32.1 mg, 0.120 mmol) synthesized in Reference Synthesis Example 18 was used instead of the Rf14 compound to obtain the title compound (Ex 13) (yield 81%) as a colorless solid by synthesis in a similar manner to Synthesis Example 11.

### Synthesis Example 14

### 4-[6-(Difluoromethoxy)-3,4-dihydroisoquinolin-2(1H)-yl]-6-methyl-1-{[5-(trifluorometh yl)thiophen-2-yl]methyl}-1,3,5-triazin-2(1H)-one

6-Methyl-4-(methylthio)-1-{[5-(trifluoromethyl)thiophen-2-yl]methyl}-1,3,5-triazin -2(1H)-one (16.5 mg, 50.0 µmol) synthesized in Reference Synthesis Example 34 and 6-(difluoromethoxy)-1,2,3,4-tetrahydroisoquinoline hydrochloride (21.2 mg, 90.0 µmol) synthesized in Reference Synthesis Example 14 were used to obtain the title compound (Ex14) (14.5 mg, yield 61%) as a colorless amorphous product by synthesis in a similar manner to Synthesis Example 11.

### Synthesis Example 15

### 4-[4-Hydroxy-6-(2,2,2-trifluoroethoxy)-3,4-dihydroisoquinolin-2(1H)-yl]-6-methyl-1-{[5 -(trifluoromethyl)thiophen-2-yl]methyl}-1,3,5-triazin-2(1H)-one

The crude product of 6-(2,2,2-trifluoroethoxy)-1,2,3,4-tetrahydroisoquinolin-4-ol hydrochloride synthesized in Reference Synthesis Example 20 was used instead of the Rf14 compound to obtain the title compound (Ex15) (yield 62%) as a light yellow amorphous product by synthesis in a similar manner to Synthesis Example 14.

### Synthesis Example 16

### 4-(6-Chloro-3,4-dihydro-2,7-naphthyridin-2(1H)-yl)-6-methyl-1-{[5-(trifluoromethyl)thi ophen-2-yl]methyl}-1,3,5-triazin-2(1H)-one

6-Chloro-1,2,3,4-tetrahydro-2,7-naphthyridine hydrochloride (24.5 mg, 0.120 mmol) was used instead of the Rf14 compound to obtain the title compound (Ex16) (yield 50%) as a colorless solid by synthesis in a similar manner to Synthesis Example 14.

### Synthesis Example 17

### 4-(2-Chloro-7,8-dihydro-1,6-naphthyridin-6(5H)-yl)-6-methyl-1-{[5-(trifluoromethyl)thi ophen-2-yl]methyl}-1,3,5-triazin-2(1H)-one

2-Chloro-5,6,7,8-tetrahydro-1,6-naphthyridine hydrochloride (24.5 mg, 0.120 mmol) was used instead of the Rf14 compound to obtain the title compound (Ex17) (yield 50%) as a light yellow solid by synthesis in a similar manner to Synthesis Example 14.

### Synthesis Example 18

### 4-(6-Ethoxy-4-hydroxy-3,4-dihydroisoquinolin-2(1H)-yl)-6-methyl-1-{[5-(trifluoromethyl l)thiophen-2-yl]methyl}-1,3,5-triazine-2(1H)-one

6-Ethoxy-1,2,3,4-tetrahydroisoquinolin-4-ol hydrochloride (4.50 mg, 19.6 µmol) was used instead of the Rf14 compound to obtain the title compound (Ex18) (yield 70%) as a yellow amorphous product by synthesis in a similar manner to Synthesis Example 14.

### Synthesis Example 19

### 6-Methyl-4-(2-methyl-6,7-dihydrothieno[3,2-c]pyridin-5(4H)-yl)-1-{[5-(trifluoromethyl)t hiophen-2-yl]methyl}-1,3,5-triazin-(1H)-one

2-Methyl-4,5,6,7-tetrahydrothieno[3,2-c]pyridine trifluoroacetate (72.2 mg, 0.270 mmol) was used instead of the Rf14 compound to obtain the title compound (Ex 19) (quantitative) as a colorless solid by synthesis in a similar manner to Synthesis Example 14.

### Synthesis Example 20

### 4-[2-(Hydroxymethyl)-6,7-dihydrothieno[3,2-c]pyridin-5(4H)-yl]-6-methyl-1-{[5-(trifluo romethyl)thiophen-2-yl]methyl}-1,3,5-triazin-2(1H)-one

(4,5,6,7-Tetrahydrothieno[3,2-c]pyridin-2-yl)methanol trifluoroacetate (51.0 mg, 0.180 mmol) was used instead of the Rf14 compound to obtain the title compound (Ex20) (quantitative) as a colorless solid by synthesis in a similar manner to Synthesis Example 14.

### Synthesis Example 21

### 4-(2-Methoxy-6,7-dihydrothiazolo[5,4-c]pyridin-5(4H)-yl)-6-methyl-1-{[5-(trifluoromet hyl)thiophen-2-yl]methyl}-1,3,5-triazin-2(1H)-one

The crude product of 2-methoxy-4,5,6,7-tetrahydrothiazolo[5,4-c]pyridine trifluoroacetate synthesized in Reference Synthesis Example 4 was used instead of the Rf14 compound to obtain the title compound (Ex21) (yield 27% through two steps) as a colorless solid by synthesis in a similar manner to Synthesis Example 14.

### Synthesis Example 22

### 4-(2-Methoxy-7,8-dihydro-1,6-naphthyridin-6(5H)-yl)-6-methyl-1-{[5-(trifluoromethyl) thiophen-2-yl]methyl}-1,3,5-triazin-2(1H)-one

2-Methoxy-5,6,7,8-tetrahydro-1,6-naphthyridine (19.7 mg, 0.120 mmol) was used instead of the Rf14 compound to obtain the title compound (Ex22) (yield 81 %) as a colorless solid by synthesis in a similar manner to Synthesis Example 14.

### Synthesis Example 23

### 4-(4-Hydroxy-6-(2,2,2-trifluoroethoxy)-3,4-dihydroisoquinolin-2(1H)-yl)-6-methyl-1-{[2 -(trifluoromethyl)thiazol-5-yl]methyl}-1,3,5-triazin-2(1H)-one

6-Methyl-4-(methylthio)-1-{[2-(trifluoromethyl)thiazol-5-yl]methyl}-1,3,5-triazin-2 (1H)-one (35.0 mg, 0.101 mmol) synthesized in Reference Synthesis Example 36 and the crude product of 6-(2,2,2-trifluoroethoxy)-1,2,3,4-tetrahydroisoquinolin-4-ol hydrochloride synthesized in Reference Synthesis Example 20 were used to obtain the title compound (Ex23) (20.6 mg, yield 55%) as a colorless solid by synthesis in a similar manner to Synthesis Example 11.

### Synthesis Example 24

### 4-(6-Methoxy-3,4-dihydroisoquinolin-2(1H)-yl)-6-methyl-1-{[2-(trifluoromethyl)thiazol-5-yl]methyl}-1,3,5-triazin-2(1H)-one

6-Methoxy-1,2,3,4-tetrahydroisoquinoline hydrochloride (40.0 mg, 0.200 mmol) was used instead of the Rf20 compound to obtain the title compound (Ex24) (24.6 mg, yield 56%) as a colorless amorphous product by synthesis in a similar manner to Synthesis Example 23.

### Synthesis Example 25

### 4-(4-Hydroxy-6-methoxy-3,4-dihydroisoquinolin-2(1H)-yl)-6-methyl-1-{[2-(trifluoromet hyl)thiazol-5-yl]methyl}-1,3,5-triazin-2(1H)-one

6-Methyl-4-(methylthio)-1-{[2-(trifluoromethyl)thiazol-5-yl]methyl}-1,3,5-triazin-2 (1H)-one (50.0 mg, 0.155 mmol) synthesized in Reference Synthesis Example 36 and 6-methoxy-1,2,3,4-tetrahydroisoquinolin-4-ol hydrochloride (43.5 mg, 0.202 mmol) were used to synthesize in a similar manner to Synthesis Example 23. To the obtained crude product, acetonitrile was added and the precipitated solid was collected by filtration and washed with ethyl acetate to obtain the title compound (Ex25) (32.8 mg, yield 47%) as a colorless solid.

### Synthesis Example 26

### 1-(4-Fluorobenzyl)-4-(4-hydroxy-6-methoxy-3,4-dihydroisoquinolin-2(1H)-yl)-6-methyl-1,3,5-triazin-2(1H)-one

1-(4-Fluorobenzyl)-6-methyl-4-(methylthio)-1,3,5-triazin-2(1H)-one (50.0 mg, 0.188 mmol) synthesized in Reference Synthesis Example 27 and 6-methoxy-1,2,3,4-tetrahydroisoquinolin-4-ol hydrochloride (61.0 mg, 0.283 mmol) were used to synthesize in a similar manner to Synthesis Example 11. The obtained crude product was purified by silica gel thin-layer chromatography (hexane/ethyl acetate =1/10) to obtain the title compound (Ex26) (19.2 mg, yield 25%) as a colorless amorphous product.

### Synthesis Example 27

### Ethyl 2-(6-methyl-4-oxo-5-{[5-(trifluoromethyl)thiophen-2-yl]methyl}-4,5-dihydro-1,3,5-triazi n-2-yl)-1,2,3,4-tetrahydroisoquinoline-3-carboxylate

6-Methyl-4-(methylthio)-1-{[5-(trifluoromethyl)thiophen-2-yl]methyl}-1,3,5-triazin -2(1H)-one (32.1 mg, 0.100 mmol) synthesized in Reference Synthesis Example 34 and ethyl 1,2,3,4-tetrahydroisoquinoline-3-carboxylate hydrochloride (31.4 mg, 0.130 mmol) were used to synthesize in a similar manner to Synthesis Example 11. The obtained crude product was purified by preparative reverse phase high-performance liquid chromatography (gradient: acetonitrile/water (0.1% formic acid) = 30/70 → 90/10, column: Waters XBridge C 18 5 µm, φ19 mmx 100 mm) to obtain the title compound (Ex27) (16.0 mg, yield 33%) as a yellow amorphous product.

### Synthesis Example 28

### 4-(5,8-Dichloro-3,4-dihydroisoquinolin-2(1H)-yl)-6-methyl-1-{[5-(trifluoromethyl)thiop hen-2-yl]nmethyl}-1,3,5-triazin-2(1H)-one

6-Methyl-4-(methylthio)-1-{{[5-(trifluoromethyl)thiophen-2-yl]methyl}-1,3,5-triazin -2(1H)-one (32.1 mg, 0.100 mmol) synthesized in Reference Synthesis Example 34 and 5,8-dichloro-1,2,3,4-tetrahydroisoquinoline hydrochloride (35.8 mg, 0.150 mmol) were used to obtain a crude product by synthesis in a similar manner to Synthesis Example 11. To the obtained crude product, diethyl ether was added and the precipitated solid was collected by filtration and washed to obtain the title compound (Ex28) (28.7 mg, yield 60%) as a colorless solid.

### Synthesis Example 29

### 4-(7-Methoxy-4,5-dihydro-1H-benzo[d]azepin-3(2H)-yl)-6-methyl-1-{[5-(trifluoromethy l)thiophen-2-yl]methyl}-1,3,5-triazin-2(1H)-one

7-Methoxy-2,3,4,5-tetrahydro-1H-benzo[d]azepine hydrochloride (25.6 mg, 0.120 mmol) was used instead of 5,8-dichloro-1,2,3,4-tetrahydroisoquinoline hydrochloride to obtain the title compound (Ex29) (9.50 mg, yield 21%) as a light yellow solid by synthesis in a similar manner to Synthesis Example 28.

### Synthesis Example 30

### 6-Methyl-4-(2-methyl-7,8-dihydro-1.6-naphthyridin-6(5H)-yl)-1-{[5-(trifluoromethyl)thi ophen-2-yl]methyl}-1,3,5-triazin-2(1H)-one

To a 1,4-dioxane solution (320 µL) of 6-methyl-4-(methylthio)-1-{[5-(trifluoromethyl)thiophen-2-yl]methyl}-1,3,5-triazin-2(1H )-one (32.1 mg, 0.100 mmol) synthesized in Reference Synthesis Example 34, 2-methyl-5,6,7,8-tetrahydro-1,6-naphthyridine hydrochloride (22.2 mg, 0.120 mmol) and N,N-diisopropylethylamine (34.0 µL, 0.200 mmol) were added and the resultant reaction solution was stirred for 9 hours under reflux by heating. After completion of the reaction, the reaction solution was concentrated and the obtained residue was purified by silica gel (amino-based) column chromatography (hexane/ethyl acetate = 41/59 → 0/100) to obtain the title compound (Ex30) (20.3 mg, yield 48%) as a light brown solid.

### Synthesis Example 31

### 4-(2-Cyclopropyl-7,8-dihydro-1,6-naphthyridin-6(5H)-yl)-6-methyl-1-{[5-(trifluorometh yl)thiophen-2-yl]methyl}-1,3,5-triazin-2(1H)-one

2-Cyclopropyl-5,6,7,8-tetrahydro-1,6-naphthyridine trifluoroacetate (13.5 mg, 0.0499 mmol) was used instead of 2-methyl-5,6,7,8-tetrahydro-1,6-naphthyridine hydrochloride to obtain the title compound (Ex31) (15.9 mg, yield 85%) as a colorless solid by synthesis in a similar manner to Synthesis Example 30.

### Synthesis Example 32

### 4-(4-Amino-6-methoxy-3,4-dihydroisoquinolin-2(1H)-yl)-6-methyl-1-{[5-(trifluorometh yl)thiophen-2-yl]methyl}-1,3,5-triazin-2(1H)-one

To a 1,4-dioxane solution (600 µL) of 6-methyl-4-(methylthio)-1-{[5-(trifluoromethyl)thiophen-2-yl]methyl}-1,3,5-triazin-2(1H )-one (15.0 mg, 0.0467 mmol) synthesized in Reference Synthesis Example 34, 6-methoxy-1,2,3,4-tetrahydroisoquinolin-4-amine (8.60 mg, 0.0483 mmol) synthesized in Reference Synthesis Example 23 was added and the resultant reaction solution was stirred for 13.5 hours under reflux by heating. After completion of the reaction, the reaction solution was purified by silica gel column chromatography (hexane/ethyl acetate = 1/1 → methanol) to obtain the title compound (Ex32) (1.11 mg, yield 37%) as a colorless solid.

### Synthesis Example 33

### 6-Methyl-4-[2-(2,2,2-trifluoroethyl)-4,5-dihydro-2H-pyrazolo[3,4-c]pyridin-6(7H)yl]-1-{[5-(trifluoromethyl)thiophen-2-yl]methyl}-1,3,5-triazin-2(1H)-one and, 6-Methyl-4-[1-(2,2,2-trifluoroethyl)-4,5-dihydro-1H-pyrazolo[3,4-c]pyridin-6(7H)-yl]-1-{[5-(trifluoromethyl)thiophen-2-yl]methyl}-1,3,5-triazin-2(1H)-one

To an acetonitrile solution (1.0 mL) of 4-(4,5-dihydro-1H-pyrazolo[3,4-c]pyridin-6(7H)-yl)-6-methyl-1-{[5-(trifluoromethyl)thi ophen-2-yl]methyl}-1,3,5-triazin-2(1H)-one (39.6 mg, 0.100 mmol) synthesized in Reference Synthesis Example 44, potassium carbonate (27.6 mg, 0.200 mmol) and 2,2,2-trifluoroethyl trifluoromethanesulfonate (28.8 µL, 0.200 mmol) were added and the resultant reaction solution was stirred at 80°C for 6 hours. After completion of the reaction, water was added to the reaction solution and the resultant mixture was extracted with ethyl acetate. The organic layer was dried over anhydrous sodium sulfate and concentrated under reduced pressure. The obtained residue was purified by silica gel thin-layer chromatography (ethyl acetate) to obtain the mixture of approximately 1:1 of the title compounds (Ex33) (13.1 mg, yield 27%) as a colorless solid.

### Synthesis Example 34

### 4-(Indolin-1-yl)-6-methyl-1-{[5-(trifluoromethyl)thiopen-2-yl]methyl}-1,3,5-triazin-2(1 H)-one

To a chloroform solution (1.0 mL) of 4-chloro-6-methyl-1-{[5-(trifluoromethyl)thiophen-2-yl]methyl}-1,3,5-triazin-2(1H)-one (31.0 mg, 0.100 mmol) synthesized in Reference Synthesis Example 25, indoline (22.0 µL, 0.200 mmol) was added and the resultant reaction solution was stirred at room temperature for 1 hour. After completion of the reaction, the reaction solution was concentrated and the obtained residue was purified by silica gel column chromatography (hexane/ethyl acetate =1/1) to obtain the title compound (Ex34) (10.0 mg, yield 25%) as a pink solid.

### Synthesis Example 35

### 4-(4-Fluoro-6-methoxy-3,4-dihydroisoquinolin-2(1H)-yl)-6-methyl-1-{[5-(trifluoromethy l)thiophen-2-yl]methyl}-1,3,5-triazin-2(1H)-one

To a dichloromethane solution (1.1 mL) of 4-(4-hydroxy-6-methoxy-3,4-dihydroisoquinolin-2(1H)-yl)-6-methyl-1-{[5-(trifluoromet hyl)thiophen-2-yl]methyl}-1,3,5-triazin-2(1H)-one (55.0 mg, 0.122 mmol) synthesized in Synthesis Example 4, N,N-diethylaminosulfur trifluoride (33.1 µL, 0.243 mmol) was added dropwise at 0°C and the resultant reaction solution was stirred at room temperature for 1 hour. After completion of the reaction, water was added to the reaction solution and the resultant mixture was extracted with dichloromethane. The organic layer was dried over magnesium sulfate and concentrated. The obtained residue was purified by silica gel column chromatography (hexane/ethyl acetate) to obtain the title compound (Ex35) (26.8 mg, yield 49%) as a white solid.

### Synthesis Example 36

### 6-Methoxy-2-(6-methyl-4-oxo-5-{[5-(trifluoromethyl)thiophen-2-yl]methyl}-4,5-dihydro -1,3,5-triazin-2-yl)-2.3-dihydroisoquinolin-4(1H)-one

To a dichloromethane solution (2.0 mL) of 4-(4-hydroxy-6-methoxy-3,4-dihydroisoquinolin-2(1H)-yl)-6-methyl-1-{[5-(trifluoromet hyl)thiophen-2-yl]methyl}-1,3,5-triazin-2(1H)-one (10.0 mg, 22.1 µmol) synthesized in Synthesis Example 4, 1,1,1-triacetoxy-1,1-dihydro-1,2-benziodoxol-3(1H)-one (14.1 mg, 33.2 µmol) was added and the resultant reaction solution was stirred at room temperature for 1 hour. After completion of the reaction, the reaction solution was purified by silica gel column chromatography (hexane/ethyl acetate =1/5 → 0/1) to obtain the title compound (Ex36) (5.10 mg, yield 51%) as a light yellow amorphous product.

### Synthesis Example 37

### 4-[4-(Hydroxyimino)-6-methoxy-3,4-dihydroisoquinolin-2(1H)-yl]-6-methyl-1-{[5-triflu oromethyl)thiophen-2-yl]methyl}-1,3,5-triazin-2(1H)-one

To a methanol solution (1.0 mL) of 6-methoxy-2-(6-methyl-4-oxo-5-{[5-(trifluoromethyl)thiophen-2-yl]methyl}-4,5-dihydro -1,3,5-triazin-2-yl)-2,3-dihydroisoquinolin-4(1H)-one (2.00 mg, 4.44 µmol) synthesized in Synthesis Example 36, hydroxylammonium sulfate (10.0 mg, 60.9 µmol) was added and the resultant reaction solution was stirred at 70°C for 1 hour. After completion of the reaction, the reaction solution was purified by silica gel column chromatography (ethyl acetate) to obtain the title compound (Ex37) (1.21 mg, yield 61%) as a colorless solid.

### Synthesis Example 38

### 4-(4-Hydroxy-6-isopropoxy-3,4-dihydroisoquinolin-2(1H)-yl)-6-methyl-1-{[5-(trifluoro methyl)thiophen-2-yl]methyl}-1,3,5-triazin-2(1H)-one

To a 1,4-dioxane solution (1.0 mL) of 6-methyl-4-(methylthio)-1-{[5-(trifluoromethyl)thiophen-2-yl]methyl}-1,3,5-triazin-2(1H )-one (16.9 mg, 0.0526 mmol) synthesized in Reference Synthesis Example 34, the crude product of 6-isopropoxy-1,2,3,4-tetrahydroisoquinolin-4-ol hydrochloride synthesized in Reference Synthesis Example 6, 1,8-diazabicyclo[5.4.0]undec-7-ene (11.6 µL, 0.116 mmol), and sodium iodide (7.90 mg, 0.00526 mmol) were added and the resultant reaction solution was stirred overnight under reflux by heating. After completion of the reaction, the reaction solution was concentrated and the obtained residue was purified by silica gel column chromatography (hexane/ethyl acetate =1/5) to obtain the title compound (Ex38) (11.0 mg, yield 44%) as a light yellow solid.

### Synthesis Example 39

### 4-(4-Hydroxy-6-isobutoxy-3,4-dihydroisoquinolin-2(1H)-yl)-6-methyl-1-{{5-trifluorom ethyl)thiophen-2-yl]methyl}-1,3,5-triazin-2(1H)-one

The crude product of 6-isobutoxy-1,2,3,4-tetrahydroisoquinolin-4-ol hydrochloride synthesized in Reference Synthesis Example 8 was used instead of the Rf6 compound to obtain the title compound (Ex39) (yield 25%) as a light yellow solid by synthesis in a similar manner to Synthesis Example 38.

### Synthesis Example 40

### 6-Methyl-4-[6-(2,2,2-trifluoroethoxy)-3,4-dihydroisoquinolin-2(1H)-yl]-1-{[5-(trifluoro methyl)thiophen-2-yl]methyl}-1,3,5-triazin-2(1H)-one

6-(2,2,2-Trifluoroethoxy)-1,2,3,4-tetrahydroisoquinoline hydrochloride (34.0 mg, 0.120 mmol) synthesized in Reference Synthesis Example 18 was used instead of the Rf6 compound to obtain the title compound (Ex40) (yield 76%) as a light yellow solid by synthesis in a similar manner to Synthesis Example 38.

### Synthesis Example 41

### 2-(6-Methyl-4-oxo-5-{[5-(trifluoromethyl)thiophen-2-yl]methyl}-4,5-dihydro-1,3,5-triazi n-2-yl)-1,2,3,4-tetrahydroisoquinolin-6-yltrifluoromethanesulfonate

1,2,3,4-Tetrahydroisoquinolin-6-yl trifluoromethanesulfonate hydrochloride (40.0 mg, 0.120 mmol) synthesized in Reference Synthesis Example 16 was used instead of the Rf6 compound to obtain the title compound (Ex41) (yield 76%) as a colorless solid by synthesis in a similar manner to Synthesis Example 38.

### Synthesis Example 42

### 4-[4,6-Dihydroxy-3,4-dihydroisoquinolin-2(1H)-yl]-6-methyl-1-{[5-(trifluoromethyl)thio phen-2-yl]methyl}-1,3,5-triazin-2(1H)-one

1,2,3,4-Tetrahydroisoquinoline-4,6-diol hydrochloride (98.8 mg, 0.490 mmol) was used instead of the Rf6 compound to obtain the title compound (Ex42) (yield 7%) as a light yellow solid by synthesis in a similar manner to Synthesis Example 38.

### Synthesis Example 43

### 6-Methyl-4-{[7-(trifluoromethyl)-3,4-dihydroisoquinolin-2(1H)-yl]-1-{5-(trifluoromethyl )thiopheh-2-yl]methy}-1,3,5-triazin-(1H)-one

7-(Trifluoromethyl)-1,2,3,4-tetrahydroisoquinoline hydrochloride (30.1 mg, 0.120 mmol) was used instead of the Rf6 compound to obtain the title compound (Ex43) (yield 66%) as a light yellow solid by synthesis in a similar manner to Synthesis Example 38.

### Synthesis Example 44

### 4-(3,4-dihydroisoquinolin-2(1H)-yl)-6-methyl-1-{[5-(trifluoromethyl)thiophen-2-yl]meth yl}-1,3,5-triazin-2(1H)-one

1,2,3,4-Tetrahydroisoquinoline (16.0 µL, 0.120 mmol) was used instead of the Rf6 compound to obtain the title compound (Ex44) (yield 68%) as a light yellow solid by synthesis in a similar manner to Synthesis Example 38.

### Synthesis Example 45

### 2-{[4-Hydroxy-(6-methyl-4-oxo-5-{[5-(trifluoromethyl)thiophen-2-yl]methyl}-4,5-dih ydro-1,3,5-triazin-2-yl)-1,2,3,4-tetrahydroisoquinolin-6-yl]oxy}acetamide

The crude product of 2-[(4-hydroxy-1,2,3,4-tetrahydroisoquinolin-6-yl)oxy]acetonitrile hydrochloride synthesized in Reference Synthesis Example 10 was used instead of the Rf6 compound to obtain the title compound (Ex45) (yield 10%) as a light yellow solid by synthesis in a similar manner to Synthesis Example 38.

### Synthesis Example 46

### 6-Methyl-4-[5-(2,2,2-trifluoroethoxy)isoindolin-2-yl]-1-{[5-(trifluoromethyl)thiophen-2-yl]methyl}-1,3,5-triazin-2(1H)-one

The crude product of 5-(2,2,2-trifluoroethoxy)isoindoline hydrochloride synthesized in Reference Synthesis Example 12 was used instead of the Rf6 compound to obtain the title compound (Ex46) (yield 26%) as a light yellow solid by synthesis in a similar manner to Synthesis Example 38.

### Synthesis Example 47

### 4-[6-(Cyclopropylmethoxy)-4-hydroxy-3,4-dihydroisoquinolin-2(1H)-yl]-6-methyl-1-{[5 -(trifluoromethyl)thiophen-2-yl]methyl}-1.3,5-triazin-2(1H)-one

The crude product of 6-(cyclopropylmethoxy)-1,2,3,4-tetrahydroisoquinolin-4-ol hydrochloride synthesized in Reference Synthesis Example 29 was used instead of the Rf6 compound to obtain the title compound (Ex47) (yield 24%) as a colorless solid by synthesis in a similar manner to Synthesis Example 38.

### Synthesis Example 48

### 4-(5-Methoxyisoindolin-2-yl)-6-methyl-1-{[5-(trifluoromethyl)thiophen-2-yl]methyl}-1, 3,5-triazin-2(1H)-one

5-Methoxyisoindoline hydrochloride (22.2 mg, 0.120 mmol) was used instead of the Rf6 compound to obtain a crude product by synthesis in a similar manner to Synthesis Example 38. To the obtained crude product, methanol was added and precipitated solid was collected by filtration to obtain the title compound (Ex48) (3.98 mg, yield 9%) as a colorless solid.

### Synthesis Example 49

### 4-(7-Methoxy-4,5-dihydro-1H-benzo[c]azepin-2(3H)-yl)-6-methyl-1-{[5-(trifluoromethyl )thiophen-2-yl]methyl-1,3,5-triazin}-2(1H)-one

To a 1,4-dioxane solution (1.0 mL) of 6-methyl-4-(methylthio)-1-{[5-(trifluoromethyl)thiophen-2-yl]methyl}-1,3,5-triazin-2(1H )-one (35.0 mg, 0.109 mmol) synthesized in Reference Synthesis Example 34, 7-methoxy-2,3,4,5-tetrahydro-1H-benzo[c]azepine hydrochloride (28.0 mg, 0.131 mmol) and 1,8-diazabicyclo[5.4.0]undec-7-ene (49.0 µL, 0.328 mmol) were added and the resultant reaction solution was stirred for 4 hours under reflux by heating. After completion of the reaction, the reaction solution was concentrated under reduced pressure and the obtained residue was purified by silica gel column chromatography (ethyl acetate/chloroform =1/1). To the obtained crude product, ethyl acetate and hexane were added and the precipitated solid was collected by filtration to obtain the title compound (Ex49) (24.0 mg, yield 47%) as a pink solid.

### Synthesis Example 50

### 4-(4-Hydroxy-5,6-dimethoxy-3,4-dihydroisoquinolin-2(1H)-yl)-6-methyl-1-{[5-(trifluoro methyl)thiophen-2-yl]methyl}-1,3,5-triazin-2(1H)-one

The crude product of 5,6-dimethoxy-1,2,3,4-tetrahydroisoquinolin-4-ol hydrocloride (23.0 mgl) synthesized in Reference Synthesis Example 30 was used instead of 7-methoxy-2,3,4,5-tetrahydro-1H-benzo[c]azepine hydrochloride to obtain the title compound (Ex50) (15.0 mg, yield 28%) as a pink solid by synthesis in a similar manner to Synthesis Example 49.

### Synthesis Example 51

### Ethyl 5-(6-methyl-4-oxo-5-{[5-(trifluoromethyl)thiophen-2-yl]methyl}-4,5-dihydro-1,3,5-triazi n-2-yl)-4,5,6,7-tetrahydrothieno[3,2-c]pyridine-2-carboxylate

To a 1,4-dioxane solution (1.0 mL) of 6-methyl-4-(methylthio)-1-{[5-(trifluoromethyl)thiophen-2-yl]methyl}-1,3,5-triazin-2(1H )-one (36.0 mg, 0.112 mmol) synthesized in Reference Synthesis Example 34, 4,5,6,7-tetrahydrothieno[3,2-c]pyridine-2-carboxylate hydrochloride (33.0 mg, 0.133 mmol) and 1,8-diazabicyclo[5.4.0]undec-7-ene (49.0 µL, 0.328 mmol) were added and the resultant reaction solution was stirred overnight under reflux by heating. After completion of the reaction, the reaction solution was concentrated under reduced pressure and the obtained residue was purified by silica gel column chromatography (ethyl acetate/methanol = 1/10) to obtain the title compound (Ex51) (48.0 mg, yield 91%) as a yellow oily product.

### Synthesis Example 52

### 4-(6-Fluoro-3,4-dihydroisoquinolin-2(1H)-yl)-6-methyl-1-{[5-(trifluoromethyl)thiophen-2-yl] methyl}-1,3,5-triazin-2(1H)-one

To a 1,4-dioxane solution (1.0 mL) of 6-methyl-4-(methylthio)-1-{[5-(trifluoromethyl)thiophen-2-yl]methyl}-1,3,5-triazin-2(1H )-one (42.0 mg, 0.131 mmol) synthesized in Reference Synthesis Example 34, 6-fluoro-1,2,3,4-tetrahydroisoquinoline hydrochloride (29.0 mg, 0.155 mmol) and triethylamine (54.0 µL, 0.387 mmol) were added and the resultant reaction solution was stirred for 14 hours under reflux by heating. After completion of the reaction, the reaction solution was concentrated under reduced pressure and the obtained residue was purified by silica gel column chromatography (hexane/ethyl acetate =1/1) to obtain the title compound (Ex52) (14.0 mg, yield 26%) as a yellow amorphous product.

### Synthesis Example 53

### 4-[6-(Difluoromethoxy)-4-hydroxy-3.4-dihydroisoquinolin-2(1H)-yl]-6-methyl-1-{[5-(tri fluoromethyl)thiophen-2-yl]methyl-1,3,5-triazin}-2(1H)-one

To a N,N-dimethylformamide solution (1.0 mL) of 4-[4,6-dihydroxy-3,4-dihydroisoquinolin-2(1H)-yl]-6-methyl-1-{[5-(trifluoromethyl)thio phen-2-yl]methyl}-1,3,5-triazin-2(1H)-one (5.00 mg, 11.4 µmol) synthesized in Synthesis Example 42, sodium chlorodifluoroacetate (2.10 mg, 13.7 µmol) and potassium carbonate (3.20 mg, 22.8 µmol) were added and the resultant reaction solution was stirred at 100°C for 1 hour. After completion of the reaction, the reaction solution was purified by silica gel column chromatography (hexane/ethyl acetate = 1/1 → 0/1) to obtain the title compound (Ex53) (0.810 mg, yield 15%) as a light yellow amorphous product.

### Synthesis Example 54

### 4-(8-Hydroxy-7,8-dihydro-[1,3]dioxolo[4,5-g]isoquinolin-6(5H)-yl)-6-methyl-1-{[5-(trifl uoromethyl)thiophen-2-yl]methyl}-1,3,5-triazin-2(1H)-one

5,6,7,8-Tetrahydro-[1,3]dioxolo[4,5-g]isoquinolin-8-ol (38.6 mg, 0.200 mmol) was used instead of 6-fluoro-1,2,3,4-tetrahydroisoquinoline hydrochloride to obtain the title compound (Ex54) (26.0 mg, yield 28%) as a colorless solid by synthesis in a similar manner to Synthesis Example 52.

### Synthesis Example 55

### 4-(2-Ethyl-4,5-dihydro-2H-pyrazolo[3,4-c]pvridin-6(7H)-yl)-6-methyl-1-{[5-(trifluorome thyl)thiophen-2-yl]methyl}-1,3,5-triazin-2(1H)-one and, 4-(1-Ethyl-4,5-dihydro-1H-pyrazolo[3,4-c]pyridin-6(7H)-yl)-6-methyl-1-{[5-(trifluorome thyl)thiophen-2-yl]methyl}-1,3,5-triazin-2(1H)-one

Iodoethane (78.0 mg, 0.500 mmol) was used instead of 2,2,2-trifluoroethyl trifluoromethanesulfonate to obtain an approximately 2:1 mixture (2-ethyl-dihydropyrazolopyridine form: 1-ethyl-dihydropyrazolopyridine form) of the title compounds (Ex55) (7.80 mg, yield 18%) as a yellow solid by synthesis in a similar manner to Synthesis Example 33.

### Synthesis Example 56

### 5-{[4-(4-Hydroxy-6-methoxy-3,4-dihydroisoquinolin-2(1H)-yl)-2-oxo-1,3,5-triazin-1(2H )-yl]methyl}pyridin-2-yl trifluoromethanesulfonate

5-{[4-(Methylthio)-2-oxo-1,3,5-triazin-1(2H)-yl]methyl}pyridin-2-yl trifluoromethanesulfonate (50.0 mg, 0.131 mmol) synthesized in Reference Synthesis Example 42 was used instead of the Rf43 compound to obtain the title compound (Ex56) (2.40 mg, yield 3%) as a white solid by synthesis in a similar manner to Synthesis Example 10.

### Synthesis Example 57

### 4-(7-Fluoro-3,4-dihydro-1H-pyrido[3,4-b]indol-2(9H)-yl)-6-methyl-1-{[5-(trifluorometh yl)thiophen-2-yl]methyl}-1,3,5-triazin-2(1H)-one

6-Methyl-4-(methylthio)-1-{[5-(trifluoromethyl)thiophen-2-yl]methyl}-1,3,5-triazin -2(1H)-one (8.45 mg, 0.0263 mmol) synthesized in Reference Synthesis Example 34 and 7-fluoro-2,3,4,9-tetrahydro-1H-pyrido[3,4-b]indole (5.00 mg, 0.0263 mmol) were used to obtain the title compound (Ex57) (1.90 mg, yield 16%) as a colorless solid by synthesis in a similar manner to Synthesis Example 6.

### Synthesis Example 58

### 4-(7-Fluoro-3,4-dihydro-1H-pyrido[3,4-b]indol-2(9H)-yl)-6-methyl-1-{[2-(trifluorometh yl)thiazol-5-yl]methyl}-1,3,5-triazin-2(1H)-one

6-Methyl-4-(methylthio)-1-{[2-(trifluoromethyl)thiazol-5-yl]methyl}-1,3,5-triazin-2 (1H)-one (10.0 mg, 0.0311 mmol) synthesized in Reference Synthesis Example 36 and 7-fluoro-2,3,4,9-tetrahydro-1H-pyrido[3,4-b]indole (6.50 mg, 0.0342 mmol) were used to obtain the title compound (Ex58) (1.60 mg, yield 11%) as a colorless solid by synthesis in a similar manner to Synthesis Example 6.

### Synthesis Example 59

### 4-(8-Fluoro-3,4-dihydrobenzo[4,5]imidazo[1,2-a]pyrazin-2(1H)-yl-6-methyl-1-{[5-(trifl uoromethyl)thiophen-2-yl]methyl}-1,3,5-triazin-2(1H)-one

6-Methyl-4-(methylthio)-1-{[5-(trifluoromethyl)thiophen-2-yl]methyl}-1,3,5-triazin -2(1H)-one (16.9 mg, 0.0530 mmol) synthesized in Reference Synthesis Example 34 and 8-fluoro-1,2,34-tetrahydrobenzo[4,5]imidazo[1,2-a]pyrazine (15.3 mg, 0.0800 mmol) were used to obtain the title compound (Ex59) (3.12 mg, yield 13%) as a colorless solid by synthesis in a similar manner to Synthesis Example 6.

The chemical structural formulae of the compounds synthesized in Reference Synthesis Examples and Synthesis Examples will be shown hereinafter. Tables 3 and 4 show the data of physical properties of the compounds synthesized in Reference Synthesis Examples, and Tables 5 to 7 show the data of physical properties of the compounds synthesized in Synthesis Examples. As described above, the sign Rf in the drawings means Reference Synthesis Example, and the sign Ex means Synthesis Example.

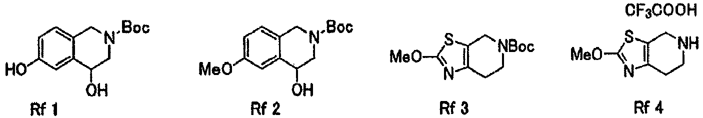

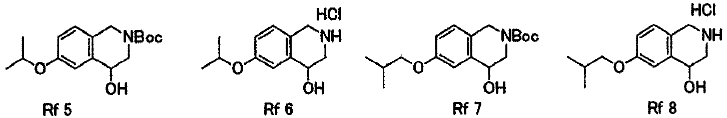

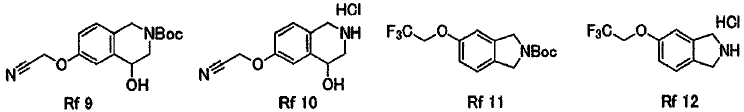

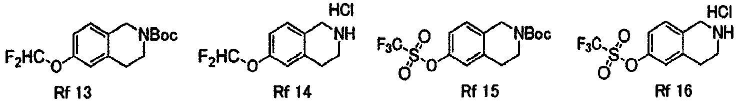

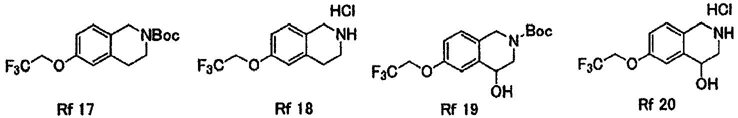

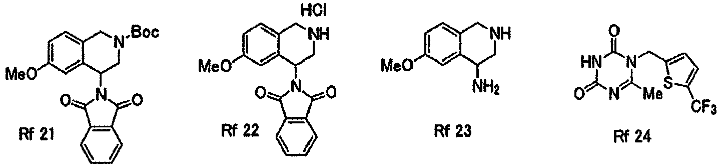

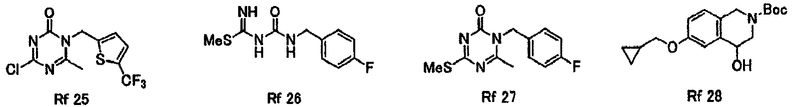

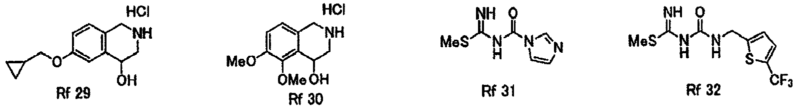

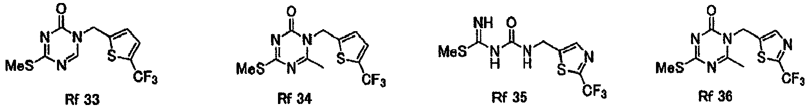

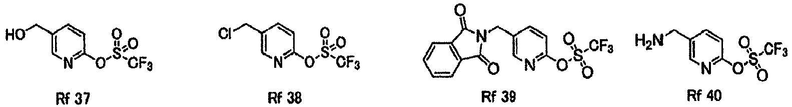

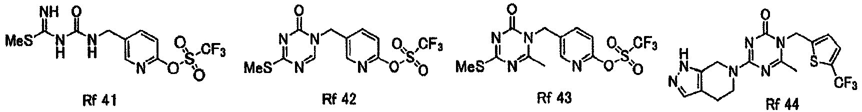

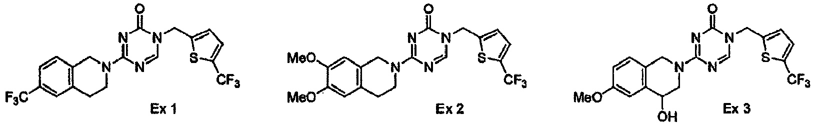

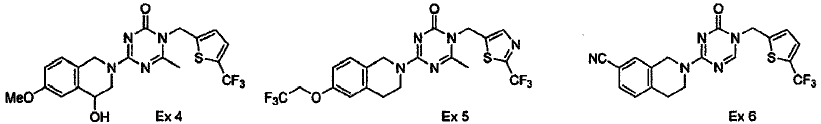

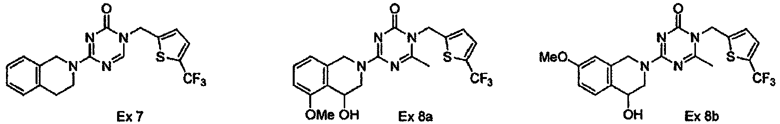

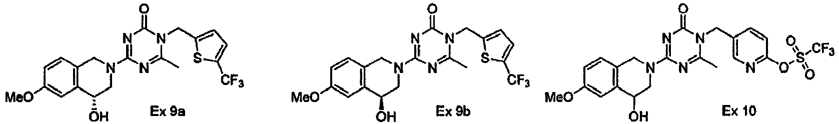

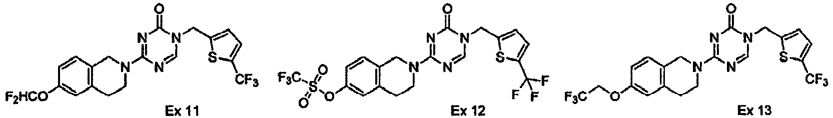

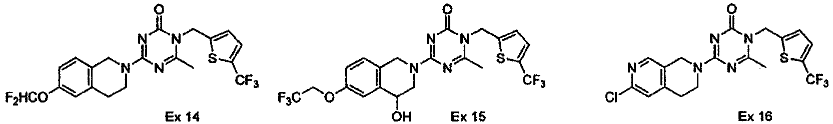

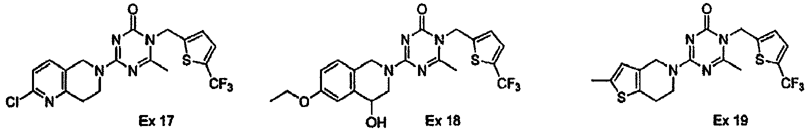

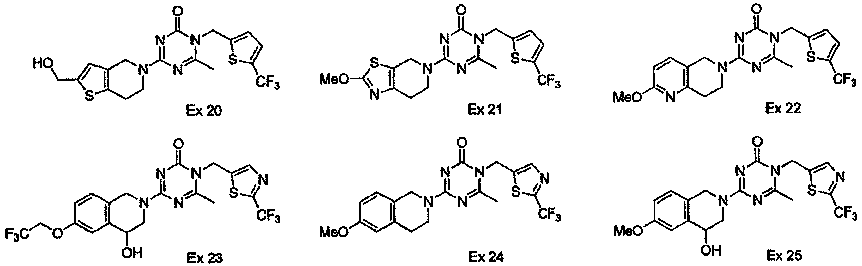

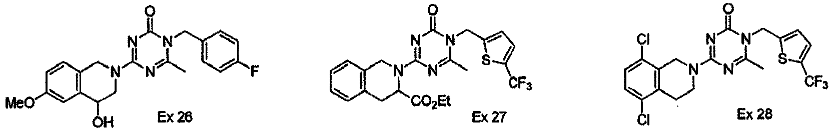

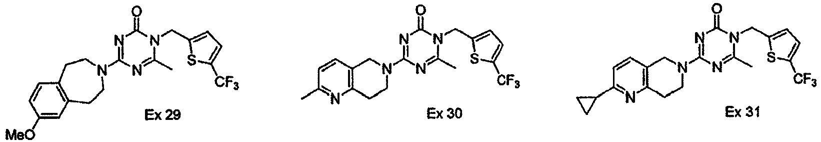

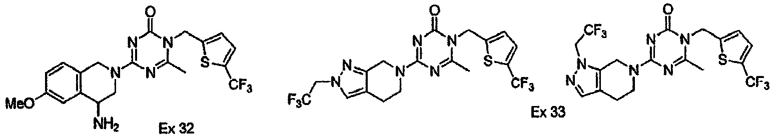

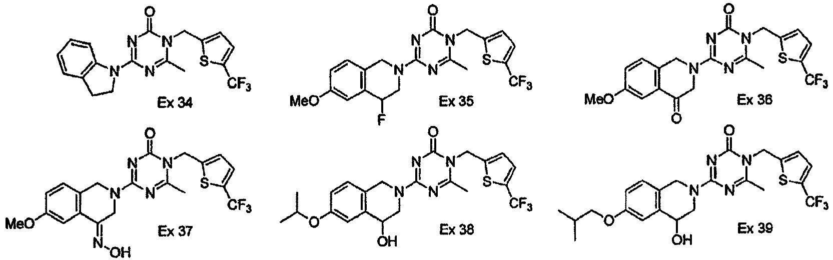

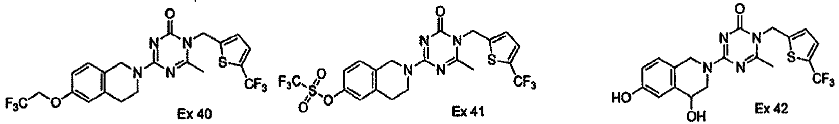

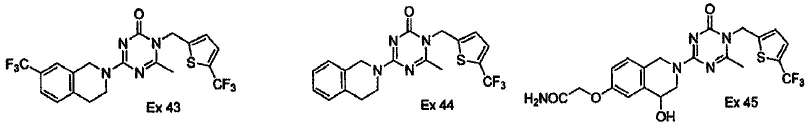

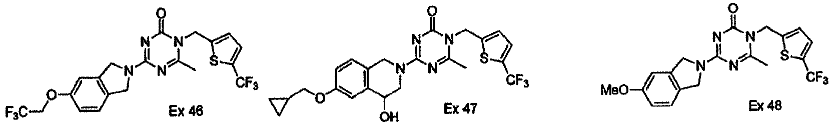

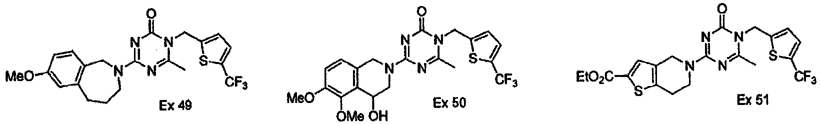

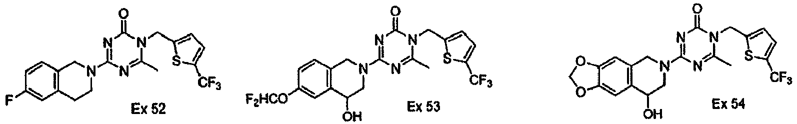

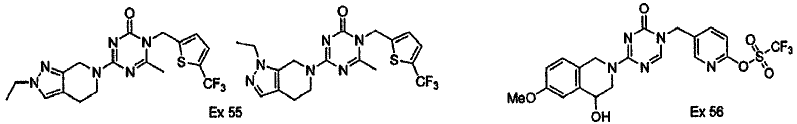

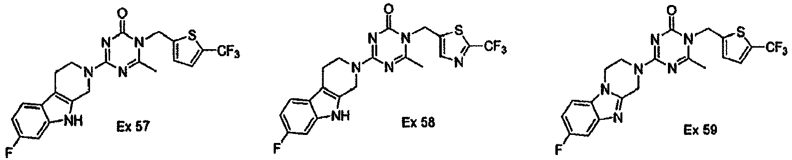

**Table 3**

| Rf | Data |
|---|---|
| 1 | LC/MS: cond. 1 RT 3.22 min LC/MS (ESI⁺) m/z; 266 [M+H]⁺ |
| 2 | LC/MS: cond. 1 RT 3.72 min LC/MS (ESI⁺) m/z; 280 [M+H]⁺ 1H-NMR (CDCl3) δ: 1.50 (s, 9H), 3.62-3.68(m, 1H), 3.83(s, 3H), 3.85-3.90(m, 1H), 4.38-4. 44 (m, 1H), 4.68-4.73(m, 2H), 6.84-6.87(m, 1H), 6.99-7.05(m, 2H). |
| 3 | LC/MS: cond. 1 RT 3.97 min LC/MS (ESI⁺) m/z; 271 [M+H]⁺ |
| 14 | LC/MS: cond. 1 RT 3. 74 min LC/MS (ESI⁺) m/z; 200 [M+H]⁺ |
| 15 | LC/MS: cond. 2 RT 2.93 min LC/MS (ESI⁺) m/z; 282 [M+H-Boc]⁺ |
| 16 | LC/MS: cond. 1 RT 1. 20 min LC/MS (ESI⁺) m/z; 282 [M+H]⁺ |
| 18 | LC/MS: cond. 1 RT 0.65 min LC/MS (ESI⁺) m/z; 232 [M+H]⁺ |
| 20 | LC/MS: cond. 2 RT 1.23 min LC/MS (ESI⁺) m/z; 248 [M+H]⁺ |
| 21 | LC/MS: cond. 2 RT 2. 61 min LC/MS (ESI⁺) m/z; 409 [M+H]⁺ |
| 22 | LC/MS: cond. 2 RT 1.51 min LC/MS (ESI⁺) m/z; 309 [M+H]⁺ |
| 23 | LC/MS: cond. 2 RT 0.33 min LC/MS (ESI⁺) m/z; 179 [M+H]⁺ |
| 24 | LC/MS: cond. 2 RT 1.73 min LC/MS (ESI⁺) m/z; 292 [M+H]⁺ LC/MS (ESI⁻) m/z; 290 [M-H]⁻ |
| 26 | LC/MS: cond. 2 RT 1.30 min LC/MS (ESI⁺) m/z; 242 [M+H]⁺ LC/MS (ESI⁻) m/z; 286 [M+HC02]⁻ |
| 27 | LC/MS: cond. 2 RT 1.87 min LC/MS (ESI⁺) m/z; 266 [M+H]⁺ |
| 31 | LC/MS: cond. 2 RT 0.40 min LC/MS (ESI⁺) m/z; 185 [M+H]⁺ 1H-MMR (DMSO-d6) δ: 2.49 (s, 3H), 7.02 (s, 1H), 7.60 (s, 1H), 8.28 (s, 1H), 9.26 (brs, 2H). |
| 32 | LC/MS: cond. 2 RT 1.68 min LC/MS (ESI⁺) m/z; 298 [M+H]⁺ |
| 33 | LC/MS: cond. 1 RT 3.72 min LC/MS (ESI⁺) m/z; 308 [M+H]⁺ 1H-MMR (CDC13) δ : 2.43 (s, 3H), 5.22 (s, 2H), 7.32 (s, 1H), 7.61 (s, 1H), 8.77 (s, 1H). |
| 34 | LC/MS: cond. 1 RT 3.84 min LC/MS (ESI⁺) m/z; 322 [M+H]⁺ |
| 35 | LC/MS: cond. 2 RT 1.37 min LC/MS (ESI⁺) m/z; 299 [M+H]⁺ LC/MS (ESI⁻) m/z; 297 [M-H]⁻ |
| 36 | LC/MS: cond. 2 RT 1.93 min LC/MS (ESI⁺) m/z; 323 [M+H]⁺ LC/MS (ESI⁻) m/z; 321 [M-H]⁻ |
| 37 | LC/MS: cond. 2 RT 1.90 min LC/MS (ESI⁺) m/z; 258 [M+H]⁺ |

**Table 4**

| Rf | Data |
|---|---|
| 38 | 1H-NMR (CDCl3) δ: 4.61 (s, 2H), 7.19 (d, J = 8.6 Hz, 1H), 7.94 (dd, J = 2.5 Hz, 8.2 Hz, 1H), 8.39 (d, J = 2.4 Hz, 1H). |
| 41 | LC/MS: cond. 2 RT 1.78 min LC/MS (ESI⁺) m/z; 373 [M+H]⁺ |
| 44 | LC/MS: cond. 2 RT 2.03 min LC/MS (ESI⁺) m/z; 397 [M⁺H]⁺ |

**Table 5**

| Ex | Data |
|---|---|
| 1 | LC/MS: cond. 2 RT 2.70 min LC/MS (ESI⁺) m/z; 461 [M+H]⁺ |
| 2 3 | LC/MS: cond. 2 RT 2.32 min LC/MS (ESI⁺) m/z; 453 [M+H]⁺ LC/MS: cond. 2 RT 2.14 min LC/MS (ESI⁺) m/z; 439 [M+H]⁺ |
| 4 | LC/MS: cond. 1 RT 3.88 min LC/MS (ESI⁺) m/z; 453 [M+H]⁺ |
| 5 | LC/MS: cond. 2 RT 2.59 min LC/MS (ESI⁺) m/z; 506 [M+H]⁺ LC/MS (ESI⁻) m/z; 504 [M+H]⁻ |
| 6 | LC/MS: cond. 2 RT 2.35 min LC/MS (ESI⁺) m/z; 418 [M+H]⁺ |
| 7 | LC/MS: cond. 2 RT 2.35 min LC/MS (ESI⁺) m/z; 418 [M+H]⁺ |
| 8a | 1H-MMR (CDC13) δ : 2.50(d, J = 8.25 Hz, 3H), 3. 63-3. 82 (m, 1H), 3. 88 (s, 3H), 4.56-4.80(m, 2H), 5.10-5.41(m, 4H), 6.76-6.82(m, 2H), 7.03(d, J = 2.88, 1H), 7.22-7.29(m, 2H). LC/MS: cond. 2 RT 2.26 min LC/MS (ESI⁺) m/z; 453 [M+H]⁺ |
| 8b | 1H-NMR (CDC13) δ :2.51 (d, J = 4.53 Hz, 3H), 3.63-3.88(m, 4H), 4.52-4.89(m, 3H), 5.23-5.40(m, 3H), 6.67(s, 1H), 6.81(dd, J = 8.64, 2.46, 1H), 7. 03 (d, J = 3.72, 1H), 7.25-7.36(m, 2H). LC/MS: cond. 2 RT 2.23 min LC/MS (ESI⁺) m/z; 453 [M+H]⁺ |
| 9a | LC/MS: cond. 2 RT 2.24 min LC/MS (ESI⁺) m/z; 453 [M+H]⁺ LC/MS (ESI⁻) m/z; 451 [M+H]⁻ |
| 9b | LC/MS: cond. 2 RT 2.24 min LC/MS (ESI⁺) m/z; 453 [M+H]⁺ LC/MS (ESI⁻) m/z; 451 [M+H]⁻ |
| 10 | LC/MS: cond. 1 RT 3.92 min LC/MS (ESI⁺) m/z; 528 [M+H]⁺ |
| 11 | LC/MS: cond. 1 RT 4.29 min LC/MS (ESI⁺) m/z; 459 [M+H]⁺ LC/MS (ESI⁻) m/z; 503 [M+HC02]⁻ |
| 12 | LC/MS: cond. 1 RT 4.57 min LC/MS (ESI⁺) m/z; 541 [M+H]⁺ LC/MS (ESI⁺) m/z; 585 [M+HC02]⁻ |
| 13 | LC/MS: cond. 1 RT 4.45 min LC/MS (ESI⁺) m/z; 491 [M+H]⁺ LC/MS (ESI⁺) m/z; 535 [M+HC02]⁻ |
| 14 | LC/MS: cond. 2 RT 2.65 min LC/MS (ESI⁺) m/z; 473 [M+H]⁺ |
| 15 | LC/MS: cond. 2 RT 2.47 min LC/MS (ESI⁺) m/z; 521 [M+H]⁺ LC/MS (ESI⁻) m/z; 519 [M-H]⁻ |
| 16 | LC/MS: cond. 2 RT 2.37 min LC/MS (ESI⁺) m/z; 442 [M+H]⁺ |
| 17 | LC/MS: cond. 2 RT 2.39 min LC/MS (ESI⁺) m/z; 442 [M+H]⁺ |
| 18 | LC/MS: cond. 2 RT 2.37 min LC/MS (ESI⁺) m/z; 467 [M+H]⁺ LC/MS (ESI⁻) m/z; 465 [M+H]⁻ |
| 19 | LC/MS: cond. 2 RT 2.75 min LC/MS (ESI⁺) m/z; 427 [M+H]⁺ |

**Table 6**

| Ex | Data |
|---|---|
| 20 | LC/MS: cond. 2 RT 2. 20 min LC/MS (ESI⁺) m/z; 443 [M+H]⁺ |
| 21 | LC/MS: cond. 1 RT 3. 97 min LC/MS (ESI⁺) m/z; 444 [M+H]⁺ |
| 22 | LC/MS: cond. 2 RT 2.41 min LC/MS (ESI⁺) m/z; 438 [M+H]⁺ |
| 23 | LC/MS: cond. 2 RT 2. 30 min LC/MS (ESI⁺) m/z; 522 [M+H]⁺ LC/MS (ESI⁻) m/z; 520 [M+H]⁻ |
| 24 | LC/MS: cond. 2 RT 2.39 min LC/MS (ESI⁺) m/z; 438 [M+H]⁺ |
| 25 | LC/MS: cond. 2 RT 2. 04 min LC/MS (ESI⁺) m/z; 454 [M+H]⁺ LC/MS (ESI⁻) m/z; 452 [M+H]⁻ |
| 26 | 1H-NMR (CDCl3) δ : 2.12-2. 18 (m, 1H), 2. 33-2. 38 (m, 3H), 3. 77-4. 03 (m, 1H), 3. 81 (s, 3H), 4. 31-4.58(m, 1H), 4.62-4.92(m, 2H), 5.07-5.29(m, 3H), 6.83-6.91(m, 1H), 6.96-7.14(m, 4H), 7.18-7.28(m, 2H) LC/MS: cond. 2 RT 2.02 min LC/MS (ESI⁺) m/z; 397 [M+H]⁺ |
| 27 | LC/MS: cond. 2 RT 2.69 min LC/MS (ESI⁺) m/z; 479 [M+H]⁺ |
| 28 | LC/MS: cond. 2 RT 2.97 min LC/MS (ESI⁺) m/z; 475 [H+H]⁺ |
| 29 | LC/MS: cond. 2 RT 2. 63 min LC/MS (ESI⁺) m/z; 451 [M+H]⁺ |
| 30 | LC/MS: cond. 2 RT 1. 71 min LC/MS (ESI⁺) m/z; 422 [M+H]⁺ |
| 31 | LC/MS: cond. 2 RT 1. 90 min LC/MS (ESI⁺) m/z; 448 [M+H]⁺ |
| 32 | LC/MS: cond. 2 RT 1. 90 min LC/MS (ESI⁺) m/z; 452 [M+H]⁺ |
| 33 | LC/MS: cond. 2 RT 2. 43 min LC/MS (ESI⁺) m/z; 479 [M+H]⁺ |
| 34 | LC/MS: cond. 2 RT 2.61 min LC/MS (ESI⁺) m/z; 393 [M+H]⁺ |
| 35 | LC/MS: cond. 2 RT 2. 50 min LC/MS (ESI⁺) m/z; 455 [M+H]⁺ |
| 36 | LC/MS: cond. 2 RT 2. 42 min LC/MS (ESI⁺) m/z; 451 [M+H]⁺ |
| 37 | LC/MS: cond. 2 RT 2.34 min LC/MS (ESI⁺) m/z; 466 [M+H]⁺ LC/MS (ESI⁻) m/z; 464 [M-H]⁻ |
| 38 | LC/MS: cond. 2 RT 2.46 min LC/MS (ESI⁺) m/z; 481 [M+H]⁺ |
| 39 | LC/MS: cond. 2 RT 2. 67 min LC/MS (ESI⁺) m/z; 495 [M+H]⁺ LC/MS (ESI⁻) m/z; 493 [M+H]⁻ |
| 40 | 1H-NMR (CDC13) δ : 2.50(s, 3H), 2.88(dd, J = 14.0, 6.2 Hz, 2H), 4.03-4.07(m, 2H), 4.33(q, J = 8.1 Hz, 2H), 4.91(d, J = 4.1 Hz, 2H), 5.29(s, 3H), 6.74(brs, 1H), 6.79-6.82(m, 1H), 7.04(brs, 1H), 7.07-7.12(m, 1H), 7.28-7.29(m, 1H), LC/MS: cond. 2 RT 2. 75 min LC/MS (ESI⁺) m/z; 505 [M+H]⁺ LC/MS (ESI⁻) m/z; 503 [M-H]⁻ |

**Table 7**

| Ex | Data |
|---|---|
| 41 | 1H-NMR (CDC13) δ : 2. 51 (s, 3H), 2. 94 (dd, J = 41, 6. 2 Hz, 2H), 4. 09 (dd, J = 5. 9, 5. 9 Hz, 2H) , 5. 29 (s, 2H) , 4. 99 (s, 2H), 7.04-7.13(m, 3H), 7. 20-7. 25 (m, 1H), 7. 28-7.30 (m, 1H). LC/MS: cond. 2 RT 2.85 min LC/MS (ESI⁺) m/z: 555 [N+H]⁺ LC/MS (ESI⁻) m/z; 553 [M-H]⁻ |
| 42 | LC/MS: cond. 2 RT 2.00 min LC/MS (ESI⁺) n/z; 439 [N+H]⁺ LC/MS (ESI⁻) m/z; 437 [M-H]⁻ |
| 43 | LC/MS: cond. 2 RT 2.81 min LC/MS (ESI⁺) m/z; 475 [M+H]⁺ |
| 44 | LC/MS: cond. 2 RT 2.62 min LC/MS (ESI⁺) m/z; 407 [N+H]⁺ |
| 45 | LC/MS: cond. 2 RT 1.92 min LC/MS (ESI⁺) m/z; 496 [M+H]⁺ LC/NS (ESI⁻) m/z; 494 (M-H]⁻ |
| 46 | LC/MS: cond. 2 RT 2.67 min LC/(MS (ESI⁺) m/z; 491 [W+H]⁺ |
| 47 | LC/MS (ESI⁺) m/z: 493 [M+H]⁺ LC/MS (ESI⁻) m/z; 491 [M+H]⁻ |
| 48 | LC/MS: cond. 2 RT 2.49 min LC/MS (ESI⁺) m/z; 423 [M+H]⁺ |
| 49 | LC/MS: cond. 2 RT 2.62 min LC/MS (ESI⁺) m/z; 451 [M+H]⁺ |
| 50 | 1H-NMR (CDCl3) δ : 2.55(d, J = 16 Hz, 3H), 3.70-3.77(m, 1H), 3. 86 (s, 3H), 3.88(s, 3H), 4. 53-4. 91 (m, 3H), 5. 21-5. 42(m, 3H), 6. 62 (d, J = 9. 5 Hz, 1H), 6.92(d, J = 8. 3 Hz, 1H), 7.04-7.07(m, 1H), 7.26-7.29(m, 1H). LC/MS: cond. 2 RT 2.12 min LC/MS (ESI⁺) m/z; 483 [N+H]⁺ LC/MS (ESI⁻) m/z; 481 [M-H]⁻ |
| 51 | LC/MS: cond. 2 RT 2.67 min LC/MS (ESI⁺) m/z; 485 [N+H]⁺ LC/MS (ESI⁻) m/z; 483 [M+H]⁻ |
| 52 | LC/MS: cond. 2 RT 2.63 min LC/MS (ESI⁺) m/z; 425 [M+H]⁺ |
| 53 | LC/MS: cond. 2 RT 2.36 min LC/MS (ESI⁺) m/z; 489 [M+H]⁺ LC/MS (ESI⁻) m/z; 487 [M+H]⁻ |
| 54 | LC/MS: cond. 2 RT 2.20 min LC/MS (ESI⁺) m/z; 467 [M+H]⁺ LC/MS (ESI⁻) m/z; 465 [M+H]⁻ |
| 55 | LC/MS: cond. 2 RT 2.23 min LC/MS (ESI⁺) m/z; 425 [M+H]⁺ |
| 56 | LC/MS: cond. 1 RT 3.77 min LC/MS (ESI⁺) m/z; 514 [M+H]⁺ |
| 57 | LC/MS: cond. 2 RT 2.66 min LC/MS (ESI⁺) m/z; 464 [M+H]⁺ LC/MS (ESI⁻) m/z; 462 [M+H]⁻⁻ |
| 58 | LC/MS: cond. 2 RT 2.49 min LC/MS (ESI⁺) m/z; 465 [M+H]⁺ |
| 59 | LC/MS: cond. 2 RT 2.09 min LC/MS (ESI⁺) m/z; 465 [N+H]⁺ LC/MS (ESI⁻) m/z; 463 [M+H]⁻ |

### Pharmacological analysis

The pharmacological analysis on the compound of the present invention will be described next.

### 1. Calcium influx inhibition assay

Human T-type calcium channel (Cav 3.2) expressing HEK293 cells were obtained from Prof. Edward Perez-Reyes, University Virginia, USA. The calcium-sensitive fluorescent dye used was FLIPR Calcium 4 Assay Kit (Molecular Devices).

To a black 96-well plate with a clear bottom coated with type I collagen, the human T-type calcium channel (Cav 3.2) expressing HEK293 cells were seeded and cultured overnight, and the culture medium was removed. A solution of the calcium-sensitive fluorescent dye was added, and the whole was incubated at 37°C in an atmosphere of 5% carbon dioxide for 30 minutes. To the plate, a diluted solution of a compound was added, and the whole was further incubated at 37°C in an atmosphere of 5% carbon dioxide for 30 minutes. While fluorescence was continuously analyzed from the bottom with a FlexStation 3 (Molecular Devices), a calcium solution was added. The increase in the fluorescence due to calcium influx caused by the stimulus was observed for 70 seconds. From the rise in the fluorescence from the base line, the inhibition percentage was calculated. The logarithms of compound concentrations were plotted with respect to the inhibition activities to obtain an IC₅₀ value (50% inhibition concentration).

The IC₅₀ values of all the compounds of Synthesis Examples showed 10 µM or less. Table 8 shows the resulting T-type calcium channel inhibition concentrations of the compounds of Synthesis Examples.

**(Table 8)**

| Synthesis Example No. | | | C₅₀ (µM) |
|---|---|---|---|
| | 1 | | 0. 0 6 2 |
| | 2 | | 0. 5 4 |
| | 3 | | 0. 0 9 5 |
| | 4 | | 0. 0 2 0 |
| | 5 | | 0. 3 2 |
| | 6 | | 1. 8 |
| | 7 | | 0. 4 7 |
| | 8 | a | 0. 0 6 3 |
| | 8 | b | 0. 7 9 |
| | 9 | a | 0. 0 0 4 9 |
| | 9 | b | 0. 1 3 |
| 1 | 0 | | 0. 3 2 |
| 1 | 1 | | 0. 0 3 4 |
| 1 | 2 | | 0. 0 5 4 |
| 1 | 3 | | 0. 0 5 5 |
| 1 | 4 | | 0. 0 4 5 |
| 1 | 5 | | 0. 0 1 0 |
| 1 | 6 | | 0. 6 5 |
| 1 | 7 | | 0. 3 5 |
| 1 | 8 | | 0. 0 1 8 |
| 1 | 9 | | 0. 2 6 |
| 2 | 0 | | 0. 4 0 |
| 2 | 1 | | 0. 3 6 |
| 2 | 2 | | 0. 3 1 |
| 2 | 3 | | 0. 3 4 |
| 2 | 4 | | 0. 9 4 |
| 2 | 5 | | 0. 4 5 |
| 2 | 6 | | 3. 0 |
| 2 | 7 | | 2. 8 |
| 2 | 8 | | 0. 3 2 |
| 2 | 9 | | 0. 0 4 0 |
| 3 | 0 | | 3. 2 |
| 3 | 1 | | 0. 2 4 |
| 3 | 2 | | 0. 0 2 7 |
| 3 | 3 | | 4. 5 |
| 3 | 4 | | 0. 7 5 |
| 3 | 5 | | 0. 0 5 5 |
| 3 | 6 | | 0. 5 2 |
| 3 | 7 | | 0. 1 3 |
| 3 | 8 | | 0. 0 3 8 |
| 3 | 9 | | 0. 0 7 5 |
| 4 | 0 | | 0. 2 5 |
| 4 | 1 | | 0. 0 5 0 |
| 4 | 2 | | 0. 2 9 |
| 4 | 3 | | 0. 3 2 |
| 4 | 4 | | 0. 3 2 |
| 4 | 5 | | 0. 3 2 |
| 4 | 6 | | 0. 3 6 |
| 4 | 7 | | 0. 0 5 2 |
| 4 | 8 | | 1. 9 |
| 4 | 9 | | 0. 4 8 |
| 5 | 0 | | 0. 4 1 |
| 5 | 1 | | 0. 3 4 |
| 5 | 2 | | 0. 2 9 |
| 5 | 3 | | 0. 0 1 8 |
| 5 | 4 | | 0. 0 4 2 |
| 5 | 5 | | 5. 4 |
| 5 | 6 | | 6. 0 3 5 |
| 5 | 7 | | 0. 0 0 6 4 |
| 5 | 8 | | 0. 0 5 2 |
| 5 | 9 | | 0. 2 7 |

### Formulation Example 1

Granules containing the following components are produced.

| | | |
|---|---|---|
| Component | Compound of Formula (I) | 10 mg |
| | Lactose | 700 mg |
| | Cornstarch | 274 mg |
| | HPC-L | 16 mg |
| | Total | 1,000 mg |

The compound of Formula (I) and lactose are sieved through a 60-mesh sieve. Cornstarch is sieved through a 120-mesh sieve. The sieved materials are mixed in a V-type mixer. To the mixed powder, an aqueous solution of low-viscosity hydroxypropylcellulose (HPC-L) is added, then the whole is kneaded and granulated (extruding granulation, a hole diameter of 0.5 mm to 1 mm), and the granules are dried. The resulting dried granules are sieved through a vibrating screen (12/60 mesh) to obtain granules.

### Formulation Example 2

Powders that are to be filled into capsules and contain the following components and capsules are produced.

| | | |
|---|---|---|
| Component | Compound of Formula (I) | 10 mg |
| | Lactose | 79 mg |
| | Cornstarch | 10 mg |
| | Magnesium stearate | 1 mg |
| | | |
| | Total | 100 mg |

The compound of Formula (I) and lactose are sieved through a 60-mesh sieve. Cornstarch is sieved through a 120-mesh sieve. The sieved materials and magnesium stearate are mixed in a V-type mixer to obtain a 10% mixture. Into a No. 5 hard gelatin capsule, 100 mg of the obtained 10% mixture is filled to obtain the capsule.

### Formulation Example 3

Granules that are to be filled into capsules and contain the following components and capsules are produced.

| | | |
|---|---|---|
| Component | Compound of Formula (I) | 15 mg |
| | Lactose | 90 mg |
| | Cornstarch | 42 mg |
| | HPC-L | 3 mg |
| | Total | 150 mg |

The compound of Formula (I) and lactose are sieved through a 60-mesh sieve. Cornstarch is sieved through a 120-mesh sieve. The sieved materials are mixed in a V-type mixer. To the mixed powder, an aqueous solution of low-viscosity hydroxypropylcellulose (HPC-L) is added, then the whole is kneaded and granulated, and the granules are dried. The resulting dried granules are sieved through a vibrating screen (12/60 mesh), and 150 mg of the sieved granules are filled into a No. 4 hard gelatin capsule to obtain the capsule.

### Formulation Example 4

Tablets containing the following components are produced.

| | | |
|---|---|---|
| Component | Compound of Formula (I) | 10 mg |
| | Lactose | 90 mg |
| | Microcrystalline cellulose | 30 mg |
| | Magnesium stearate | 5 mg |
| | CMC-Na | 15 mg |
| | Total | 150 mg |

The compound of Formula (I), lactose, microcrystalline cellulose, and CMC-Na (sodium carboxymethylcellulose) are sieved through a 60-mesh sieve and mixed. To the mixed powder, magnesium stearate is added to obtain a mixed powder for formulation. The mixed powder is directly compressed to form 150-mg tablets.

### Formulation example 5

An intravenous formulation is prepared as follows:

| | |
|---|---|
| Compound of Formula (I) | 100 mg |
| Saturated fatty acid glyceride | 1,000 mL |

Usually, the formulated solution is intravenously administered to a patient at a speed of 1 mL/min.

### INDUSTRIAL APPLICABILITY

The compound of the present invention has an excellent T-type calcium channel inhibitory activity and is specifically useful for prevention or treatment of pains, such as chronic pains and acute pains including neuropathic pain, inflammatory pain, cancer pain, and visceral pain, which are caused by diabetic neuropathy, postherpetic neuralgia, trigeminal neuralgia, phantom limb pain, postoperative pain, stump pain, traumatic neurological disorder, carpal tunnel syndrome, plexus neuropathy, glossopharyngeal neuralgia, laryngeal neuralgia, migraine, fibromyalgia syndrome, rheumatoid arthritis, multiple sclerosis, HIV, herpes simplex, syphilis, carcinomatous neuropathy, polyneuropathy, causalgia, low back pain, complex regional pain syndrome (CRPS), thalamic pain, osteoarthritis, spinal cord injury, and cerebral apoplexy.

## Claims

1. A compound of Formula (I): 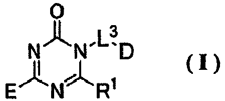 [wherein
R¹ means
a hydrogen atom
a halogen atom,
a C₁₋₆ alkyl group,
a C₁₋₆ alkoxy group,
a C₁₋₆ alkylthio group,
a mono-C₁₋₆ alkylamino group,
a di-C₁₋₆ alkylamino group,
(the C₁₋₆ alkyl group, the C₁₋₆ alkoxy group, the C₁₋₆ alkylthio group, the mono-C₁₋₆ alkylamino group, and the di-C₁₋₆ alkylamino group are unsubstituted or substituted with one or more substituents identically or differently selected from a substituent group V⁸) or,
a C₃₋₁₁ cycloalkyl group
(the C₃₋₁₁ cycloalkyl group is unsubstituted or substituted with one or more substituents identically or differently selected from a substituent group V⁶));
E means
a 7 to 14-membered non-aromatic fused heterocyclic group
(the 7 to 14-membered non-aromatic fused heterocyclic group is bonded to a carbon atom of a triazine skeleton in Formula (I) at a non-aromatic ring side; the non-aromatic ring side is unsubstituted or substituted with one of more substituents identically or differently selected prom a substituent group V⁹; an oxo group, a thioxo group, or a hydroxyimino group is optionally substituted for hydrogen atoms of a methylene group in the non-aromatic ring; and an aromatic ring side is unsubstituted or substituted with one or more substituents identically or differently selected from the substituent group V⁶ (when the aromatic ring side has two or more substituents, the two substituents optionally form a ring together);
L³ means
a C₁₋₆ alkylene group
(the C₁₋₆ alkylene group is unsubstituted or substituted with one or more substituents identically or differently selected from the substituent group V⁸ and one methylene group in the C₁₋₆ alkylene group is optionally replaced by a carbonyl group (>C=O) or a thiocarbonyl group (>C=S));
D means
a C₆₋₁₄ aryl group,
a 5 to 10-membered heteroaryl group,
or a 7 to 14-membered non-aromatic fused heterocyclic group
(the C₆₋₁₄ aryl group, the 5 to 10-membered heteroaryl group, and the 7 to 14-membered non-aromatic fused heterocyclic group are unsubstituted or substituted with one or more substituents identically or differently selected from the substituent group V⁶);
the substituent group V⁶ means a substituent group consisting of substituents constituting the substituent group V⁸, C₁₋₆ alkyl groups, C₂₋₆ alkenyl groups, and C₂₋₆ alkynyl groups (the C₁₋₆ alkyl groups, the C₂₋₆ alkenyl groups, and the C₂₋₆ alkynyl groups are unsubstituted or substituted with one or more substituents identically or differently selected from a substituent group V¹);
the substituent group V⁸ means a substituent group consisting of substituents constituting a substituent group V^{a}, C₁₋₁₀ alkoxy groups, C₆₋₁₄ aryloxy groups, C₁₋₆ alkylthio groups, C₁₋₆ alkylcarbonyl groups, C₁₋₆ alkylsulfonyl groups, C₁₋₆ alkoxycarbonyl groups, mono-C₁₋₆ alkylamino groups, di-C₁₋₆ alkylamino groups, mono-C₁₋₆ alkylaminocarbonyl groups, di-C₁₋₆ alkylaminocarbonyl groups, mono-C₁₋₆ alkylaminosulfonyl groups, di-C₁₋₆ alkylaminosulfonyl groups, C₁₋₆ alkylcarbonylamino groups, C₁₋₆ alkylcarbonyloxy groups, C₁₋₆ alkylsulfonylamino groups, C₁₋₆ alkylsulfonyloxy groups (the C₁₋₁₀ alkoxy groups, the C₆₋₁₄ aryloxy groups, the C₁₋₆ alkylthio groups, the C₁₋₆ alkylcarbonyl groups, the C₁₋₆ alkylsulfonyl groups, the C₁₋₆ alkoxycarbonyl groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the mono-C₁₋₆ alkylaminosulfonyl groups, the di-C₁₋₆ alkylaminosulfonyl groups, the C₁₋₆ alkylcarbonylamino groups, the C₁₋₆ alkylcarbonyloxy groups, the C₁₋₆ alkylsulfonylamino groups, and the C₁₋₆ alkylsulfonyloxy groups are unsubstituted or substituted with one or more substituents identically or differently selected from the substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylsulfonylamino groups, C₃₋₆ cycloalkylsulfonyloxy groups, C₃₋₆ cycloalkylthio groups, C₃₋₁₁ cycloalkyl groups, 3 to 11-membered heterocyclyl groups, C₆₋₁₄ aryl groups, 5 to 10-membered heteroaryl groups, or 7 to 14-membered non-aromatic fused heterocyclic groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylsulfonylamino groups, the C₃₋₆ cycloalkylsulfonyloxy groups, the C₃₋₆ cycloalkylthio groups, the C₃₋₁₁ cycloalkyl groups, the 3 to 11-membered heterocyclyl groups, the C₆₋₁₄ aryl groups, the 5 to 10-membered heteroaryl groups, and the 7 to 14-membered non-aromatic fused heterocyclic groups are unsubstituted, or substituted with one or more substituents identically or differently selected from a substituent group V²);
the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group;
the substituent group V¹ means a substituent group consisting of substituents constituting the substituent group V^{a}; C₁₋₆ alkoxy groups, C₁₋₃ haloalkoxy groups, mon-C₁₋₆ Alkylamino groups, di-C₁₋₆ alkylamino groups, mono-C₁₋₆ alkylaminocarbonyl groups, di-C₁-₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more hydroxy groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di C₁₋₆ alkylaminocarbonyl groups, one or more C₁₋₆ alkylcarbonylamino groups, one or more C₁₋₆ alkylthio groups, or one or more C₁₋₆ alkylsulfonyl groups), C₃₋₁₁ cycloalkyl groups, C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 to 11-membered heterocyclyl groups, C₆₋₁₄ aryl groups, 5 to 10-membered heteroaryl groups, or 7 to 14-membered non-aromatic fused heterocyclic groups (the Z₃₋₁₁ cycloalkyl groups, the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 11-membered heterocyclyl groups, the C₆₋₁₄ aryl groups, 5 to 10-membered heteroaryl groups, and 7 to 14-membered non-aromatic fused heterocyclic groups are unsubstituted or substituted with one or more hydroxyl groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkyl groups, one or more C₁₋₃ halo alkyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di C₁₋₆ alkylaminocarbonyl groups, one or more C₁₋₆ alkylcarbonylamino groups, one or more C₁₋₆ alkylthio groups, or one or more C₁₋₆ alkylsulfonyl groups);
the substituent group V² means a substituent group consisting of substituents constituting the substituent group V¹, C₁₋₆ alkyl groups, and C₁₋₃ haloalkyl groups; and
the substituent group V⁹ means substituents constituting the substituent group V^{a}, C₁₋₆ alkoxycarbonyl groups, C₁₋₆ alkylsulfonyloxy groups, C₁₋₆ alkylsulfonylamino groups, mono-C₁₋₆ alkylaminocarbonyl groups, di C₁₋₆ alkylaminocarbonyl groups, C₁₋₆ alkylcarbonylamino groups, C₁₋₆ alkylcarbonyloxy groups, mono-C₁₋₆ alkylaminosulfonyl groups, or di-C₁₋₆ alkylaminosulfonyl groups], a tautomer of the compound, or a pharmaceutically acceptable salt thereof, or a solvate thereof.

2. A compound of Formula (I): 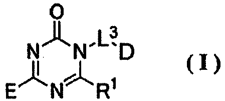 [wherein
R¹ means
a hydrogen atom,
a halogen atom,
a C₁₋₆ alkyl group,
a C₁₋₆ alkoxy group,
a C₁₋₆ alkylthio group,
a mono-C₁₋₆ alkylamino group,
a di-C₁₋₆ alkylamino group,
(the C₁₋₆ alkyl group, the C₁₋₆ alkoxy group, the C₁₋₆ alkylthio group, the mono-C₁₋₆ alkylamino group, and the di-C₁₋₆ alkylamino group are unsubstituted or substituted with one or more substituents identically or differently selected from the substituent group V⁸),
or
a C₃₋₁₁ cycloalkyl group
(the C₃₋₁₁ cycloalkyl group is unsubstituted or substituted with one or more substituents identically or differently selected from the substituent group V⁶);
E means
a 7 to 14-membered non-aromatic fused heterocyclic group,
(the 7 to 14-membered non-aromatic fused heterocyclic group is bonded to a carbon atom of a triazine skeleton in Formula (I) at a non-aromatic ring side; the non-aromatic ring side is unsubstituted or substituted with one or more substituents identically or differently selected from a substituent group V⁹; and an aromatic ring side is unsubstituted or substituted with one or more substituents identically or differently selected from the substituent group V⁶ (when the aromatic ring side has two or more substituents, the two substituents optionally form a ring together));
L³ means
a C₁₋₆ alkylene group,
(the C₁₋₆ alkylene group is unsubstituted or substituted with one or more substituents identically or differently selected from the substituent group V⁸ and one methylene group in the C₁₋₆ alkylene group is optionally replaced by a carbonyl group (>C=O) or a thiocarbonyl group (>C=S));
D means
a C₆₋₁₄ aryl group,
a 5 to 10-membered heteroaryl group, or
a 7 to 14-membered non-aromatic fused heterocyclic group
(the C₆₋₁₄ aryl group, the 5 to 10-membered heteroaryl group, and the 7 to 14-membered n_{df}r4i$f_{f}t&tici fused heterocyclic group are unsubstituted or substituted with one or more substituents identically or differently selected from the substituent group V⁶);
the substituent group V⁶ means a substituent group consisting of substituents constituting the substituent group V⁸, C₁₋₆ alkyl groups, C₂₋₆ alkenyl groups, and C₁₋₆ alkynyl groups (the C₁₋₆ alkyl groups, the C₂₋₆ alkenyl groups, and the C₂₋₆ alkynyl groups are unsubstituted or substituted with one or more substituents identically or differently selected from the substituent group V¹);
the substituent group V⁸ is a substituent group consisting of substituents constituting a substituent group V^{a}, C₁₋₁₀ alkoxy groups, C₆₋₁₄ aryloxy groups, C₁₋₆ alkylthio groups, C₁₋₆ alkylcarbonyl groups, C₁₋₆ alkylsulfonyl groups, C₁₋₆ alkoxycarbonyl groups, mono-C₁₋₆ alkylamino groups, di-C₁₋₆ alkylamino groups, mono-C₁₋₆ alkylaminocarbonyl groups, di-C₁₋₆ alkylaminocarbonyl groups, mono-C₁₋₆ alkylaminosulfonyl groups, di-C₁₋₆ alkylaminosulfonyl groups, C₁₋₆ alkylcarbonylamino groups, C₁₋₆ alkylcarbonyloxy groups, C₁₋₆ alkylsulfonylamino groups, C₁₋₆ alkylsulfonyloxy groups (the C₁₋₁₀ alkoxy groups, the C₆₋₁₄ aryloxy groups, the C₁₋₆ alkylthio groups, the C₁₋₆ alkylcarbonyl groups, the C₁₋₆ alkylsulfonyl groups, the C₁₋₆ alkoxycarbonyl groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the mono-C₁₋₆ alkylaminosulfonyl groups, the di-C₁₋₆ alkylaminosulfonyl groups, the C₁₋₆ alkylcarbonylamino groups, the C₁₋₆ alkylcarbonyloxy groups, the C₁₋₆ alkylsulfonylamino groups, and the C₁₋₆ alkylsulfonyloxy groups are unsubstituted or substituted with one or more substituents identically or differently selected from the substituent group V¹), C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylsulfonylamino groups, C₃₋₆ cycloalkylsulfonyloxy groups, C₃₋₆ cycloalkylthio groups, C₃₋₁₁ cycloalkyl groups, 3 to 11-membered heterocyclyl groups, C₆₋₁₄ aryl groups, 5 to 10-membered heteroaryl groups, or 7 to 14-membered non-aromatic fused heterocyclic groups (the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylsulfonylamino groups, the C₃₋₆ cycloalkylsulfonyloxy groups, the C₃₋₆ cycloalkylthio groups, the C₃₋₁₁ cycloalkyl groups, the 3 to 11-membered heterocyclyl groups, the C₆₋₁₄ aryl groups, the 5 to 10-membered heteroaryl groups, and the 7 to 14-membered non-aromatic fused heterocyclic groups are unsubstituted or substituted with one or more substituents identically or differently selected from a substituent group V²);
the substituent group V^{a} means a substituent group consisting of a hydroxy group, halogen atoms, a cyano group, a nitro group, an amino group, a carboxy group, a carbamoyl group, a sulfamoyl group, a phosphono group, a sulfo group, a tetrazolyl group, a formate group, and a formyl group;
the substituent group V¹ means a substituent group consisting of substituents constituting the substituent group V^{a}, C₁₋₆ alkoxy groups, C₁₋₃ haloalkoxy groups, mono-C₁₋₆ alkylamino groups, di-C₁₋₆ alkylamino groups, mono-C₁₋₆ alkylaminocarbonyl groups, di-C₁₋₆ alkylaminocarbonyl groups, C₁₋₆ alkylcarbonylamino groups, C₁₋₆ alkylthio groups, C₁₋₆ alkylsulfonyl groups, C₁₋₆ alkoxycarbonyl groups, C₁₋₆ alkylcarbonyl groups, C₁₋₆ alkylcarbonyloxy groups, C₆₋₁₄ arylcarbonyl groups, C₆₋₁₄ arylcarbonyloxy groups (the C₁₋₆ alkoxy groups, the mono-C₁₋₆ alkylamino groups, the di-C₁₋₆ alkylamino groups, the mono-C₁₋₆ alkylaminocarbonyl groups, the di-C₁₋₆ alkylaminocarbonyl groups, the C₁₋₆ alkylcarbonylamino groups, the C₁₋₆ alkylthio groups, the C₁₋₆ alkylsulfonyl groups, the C₁₋₆ alkoxycarbonyl groups, the C₁₋₆ alkylcarbonyl groups, the C₁₋₆ alkylcarbonyloxy groups, the C₆₋₁₄ arylcarbonyl groups, and the C₆₋₁₄ arylcarbonyloxy groups are unsubstituted or substituted with one or more hydroxyl groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di-C₁₋₆ alkylaminocarbonyl groups, one or more C₁₋₆ alkylcarbonylamino groups, one or more C₁₋₆ alkylthio groups, or one or more C₁₋₆ alkylsulfonyl groups), C₃₋₁₁ cycloalkyl groups, C₃₋₆ cycloalkoxy groups, mono-C₃₋₆ cycloalkylamino groups, di-C₃₋₆ cycloalkylamino groups, C₃₋₆ cycloalkylcarbonyl groups, C₃₋₆ cycloalkylsulfonyl groups, C₃₋₆ cycloalkylthio groups, 3 tol 1-membered heterocyclyl groups, C₆₋₁₄ aryl groups, 5 to 10-membered heteroaryl groups, or 7 to 14-membered non-aromatic fused heterocyclic groups (the C₃₋₁₁ cycloalkyl groups, the C₃₋₆ cycloalkoxy groups, the mono-C₃₋₆ cycloalkylamino groups, the di-C₃₋₆ cycloalkylamino groups, the C₃₋₆ cycloalkylcarbonyl groups, the C₃₋₆ cycloalkylsulfonyl groups, the C₃₋₆ cycloalkylthio groups, the 3 to 11-membered heterocyclyl groups, the C₆₋₁₄ aryl groups, 5 to 10-membered heteroaryl groups, and 7 to 14-membered non-aromatic fused heterocyclic groups are unsubstituted or substituted with one or more hydroxy groups, one or more halogen atoms, one or more cyano groups, one or more nitro groups, one or more amino groups, one or more carboxy groups, one or more carbamoyl groups, one or more sulfamoyl groups, one or more phosphono groups, one or more phosphinoyl groups, one or more sulfo groups, one or more sulfino groups, one or more tetrazolyl groups, one or more formyl groups, one or more C₁₋₆ alkyl groups, one or more C₁₋₃ haloalkyl groups, one or more C₁₋₆ alkoxy groups, one or more C₁₋₃ haloalkoxy groups, one or more mono-C₁₋₆ alkylamino groups, one or more di-C₁₋₆ alkylamino groups, one or more mono-C₁₋₆ alkylaminocarbonyl groups, one or more di C₁₋₆ alkylaminocarbonyl groups, one or more C₁₋₆ alkylcarbonylamino groups, one or more C₁₋₆ alkylthio groups, or one or more C₁₋₆ alkylsulfonyl groups);
the substituent group V² means a substituent group consisting of substituents constituting the substituent group V¹, C₁₋₆ alkyl groups, and C₁₋₃ haloalkyl groups; and
the substituent group V⁹ means substituents constituting the substituent group V^{a}, C₁₋₆ alkoxycarbonyl groups, C₁₋₆ alkylsulfonyloxy groups, C₁₋₆ alkylsulfonylamino groups, mono-C₁₋₆ alkylaminocarbonyl groups, di C₁₋₆ alkylaminocarbonyl groups, C₁₋₆ alkylcarbonylamino groups, C₁₋₆ alkylcarbonyloxy groups, mono-C₁₋₆ alkylaminosulfonyl groups, or di-C₁₋₆ alkylaminosulfonyl groups],
a tautomer of the, compound, or a pharmaceutically acceptable salt thereof, or a solvate thereof.

3. The compound, tautomer of the compound, or pharmaceutically acceptable salt thereof, or solvate thereof according to claim 1 or 2, wherein L³ is a C₁₋₃ alkylene group.

4. The compound, tautomer of the compound, or pharmaceutically acceptable salt thereof, or solvate thereof according to claim 3, wherein L³ is a methylene group.

5. The compound, tautomer of the compound, or pharmaceutically acceptable salt thereof, or solvate thereof according to any one of claims 1 to 4, wherein R¹ is a hydrogen atom or a C₁₋₆ alkyl group.

6. The compound, tautomer of the compound, or pharmaceutically acceptable salt thereof, or solvate thereof according to claim 5, wherein R¹ is a hydrogen atom or a methyl group.

7. The compound, tautomer of the compound, or pharmaceutically acceptable salt thereof, or solvate thereof according to any one of claims 1 to 6, wherein E is any one of Formula (II)-1 to Formula (II)-11 shown in (II): 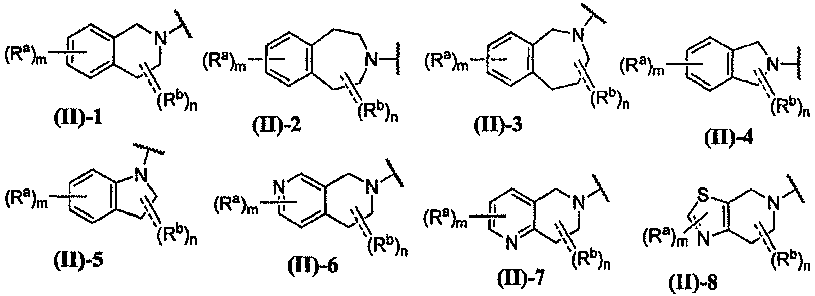 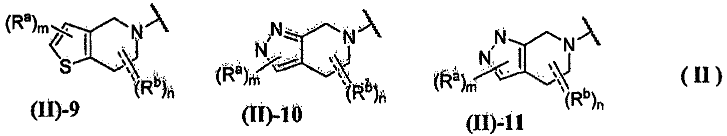 (where
R^{b} means
a hydrogen atom,
a hydroxy group,
an amino group,
a halogen atom,
a C₁₋₆ alkoxycarbonyl group
an oxo group, or
a hydroxyimino group;
n is 1;
R^{a} means a hydrogen atom,
a cyano group,
a halogen atom,
a hydroxy group,
an amino group,
a C₁₋₆ alkyl group,
a C₃₋₆ cycloalkyl group,
a C₁₋₆ alkoxy group,
a C₁₋₆ haloalkylsulfonyloxy group,
a C₁₋₆ haloalkyl group,
a C₁₋₆ haloalkoxy group,
a C₁₋₆ alkyl group substituted with one hydroxy group,
a C₁₋₆ alkoxy group substituted with one acetamido group, or
a C₁₋₆ alkoxy group substituted with one C₃₋₆ cycloalkyl group; and
m is I or 2; when m is 2, R^{a}s are the same as or different from each other and when m is 2 and two R^{a}s are adjacent, the two R^{a}s optionally form a methylenedioxy group).

8. The compound, tautomer of the compound, or Pharmaceutically acceptable salt thereof, or solvate thereof according to claim 7, wherein E is Formula (III): 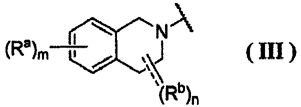 (wherein
R^{b} means
a hydrogen atom,
a hydroxy group,
an amino group,
a halogen atom,
a C₁₋₆ alkoxycarbonyl group,
an oxo group, or
a hydroxyimino group;
n is 1;
R^{a} means a hydrogen atom,
a cyano group,
a halogen atom,
a hydroxy group,
an amino group,
a C₁₋₆ alkyl group,
a C₃₋₆ cycloalkyl group,
a C₁₋₆ alkoxy group,
a C₁₋₆ haloalkylsulfonyloxy group,
a C₁₋₆ haloalkyl group,
a C₁₋₆ haloalkoxy group,
a C₁₋₆ alkyl group substituted with one hydroxy group,
a C₁₋₆ alkoxy group substituted with one acetamido group, or
a C₁₋₆ alkoxy group substituted with one C₃₋₆ cycloalkyl group; and m is 1 or 2; when m is 2, R^{a}s are the same as or different from each other and when m is 2 and two R^{a}s are adjacent, the two R^{a}s optionally form a methylenedioxy group).

9. The compound, tautomer of the compound, or pharmaceutically acceptable salt thereof, or solvate thereof according to any one of claims 1 to 8, wherein D is a phenyl group or a 5 to 6-membered heteroaryl group (the phenyl group or the 5 to 6-membered heteroaryl group is unsubstituted or substituted with a halogen atom, a nitro group, a C₁₋₆ alkyl group, a C₁₋₆ haloalkyl group, a C₁₋₆ alkoxy group, a C₁₋₆ haloalkoxy group (the C₁₋₆ alkyl group, the C₁₋₆ haloalkyl group, the C₁₋₆ alkoxy group, and the C₁₋₆ haloalkoxy group are unsubstituted or substituted with one or more nitro groups, one or more C₁₋₆ alkoxy groups, or one or more C₁₋₃ haloalkoxy groups), a C₁₋₆ alkylsulfonylamino group, or C₁₋₆ alkylsulfonyloxy group (the C₁₋₆ alkylsulfonylamino group and the C₁₋₆ alkylsulfonyloxy group are unsubstituted or substituted with one or more halogen atoms, one or more nitro groups, one or more C₁₋₆ alkoxy groups, or one or more C₁₋₃ haloalkoxy groups)).

10. The compound, tautomer of the compound, or pharmaceutically acceptable salt thereof, or solvate thereof according to claim 9, wherein D is a 5 to 6-membered heteroaryl group (the 5 to 6-membered heteroaryl group is unsubstituted or substituted with one or more halogen atoms, one or more nitro groups, one or more C₁₋₆ alkyl groups, one or more C₁₋₆ haloalkyl groups, one or more C₁₋₆ alkoxy groups, one or more C₁₋₆ haloalkoxy groups (the C₁₋₆ alkyl groups, the C₁₋₆ haloalkyl groups, the C₁₋₆ alkoxy groups, and the C₁₋₆ haloalkoxy groups are unsubstituted or substituted with one or more nitro groups, one or more C₁₋₆ alkoxy groups, or one or more C₁₋₃ haloalkoxy groups), one or more C₁₋₆ alkylsulfonylamino groups, or one or more C₁₋₆ alkylsulfonyloxy groups (the C₁₋₆ alkylsulfonylamino groups and the C₁₋₆ alkylsulfonyloxy groups are unsubstituted or substituted with one or more halogen atoms, one or more nitro groups, one or more C₁₋₆ alkoxy groups, or one or more C₁₋₃ haloalkoxy groups)).

11. The compound, tautomer of the compound, or pharmaceutically acceptable salt thereof, or solvate thereof according to claim 10, wherein D is a 5-membered heteroaryl group (the 5-membered heteroaryl group is unsubstituted or substituted with one or more C₁₋₆ alkyl groups or one or more C₁₋₆ haloalkyl groups).

12. The compound, tautomer of the compound, or pharmaceutically acceptable salt thereof, or solvate thereof according to claim 11, wherein D is a thienyl group substituted with a trifluoromethyl group or a thiazolyl group substituted with a trifluoromethyl group.

13. The compound, tautomer of the compound, or pharmaceutically acceptable salt thereof, or solvate thereof according to claim 12, wherein D is a 5-trifluoromethylthiophen-2-yl group.

14. The compound, tautomer of the compound, or pharmaceutically acceptable salt thereof, or solvate thereof according to any one of claims 1 to 6, wherein E is either Formula (IV)-1 or Formula (IV)-2 shown in (IV): 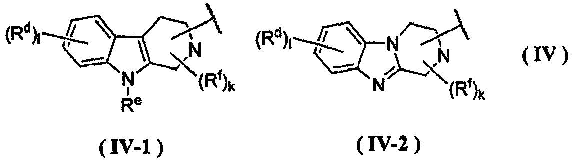 (wherein
R^{f} means
a hydrogen atom,
a hydroxy group,
an amino group, or
a halogen atom;
k is 1;
R^{e} means
a hydrogen atom,
a C₁₋₆ alkyl group, or
a C₁₋₆ haloalkyl group;
R^{d} means
a hydrogen atom,
a hydroxy group,
a halogen atom,
an amino group,
a C₁₋₆ alkyl group,
a C₁₋₆ haloalkyl group,
a C₁₋₆ alkoxy group,
a C₁₋₆ haloalkoxy group,
a mono-C₁₋₆ alkylamino group,
a di-C₁₋₆ alkylamino group, or
a C₁₋₆ alkylsulfonylamino group; and
1 is 1 or 2, and when 1 is 2, R^{d}s are the same as or different from each other).

15. The compound, tautomer of the compound, or pharmaceutically acceptable salt thereof, or solvate thereof according to claim 14, wherein R^{d} is a halogen atom and 1 is 1.

16. The compound, tautomer of the compound, or pharmaceutically acceptable salt thereof, or solvate thereof according to claim 14 or 15, wherein D is a 5-membered heteroaryl group (the 5-membered heteroaryl group is unsubstituted or substituted with one or more C₁₋₆ alkyl groups or one or more C₁₋₆ haloalkyl groups).

17. The compound, tautomer of the compound, or pharmaceutically acceptable salt thereof, or solvate thereof according to claim 16, wherein D is a thienyl group substituted with a trifluoromethyl group or a thiazolyl group substituted with a trifluoromethyl group.

18. The compound, tautomer of the compound, or pharmaceutically acceptable salt thereof, or solvate thereof according to claim 17, wherein D is a 5-trifluoromethylthiophen-2-yl group.

19. A T-type calcium channel inhibitor comprising the compound, tautomer of the compound, or pharmaceutically acceptable salt thereof, or solvate thereof as claimed in any one of claims 1 to 18, as an active component.

20. A preventive agent, a therapeutic agent, and/or an improving agent for a disease treatable by a T-type calcium channel inhibitory activity, comprising the T-type calcium channel inhibitor as claimed in claim 19 as an active component.

21. A therapeutic agent for neuropathic pain comprising the T-type calcium channel inhibitor as claimed in claim 19 as an active component.

22. A medicine comprising the compound, tautomer of the compound, or pharmaceutically acceptable salt thereof, or solvate thereof as described in any one of claims 1 to 18, as an active component.
